# EUROPEAN PATENT APPLICATION

(11) **EP 4 851 195 A1**
(43) Date of publication of application: **09.12.2026**
(21) Application number: 25748180.4
(22) Date of filing: 28.01.2025
(51) Int. Cl.: C07K 16/28, A61K 39/395, A61P 3/00, A61P 9/00, A61P 25/00, A61P 29/00, A61P 31/00, A61P 37/00, C12N 1/15, C12N 1/19, C12N 1/21, C12N 5/10, C12N 15/13

(54) **ANTIBODY, NUCLEIC ACID, CELL, AND MEDICINE**

(30) Priority: 31.01.2024 US 202463627739 P
(71) Applicant: NB Health Laboratory Co., Ltd., Sapporo-shi, Hokkaido 001-0021 (JP)
(72) Inventor: ASHIDA, Masami, Sapporo-shi, Hokkaido 001-0021 (JP); NAKAGAWA, Miki, Sapporo-shi, Hokkaido 001-0021 (JP); SHINAGAWA, Masahiko, Sapporo-shi, Hokkaido 001-0021 (JP); TANAKA, Ryoma, Sapporo-shi, Hokkaido 001-0021 (JP); HAYASHI, Yuki, Sapporo-shi, Hokkaido 001-0021 (JP); KAMADA, Yoko, Sapporo-shi, Hokkaido 001-0021 (JP); KURIBAYASHI, Saya, Sapporo-shi, Hokkaido 001-0021 (JP); KIDA, Hiroshi, Sapporo-shi, Hokkaido 060-0808 (JP)
(74) Representative: Ter Meer und Partner mbB
(86) International application number: PCT/JP2025/002546
(87) International publication number: WO 2025/164598

(57) **Abstract**

Provided is a novel anti-human CXCR3 antibody useful for an antibody drug. Provided is an antibody that specifically binds to human CXCR3, wherein the antibody specifically binds to an extracellular domain of human CXCR3A, includes an activity of blocking a CXCR3A-dependent cell function, and does not specifically bind to a human vascular endothelial cell. The antibody includes heavy chain CDRs 1-3 and light chain CDRs 1-3 containing specific amino acid sequences. It is preferable that the antibody is a humanized antibody or a chimeric antibody.

## Description

### TECHNICAL FIELD

This disclosure relates to an antibody that specifically binds to an extracellular domain of human CXCR3, a nucleic acid encoding the antibody, a cell including the nucleic acid, and a medicament including the antibody as an active ingredient.

### BACKGROUND ART

Chemokines are chemotactic cytokines secreted from various cells. Chemokines are classified into four groups, CXC, CC, C, and CX3C, according to differences in amino acid sequence and structure. Chemokine functions are important for differentiation and homeostasis of immune cells, induction of primary and secondary immune responses, and migration of immune cells in various diseases. Nearly 50 types of chemokines have been reported in humans and mice (Non-Patent Document 1). The action of chemokines is exerted by binding to a classical chemokine receptor, which is a seven-transmembrane G protein-coupled receptor (GPCR). There are also atypical chemokine receptors that bind to chemokines but do not couple with G proteins, and are responsible for the formation of chemokine concentration gradients. In mammals, about 20 classical chemokine receptors and 4 atypical chemokine receptors have been found (Non-Patent Document 1).

CXCR3, also known as G protein-coupled receptor 9 (GPR9) and CD183, is a chemokine receptor expressed primarily on T cells. CXCR3 is expressed in a variety of organisms, for example, humans, mice, rats, cows, chimpanzees, macaques, dogs, frogs, platypuses, pigs, and zebrafish. In humans, there are three types of isoforms, CXCR3A, CXCR3B, and CXCR3Alt.

CXCR3A is the most predominant isoform in vivo and is highly expressed on activated CD8-positive T cells, memory CD4-positive T cells, memory CD8-positive T cells, and NK cells. CXCR3A plays an important role in immunophysiological processes such as homing of effector cells to inflammatory sites and clearance of pathogens. CXCR3A is also expressed on other immune cells, including subsets of dendritic cells, subsets of B cells, macrophages, Th17 cells, regulatory T cells, and neutrophils (Non-Patent Documents 1 and 2). Functional ligands for CXCR3A are interferon-γ (IFN-γ)-inducible chemokines CXCL9 (MIG-9), CXCL10 (IP-10), and CXCL11 (I-TAC). These three ligands have been shown to act redundantly, synergistically, or antagonistically in vivo (Non-Patent Documents 2 and 3). Activation of CXCR3A by CXCL9, CXCL10, and CXCL11 leads to activation of Gαq and Gαi, which induces chemotaxis, cell proliferation, cell survival, and tumor cell metastasis.

CXCR3B has an N-terminal domain that is 47 amino acids longer than that of CXCR3A due to alternative splicing. Expression of CXCR3B has been confirmed in tissues such as the heart, skeletal muscle, liver, and kidney, and in vascular endothelial cells. The major ligand for CXCR3B is CXCL4. Also, CXCL9, CXCL10, and CXCL11 bind to CXCR3B in vitro. Binding of the ligand to CXCR3B activates Gαs, exhibiting inhibition of proliferation, inhibition of migration, and promotion of apoptosis in endothelial cells and tumor cells. In other words, CXCR3A and CXCR3B are thought to have opposite effects. CXCR3Alt is slightly co-expressed in CXCR3A-expressing cells and plays a role in chemotaxis to CXCL11 (Non-Patent Document 2).

Activation of a cascade by binding of ligands to CXCR3 primarily induces immune cell migration to tissues, integrin activation, and cellular infiltration. CXCL9, CXCL10, and CXCL11 are produced in large amounts at inflammatory sites in diseases such as autoimmune diseases, transplantation, infection, and tumors, and are thought to be involved in the mobilization of immune cells to inflammatory sites and to contribute to various inflammatory diseases. Examples include Th1-related diseases such as atherosclerosis, myocarditis, multiple sclerosis, asthma, chronic obstructive pulmonary disease, pulmonary fibrosis, type 1 diabetes, psoriasis, rheumatism, inflammatory bowel disease, systemic lupus erythematosus, and acute cardiac allograft rejection (Non-Patent Document 3). More recent studies have also suggested associations with a wider range of diseases, including influenza, viral infections such as novel coronavirus infection (COVID-19), and tumors (Non-Patent Documents 3 and 4). Thus, there is a need for substances (e.g., antibodies) and methods that target CXCR3 signaling to reduce the progression of disorders associated with CXCR3 signaling.

Significant efforts have been made to develop drugs that specifically target chemokine receptors. However, only three drugs have been approved by the U.S. Food and Drug Administration (FDA): the CCR5 inverse agonist Maraviroc (antiretroviral drug), the CXCR4 antagonist Plerixafor (therapeutic agent for multiple myeloma), and the anti-CCR4 monoclonal antibody Mogamulizumab (therapeutic agent for T-cell leukemia). Factors such as redundancy of receptor-ligand binding, pathological and physiological diversity of their roles in the body, and difficulty in developing specific small molecules due to similarities in receptor structures are believed to make drug development difficult (Non-Patent Document 5).

CXCR3 has also been a target for many drug discovery programs, and development of small-molecule antagonists and antibodies has been underway. However, only two small-molecule CXCR3 antagonists, AMG487 and ACT-777991, have advanced to clinical trials. AMG487 advanced to a Phase IIa trial for psoriasis, but efficacy was not demonstrated. ACT-777991, which is being developed as a therapeutic agent for type 1 diabetes, has completed a Phase I trial. Good tolerability, pharmacokinetics, and safety were confirmed in healthy subjects, and it is expected to advance to further clinical development stages (Non-Patent Document 6). On the other hand, it has been suggested that Eldelumab (BMS-936557), a human monoclonal antibody targeting CXCL10, could be an effective treatment for moderate to severe active ulcerative colitis (UC) in Phase II clinical trials for the treatment of UC and Crohn's disease (Non-Patent Document 7). These results suggest the clinical value of therapies targeting the CXCL10-CXCR3 cascade for Th1-related diseases.

Patent Document 1 discloses humanized antibodies of six types of monoclonal antibodies that specifically bind to human CXCR3. Furthermore, amino acid sequences of complementarity determining regions, heavy chain variable regions, and light chain variable regions of each monoclonal antibody are disclosed.

Patent Document 2 discloses four types of monoclonal antibodies that specifically bind to human CXCR3 and humanized antibodies thereof. Furthermore, amino acid sequences of complementarity determining regions, heavy chain variable regions, and light chain variable regions of each monoclonal antibody are disclosed. These anti-human CXCR3 antibodies have been shown to be particularly effective in the treatment of type 1 diabetes.

Patent Document 3 discloses a humanized antibody of a monoclonal antibody that specifically binds to human CXCR3. Furthermore, amino acid sequences of complementarity determining regions, heavy chain variable regions, and light chain variable regions of this monoclonal antibody are disclosed. It has been shown that depletion of CXCR3 by this antibody is effective for the treatment of vitiligo.

However, the antibodies described in Patent Documents 1 to 3 have not reached practical use as therapeutic agents. In this technical field, there is a need for further or improved anti-human CXCR3 antibodies having superior properties as pharmaceutical materials.

### PRIOR ART DOCUMENTS

### PATENT DOCUMENTS

[Patent Document 1] WO 2008/094942 A1
[Patent Document 2] WO 2013/109974 A1
[Patent Document 3] WO 2018/119288 A1

### NON-PATENT DOCUMENTS

**[**Non-Patent Document 1] Catherine E. Hughes and Robert J. B., "A guide to chemokines and their receptors." FEBS J. 2018 Aug; 285(16): 2944-2971.
**[**Non-Patent Document 2] Devi Satarkar, Chinmoy Patra, "Evolution, Expression and Functional Analysis of CXCR3 in Neuronal and Cardiovascular Diseases: A Narrative Review", Front Cell Dev Biol. 2022 Jun 20:10:882017.
**[**Non-Patent Document 3] Joanna R Groom and Andrew D Luster, "CXCR3 ligands: redundant, collaborative and antagonistic functions" Immunol Cell Biol. 2011 Feb; 89(2),207-215.
**[**Non-Patent Document 4] Daniel Reynolds, Cristina Vazquez Guillamet, Aaron Day, Nicholas Borcherding, Rodrigo Vazquez Guillamet, Jose Alberto Choreno-Parra, Stacey L. House, Jane A. O'Halloran, Joaquin Zuniga, Ali H. Ellebedy, Derek E. Byers, and Philip A. Mudd, "Comprehensive Immunologic Evaluation of Bronchoalveolar Lavage Samples from Human Patients with Moderate and Severe Seasonal Influenza and Severe COVID-19", J Immunol. 2021 Sep 1;207(5):1229-1238.
**[**Non-Patent Document 5] Wing Yee Lai and Anja Mueller, "Latest update on chemokine receptors as therapeutic targets", Biochem Soc Trans. 2021 Jun 30; 49(3): 1385-1395.
**[**Non-Patent Document 6] Marie-Laure Boof, Martine Gehin, Christine Voors-Pette, Chih-Hsuan Hsin, Virginie Sippel, Daniel S Strasser, Jasper Dingemanse, "Pharmacokinetics, pharmacodynamics and safety of the novel C-X-C chemokine receptor 3 antagonist ACT-777991: Results from the first-in-human study in healthy adults", Br J Clin Pharmacol. 2024 Feb; 90(2): 588-599.
**[**Non-Patent Document 7] Lloyd Mayer, William J Sandborn, Yuriy Stepanov, Karel Geboes, Robert Hardi, Michael Yellin, Xiaolu Tao, Li An Xu, Luisa Salter-Cid, Sheila Gujrathi, Richard Aranda, Allison Y Luo, "Anti-IP-10 antibody (BMS-936557) for ulcerative colitis: a phase II randomised study", Gut. 2014 Mar; 63(3): 442-50.

### DISCLOSURE OF INVENTION

### TECHNICAL PROBLEM

As described above, antibodies that inhibit the function of CXCR3 are expected to be useful as antibody drugs, but none have yet reached practical use as therapeutic agents. Therefore, an object of the present disclosure is to provide a novel anti-human CXCR3 antibody having superior properties as a pharmaceutical material.

### SOLUTION TO PROBLEM

An aspect of the present disclosure is an antibody that specifically binds to human CXCR3, wherein the antibody specifically binds to an extracellular domain of human CXCR3A, the antibody includes an activity of blocking a CXCR3A-dependent cell function, the antibody does not specifically bind to a human vascular endothelial cell, and the antibody satisfies any one of the following (AB1) to (AB9) and (AB12) to (AB27):
(AB26) including a heavy chain CDR1 including an amino acid sequence represented by SEQ ID NO: 251, a heavy chain CDR2 including an amino acid sequence represented by SEQ ID NO: 252, a heavy chain CDR3 including an amino acid sequence represented by SEQ ID NO: 253, a light chain CDR1 including an amino acid sequence represented by SEQ ID NO: 254, a light chain CDR2 including an amino acid sequence represented by SEQ ID NO: 255, and a light chain CDR3 including an amino acid sequence represented by SEQ ID NO: 256;
(AB23) including a heavy chain CDR1 including an amino acid sequence represented by SEQ ID NO: 221, a heavy chain CDR2 including an amino acid sequence represented by SEQ ID NO: 222, a heavy chain CDR3 including an amino acid sequence represented by SEQ ID NO: 223, a light chain CDR1 including an amino acid sequence represented by SEQ ID NO: 224, a light chain CDR2 including an amino acid sequence represented by SEQ ID NO: 225, and a light chain CDR3 including an amino acid sequence represented by SEQ ID NO: 226;
(AB24) including a heavy chain CDR1 including an amino acid sequence represented by SEQ ID NO: 231, a heavy chain CDR2 including an amino acid sequence represented by SEQ ID NO: 232, a heavy chain CDR3 including an amino acid sequence represented by SEQ ID NO: 233, a light chain CDR1 including an amino acid sequence represented by SEQ ID NO: 234, a light chain CDR2 including an amino acid sequence represented by SEQ ID NO: 235, and a light chain CDR3 including an amino acid sequence represented by SEQ ID NO: 236;
(AB25) including a heavy chain CDR1 including an amino acid sequence represented by SEQ ID NO: 241, a heavy chain CDR2 including an amino acid sequence represented by SEQ ID NO: 242, a heavy chain CDR3 including an amino acid sequence represented by SEQ ID NO: 243, a light chain CDR1 including an amino acid sequence represented by SEQ ID NO: 244, a light chain CDR2 including an amino acid sequence represented by SEQ ID NO: 245, and a light chain CDR3 including an amino acid sequence represented by SEQ ID NO: 246;
(AB27) including a heavy chain CDR1 including an amino acid sequence represented by SEQ ID NO: 261, a heavy chain CDR2 including an amino acid sequence represented by SEQ ID NO: 262, a heavy chain CDR3 including an amino acid sequence represented by SEQ ID NO: 263, a light chain CDR1 including an amino acid sequence represented by SEQ ID NO: 264, a light chain CDR2 including an amino acid sequence represented by SEQ ID NO: 265, and a light chain CDR3 including an amino acid sequence represented by SEQ ID NO: 266;
(AB1) including a heavy chain CDR1 including an amino acid sequence represented by SEQ ID NO: 1, a heavy chain CDR2 including an amino acid sequence represented by SEQ ID NO: 2, a heavy chain CDR3 including an amino acid sequence represented by SEQ ID NO: 3, a light chain CDR1 including an amino acid sequence represented by SEQ ID NO: 4, a light chain CDR2 including an amino acid sequence represented by SEQ ID NO: 5, and a light chain CDR3 including an amino acid sequence represented by SEQ ID NO: 6;
(AB2) including a heavy chain CDR1 including an amino acid sequence represented by SEQ ID NO: 11, a heavy chain CDR2 including an amino acid sequence represented by SEQ ID NO: 12, a heavy chain CDR3 including an amino acid sequence represented by SEQ ID NO: 13, a light chain CDR1 including an amino acid sequence represented by SEQ ID NO: 14, a light chain CDR2 including an amino acid sequence represented by SEQ ID NO: 15, and a light chain CDR3 including an amino acid sequence represented by SEQ ID NO: 16;
(AB3) including a heavy chain CDR1 including an amino acid sequence represented by SEQ ID NO: 21, a heavy chain CDR2 including an amino acid sequence represented by SEQ ID NO: 22, a heavy chain CDR3 including an amino acid sequence represented by SEQ ID NO: 23, a light chain CDR1 including an amino acid sequence represented by SEQ ID NO: 24, a light chain CDR2 including an amino acid sequence represented by SEQ ID NO: 25, and a light chain CDR3 including an amino acid sequence represented by SEQ ID NO: 26;
(AB5) including a heavy chain CDR1 including an amino acid sequence represented by SEQ ID NO: 41, a heavy chain CDR2 including an amino acid sequence represented by SEQ ID NO: 42, a heavy chain CDR3 including an amino acid sequence represented by SEQ ID NO: 43, a light chain CDR1 including an amino acid sequence represented by SEQ ID NO: 44, a light chain CDR2 including an amino acid sequence represented by SEQ ID NO: 45, and a light chain CDR3 including an amino acid sequence represented by SEQ ID NO: 46;
(AB8) including a heavy chain CDR1 including an amino acid sequence represented by SEQ ID NO: 71, a heavy chain CDR2 including an amino acid sequence represented by SEQ ID NO: 72, a heavy chain CDR3 including an amino acid sequence represented by SEQ ID NO: 73, a light chain CDR1 including an amino acid sequence represented by SEQ ID NO: 74, a light chain CDR2 including an amino acid sequence represented by SEQ ID NO: 75, and a light chain CDR3 including an amino acid sequence represented by SEQ ID NO: 76;
(AB16) including a heavy chain CDR1 including an amino acid sequence represented by SEQ ID NO: 151, a heavy chain CDR2 including an amino acid sequence represented by SEQ ID NO: 152, a heavy chain CDR3 including an amino acid sequence represented by SEQ ID NO: 153, a light chain CDR1 including an amino acid sequence represented by SEQ ID NO: 154, a light chain CDR2 including an amino acid sequence represented by SEQ ID NO: 155, and a light chain CDR3 including an amino acid sequence represented by SEQ ID NO: 156;
(AB17) including a heavy chain CDR1 including an amino acid sequence represented by SEQ ID NO: 161, a heavy chain CDR2 including an amino acid sequence represented by SEQ ID NO: 162, a heavy chain CDR3 including an amino acid sequence represented by SEQ ID NO: 163, a light chain CDR1 including an amino acid sequence represented by SEQ ID NO: 164, a light chain CDR2 including an amino acid sequence represented by SEQ ID NO: 165, and a light chain CDR3 including an amino acid sequence represented by SEQ ID NO: 166;
(AB18) including a heavy chain CDR1 including an amino acid sequence represented by SEQ ID NO: 171, a heavy chain CDR2 including an amino acid sequence represented by SEQ ID NO: 172, a heavy chain CDR3 including an amino acid sequence represented by SEQ ID NO: 173, a light chain CDR1 including an amino acid sequence represented by SEQ ID NO: 174, a light chain CDR2 including an amino acid sequence represented by SEQ ID NO: 175, and a light chain CDR3 including an amino acid sequence represented by SEQ ID NO: 176;
(AB20) including a heavy chain CDR1 including an amino acid sequence represented by SEQ ID NO: 191, a heavy chain CDR2 including an amino acid sequence represented by SEQ ID NO: 192, a heavy chain CDR3 including an amino acid sequence represented by SEQ ID NO: 193, a light chain CDR1 including an amino acid sequence represented by SEQ ID NO: 194, a light chain CDR2 including an amino acid sequence represented by SEQ ID NO: 195, and a light chain CDR3 including an amino acid sequence represented by SEQ ID NO: 196; and
(AB21) including a heavy chain CDR1 including an amino acid sequence represented by SEQ ID NO: 201, a heavy chain CDR2 including an amino acid sequence represented by SEQ ID NO: 202, a heavy chain CDR3 including an amino acid sequence represented by SEQ ID NO: 203, a light chain CDR1 including an amino acid sequence represented by SEQ ID NO: 204, a light chain CDR2 including an amino acid sequence represented by SEQ ID NO: 205, and a light chain CDR3 including an amino acid sequence represented by SEQ ID NO: 206;
(AB22) including a heavy chain CDR1 including an amino acid sequence represented by SEQ ID NO: 211, a heavy chain CDR2 including an amino acid sequence represented by SEQ ID NO: 212, a heavy chain CDR3 including an amino acid sequence represented by SEQ ID NO: 213, a light chain CDR1 including an amino acid sequence represented by SEQ ID NO: 214, a light chain CDR2 including an amino acid sequence represented by SEQ ID NO: 215, and a light chain CDR3 including an amino acid sequence represented by SEQ ID NO: 216;
(AB4) including a heavy chain CDR1 including an amino acid sequence represented by SEQ ID NO: 31, a heavy chain CDR2 including an amino acid sequence represented by SEQ ID NO: 32, a heavy chain CDR3 including an amino acid sequence represented by SEQ ID NO: 33, a light chain CDR1 including an amino acid sequence represented by SEQ ID NO: 34, a light chain CDR2 including an amino acid sequence represented by SEQ ID NO: 35, and a light chain CDR3 including an amino acid sequence represented by SEQ ID NO: 36;
(AB6) including a heavy chain CDR1 including an amino acid sequence represented by SEQ ID NO: 51, a heavy chain CDR2 including an amino acid sequence represented by SEQ ID NO: 52, a heavy chain CDR3 including an amino acid sequence represented by SEQ ID NO: 53, a light chain CDR1 including an amino acid sequence represented by SEQ ID NO: 54, a light chain CDR2 including an amino acid sequence represented by SEQ ID NO: 55, and a light chain CDR3 including an amino acid sequence represented by SEQ ID NO: 56;
(AB7) including a heavy chain CDR1 including an amino acid sequence represented by SEQ ID NO: 61, a heavy chain CDR2 including an amino acid sequence represented by SEQ ID NO: 62, a heavy chain CDR3 including an amino acid sequence represented by SEQ ID NO: 63, a light chain CDR1 including an amino acid sequence represented by SEQ ID NO: 64, a light chain CDR2 including an amino acid sequence represented by SEQ ID NO: 65, and a light chain CDR3 including an amino acid sequence represented by SEQ ID NO: 66;
(AB9) including a heavy chain CDR1 including an amino acid sequence represented by SEQ ID NO: 81, a heavy chain CDR2 including an amino acid sequence represented by SEQ ID NO: 82, a heavy chain CDR3 including an amino acid sequence represented by SEQ ID NO: 83, a light chain CDR1 including an amino acid sequence represented by SEQ ID NO: 84, a light chain CDR2 including an amino acid sequence represented by SEQ ID NO: 85, and a light chain CDR3 including an amino acid sequence represented by SEQ ID NO: 86;
(AB12) including a heavy chain CDR1 including an amino acid sequence represented by SEQ ID NO: 111, a heavy chain CDR2 including an amino acid sequence represented by SEQ ID NO: 112, a heavy chain CDR3 including an amino acid sequence represented by SEQ ID NO: 113, a light chain CDR1 including an amino acid sequence represented by SEQ ID NO: 114, a light chain CDR2 including an amino acid sequence represented by SEQ ID NO: 115, and a light chain CDR3 including an amino acid sequence represented by SEQ ID NO: 116;
(AB13) including a heavy chain CDR1 including an amino acid sequence represented by SEQ ID NO: 121, a heavy chain CDR2 including an amino acid sequence represented by SEQ ID NO: 122, a heavy chain CDR3 including an amino acid sequence represented by SEQ ID NO: 123, a light chain CDR1 including an amino acid sequence represented by SEQ ID NO: 124, a light chain CDR2 including an amino acid sequence represented by SEQ ID NO: 125, and a light chain CDR3 including an amino acid sequence represented by SEQ ID NO: 126;
(AB14) including a heavy chain CDR1 including an amino acid sequence represented by SEQ ID NO: 131, a heavy chain CDR2 including an amino acid sequence represented by SEQ ID NO: 132, a heavy chain CDR3 including an amino acid sequence represented by SEQ ID NO: 133, a light chain CDR1 including an amino acid sequence represented by SEQ ID NO: 134, a light chain CDR2 including an amino acid sequence represented by SEQ ID NO: 135, and a light chain CDR3 including an amino acid sequence represented by SEQ ID NO: 136;
(AB15) including a heavy chain CDR1 including an amino acid sequence represented by SEQ ID NO: 141, a heavy chain CDR2 including an amino acid sequence represented by SEQ ID NO: 142, a heavy chain CDR3 including an amino acid sequence represented by SEQ ID NO: 143, a light chain CDR1 including an amino acid sequence represented by SEQ ID NO: 144, a light chain CDR2 including an amino acid sequence represented by SEQ ID NO: 145, and a light chain CDR3 including an amino acid sequence represented by SEQ ID NO: 146; and
(AB19) including a heavy chain CDR1 including an amino acid sequence represented by SEQ ID NO: 181, a heavy chain CDR2 including an amino acid sequence represented by SEQ ID NO: 182, a heavy chain CDR3 including an amino acid sequence represented by SEQ ID NO: 183, a light chain CDR1 including an amino acid sequence represented by SEQ ID NO: 184, a light chain CDR2 including an amino acid sequence represented by SEQ ID NO: 185, and a light chain CDR3 including an amino acid sequence represented by SEQ ID NO: 186.

An aspect of the present disclosure is an antibody that specifically binds to human CXCR3, wherein the antibody specifically binds to an extracellular domain of human CXCR3A, the antibody includes an activity of blocking a CXCR3A-dependent cell function, the antibody does not specifically bind to a human vascular endothelial cell, and the antibody satisfies any one of the following (C1) to (C9) and (C12) to (C27):
(C26) including a heavy chain variable region including an amino acid sequence represented by SEQ ID NO: 257 and a light chain variable region including an amino acid sequence represented by SEQ ID NO: 259;
(C23) including a heavy chain variable region including an amino acid sequence represented by SEQ ID NO: 227 and a light chain variable region including an amino acid sequence represented by SEQ ID NO: 229;
(C24) including a heavy chain variable region including an amino acid sequence represented by SEQ ID NO: 237 and a light chain variable region including an amino acid sequence represented by SEQ ID NO: 239;
(C25) including a heavy chain variable region including an amino acid sequence represented by SEQ ID NO: 247 and a light chain variable region including an amino acid sequence represented by SEQ ID NO: 249;
(C27) including a heavy chain variable region including an amino acid sequence represented by SEQ ID NO: 267 and a light chain variable region including an amino acid sequence represented by SEQ ID NO: 269;
(C1) including a heavy chain variable region including an amino acid sequence represented by SEQ ID NO: 7 and a light chain variable region including an amino acid sequence represented by SEQ ID NO: 9;
(C2) including a heavy chain variable region including an amino acid sequence represented by SEQ ID NO: 17 and a light chain variable region including an amino acid sequence represented by SEQ ID NO: 19;
(C3) including a heavy chain variable region including an amino acid sequence represented by SEQ ID NO: 27 and a light chain variable region including an amino acid sequence represented by SEQ ID NO: 29;
(C5) including a heavy chain variable region including an amino acid sequence represented by SEQ ID NO: 47 and a light chain variable region including an amino acid sequence represented by SEQ ID NO: 49;
(C8) including a heavy chain variable region including an amino acid sequence represented by SEQ ID NO: 77 and a light chain variable region including an amino acid sequence represented by SEQ ID NO: 79;
(C16) including a heavy chain variable region including an amino acid sequence represented by SEQ ID NO: 157 and a light chain variable region including an amino acid sequence represented by SEQ ID NO: 159;
(C17) including a heavy chain variable region including an amino acid sequence represented by SEQ ID NO: 167 and a light chain variable region including an amino acid sequence represented by SEQ ID NO: 169;
(C18) including a heavy chain variable region including an amino acid sequence represented by SEQ ID NO: 177 and a light chain variable region including an amino acid sequence represented by SEQ ID NO: 179;
(C20) including a heavy chain variable region including an amino acid sequence represented by SEQ ID NO: 197 and a light chain variable region including an amino acid sequence represented by SEQ ID NO: 199;
(C21) including a heavy chain variable region including an amino acid sequence represented by SEQ ID NO: 207 and a light chain variable region including an amino acid sequence represented by SEQ ID NO: 209;
(C22) including a heavy chain variable region including an amino acid sequence represented by SEQ ID NO: 217 and a light chain variable region including an amino acid sequence represented by SEQ ID NO: 219;
(C4) including a heavy chain variable region including an amino acid sequence represented by SEQ ID NO: 37 and a light chain variable region including an amino acid sequence represented by SEQ ID NO: 39;
(C6) including a heavy chain variable region including an amino acid sequence represented by SEQ ID NO: 57 and a light chain variable region including an amino acid sequence represented by SEQ ID NO: 59;
(C7) including a heavy chain variable region including an amino acid sequence represented by SEQ ID NO: 67 and a light chain variable region including an amino acid sequence represented by SEQ ID NO: 69;
(C9) including a heavy chain variable region including an amino acid sequence represented by SEQ ID NO: 87 and a light chain variable region including an amino acid sequence represented by SEQ ID NO: 89;
(C12) including a heavy chain variable region including an amino acid sequence represented by SEQ ID NO: 117 and a light chain variable region including an amino acid sequence represented by SEQ ID NO: 119;
(C13) including a heavy chain variable region including an amino acid sequence represented by SEQ ID NO: 127 and a light chain variable region including an amino acid sequence represented by SEQ ID NO: 129;
(C14) including a heavy chain variable region including an amino acid sequence represented by SEQ ID NO: 137 and a light chain variable region including an amino acid sequence represented by SEQ ID NO: 139;
(C15) including a heavy chain variable region including an amino acid sequence represented by SEQ ID NO: 147 and a light chain variable region including an amino acid sequence represented by SEQ ID NO: 149; and
(C19) including a heavy chain variable region including an amino acid sequence represented by SEQ ID NO: 187 and a light chain variable region including an amino acid sequence represented by SEQ ID NO: 189.

An aspect of the present disclosure is a second antibody that specifically binds to human CXCR3, wherein the second antibody specifically binds to an extracellular domain of human CXCR3A, the second antibody includes an activity of blocking a CXCR3A-dependent cell function, the second antibody does not specifically bind to a human vascular endothelial cell, and the second antibody competitively inhibits a binding between a first antibody that is the above antibody and a receptor.

Preferably, the antibody or the second antibody is a humanized antibody or a chimeric antibody.

Preferably, the antibody or the second antibody is a multispecific antibody.

Preferably, the antibody or the second antibody includes an internalization activity.

Preferably, the antibody or the second antibody is a modified antibody bonded with another molecule.

Preferably, the modified antibody is an antibody-drug conjugate.

An aspect of the present disclosure is a nucleic acid encoding the above antibody or second antibody.

An aspect of the present disclosure is a cell including the above nucleic acid.

An aspect of the present disclosure is a medicament including the above antibody as an active ingredient.

Preferably, the medicament is for use in the treatment of a disease, disorder, or symptom involving impairment of a CXCR3-dependent cell function.

Preferably, the disease, disorder, or symptom is a Th1-type immune disease.

Preferably, the Th1-type immune disease is a cardiovascular disorder, a neurological disorder, an inflammatory disease, an autoimmune disease, a metabolic disease, an infectious disease, a hematological cancer, or a solid cancer.

### EFFECT OF INVENTION

According to the present disclosure, it is possible to provide a novel anti-human CXCR3 antibody having superior characteristics as a pharmaceutical material.

### BRIEF DESCRIPTION OF DRAWINGS

Fig. 1 is a graph presenting results of quantification of CXCR3A and CXCR3B gene expression in Example 6, showing that human vascular endothelial cells predominantly express CXCR3B.
Fig. 2 is a histogram presenting results of flow cytometry performed in Example 7, showing binding properties between antibodies (13B4 or 201009-2-C) and human CXCR3A-stably expressing CHO cells or human vascular endothelial cells.
Fig. 3A is a graph presenting an evaluation result of binding property of an antibody (13B4) to human CXCR3A -stably expressing CHO cells.
Fig. 3B is a graph presenting an evaluation result of binding property of an antibody (11F11) to human CXCR3A -stably expressing CHO cells.
Fig. 3C is a graph presenting an evaluation result of binding property of an antibody (201006-3-H) to human CXCR3A -stably expressing CHO cells.
Fig. 3D is a graph presenting an evaluation result of binding property of an antibody (201006-5-F) to human CXCR3A -stably expressing CHO cells.
Fig. 3E is a graph presenting an evaluation result of binding property of an antibody (201006-7-G) to human CXCR3A -stably expressing CHO cells.
Fig. 3F is a graph presenting an evaluation result of binding property of an antibody (201009-2-C) to human CXCR3A -stably expressing CHO cells.
Fig. 3G is a graph presenting an evaluation result of binding property of an antibody (201009-3-H) to human CXCR3A -stably expressing CHO cells.
Fig. 3H is a graph presenting an evaluation result of binding property of an antibody (201009-1-C) to human CXCR3A -stably expressing CHO cells.
Fig. 4A is a graph presenting a dose dependency of inhibitory activity of an antibody (13B4) on human CXCR3A.
Fig. 4B is a graph presenting a dose dependency of inhibitory activity of an antibody (11F11) on human CXCR3A.
Fig. 4C is a graph presenting a dose dependency of inhibitory activity of an antibody (201006-3-H) on human CXCR3A.
Fig. 4D is a graph presenting a dose dependency of inhibitory activity of an antibody (201006-5-F) on human CXCR3A.
Fig. 4E is a graph presenting a dose dependency of inhibitory activity of an antibody (201006-7-G) on human CXCR3A.
Fig. 4F is a graph presenting a dose dependency of inhibitory activity of an antibody (201009-2-C) on human CXCR3A.
Fig. 4G is a graph presenting a dose dependency of inhibitory activity of an antibody (201009-3-H) on human CXCR3A.
Fig. 4H is a graph presenting a dose dependency of inhibitory activity of an antibody (201009-1-C) on human CXCR3A.
Fig. 5 is a histogram presenting results of flow cytometry performed in Example 11, showing specific binding between an antibody (201006-7-G or 201009-2-C) and human CXCR3A.
Fig. 6A is a graph presenting an evaluation result of binding property of an antibody (13B4) to human peripheral blood mononuclear cell-derived T cells.
Fig. 6B is a graph presenting an evaluation result of binding property of an antibody (11F11) to human peripheral blood mononuclear cell-derived T cells.
Fig. 6C is a graph presenting an evaluation result of binding property of an antibody (201006-3-H) to human peripheral blood mononuclear cell-derived T cells.
Fig. 6D is a graph presenting an evaluation result of binding property of an antibody (201006-5-F) to human peripheral blood mononuclear cell-derived T cells.
Fig. 6E is a graph presenting an evaluation result of binding property of an antibody (201006-7-G) to human peripheral blood mononuclear cell-derived T cells.
Fig. 6F is a graph presenting an evaluation result of binding property of an antibody (201009-2-C) to human peripheral blood mononuclear cell-derived T cells.
Fig. 7 is a graph presenting a dose dependency of inhibitory activity of an antibody (201009-2-C) on the migration of human peripheral blood mononuclear cell-derived T cells.
Fig. 8 is a histogram presenting results of flow cytometry performed in Example 14, showing that an antibody is internalized together with a receptor in human peripheral blood mononuclear cell-derived T cells.
Fig. 9A is an explanatory diagram presenting an alignment of heavy chain variable regions of humanized anti-CXCR3 antibodies.
Fig. 9B is an explanatory diagram presenting an alignment of light chain variable regions of the humanized anti-CXCR3 antibodies.
Fig. 10A is a graph presenting an evaluation result of binding property of an antibody (VH1+VL1) to human peripheral blood mononuclear cell-derived T cells.
Fig. 10B is a graph presenting an evaluation result of binding property of an antibody (VH1+VL2) to human peripheral blood mononuclear cell-derived T cells.
Fig. 10C is a graph presenting an evaluation result of binding property of an antibody (VH1+VL3) to human peripheral blood mononuclear cell-derived T cells.
Fig. 10D is a graph presenting an evaluation result of binding property of an antibody (VH1+VL4) to human peripheral blood mononuclear cell-derived T cells.
Fig. 10E is a graph presenting an evaluation result of binding property of an antibody (VH2+VL1) to human peripheral blood mononuclear cell-derived T cells.
Fig. 10F is a graph presenting an evaluation result of binding property of an antibody (VH2+VL2) to human peripheral blood mononuclear cell-derived T cells.
Fig. 10G is a graph presenting an evaluation result of binding property of an antibody (VH2+VL3) to human peripheral blood mononuclear cell-derived T cells.
Fig. 10H is a graph presenting an evaluation result of binding property of an antibody (VH2+VL4) to human peripheral blood mononuclear cell-derived T cells.
Fig. 11A is a graph presenting a dose dependency of inhibitory activity of an antibody (VH1+VL1) on migration of human peripheral blood mononuclear cell-derived T cells.
Fig. 11B is a graph presenting a dose dependency of inhibitory activity of an antibody (VH1+VL4) on migration of human peripheral blood mononuclear cell-derived T cells.
Fig. 11C is a graph presenting a dose dependency of inhibitory activity of an antibody (VH2+VL1) on migration of human peripheral blood mononuclear cell-derived T cells.
Fig. 11D is a graph presenting a dose dependency of inhibitory activity of an antibody (VH2+VL4) on migration of human peripheral blood mononuclear cell-derived T cells.

### BEST MODE FOR CARRYING OUT THE INVENTION

In the present disclosure, a complementarity determining region is abbreviated as CDR. In the present disclosure, a heavy chain variable region, a heavy chain constant region, a light chain variable region, and a light chain constant region are abbreviated as VH, CH, VL, and CL respectively in some cases. In the present disclosure, the term "antibody" may be replaced with "immunoglobulin". In the present disclosure, the term "nucleic acid" may be replaced with "DNA" or "gene".

### <Human CXCR3>

Human CXCR3 receptor is a type of G protein coupled receptor (GPCR), which penetrates a cell membrane seven times and has an N-terminal extracellularly oriented and a C-terminal intracellularly oriented. Human CXCR3 includes three isoforms: CXCR3A, CXCR3B, and CXCR3Alt. A gene (cDNA) encoding human CXCR3 has already been isolated, and the amino acid sequence of human CXCR3 is also known. Sequence information of CXCR3 can be obtained from gene databases (e.g., NCBI Reference Sequence: AAO92295). As an example, the amino acid sequence of human CXCR3A is represented by SEQ ID NO: 281, and the amino acid sequence of human CXCR3B is represented by SEQ ID NO: 292.

Each domain in human CXCR3A is considered to correspond to each of the following parts in the amino acid sequence represented by SEQ ID NO: 281. The amino acid numbers are described on the left side, and domains are described on the right side. Each boundary between the domains may be slightly variable.
1 to 53: N-terminal domain
81 to 89: Intracellular first loop domain
111 to 125: Extracellular first loop domain
148 to 169: Intracellular second loop domain
190 to 212: Extracellular second loop domain
234 to 255: Intracellular third loop domain
278 to 298: Extracellular third loop domain
322 to 368: C-terminal domain

### <Anti-CXCR3 Antibody>

The antibody (anti-human CXCR3 antibody) disclosed herein specifically binds to human CXCR3, specifically binds to an extracellular domain of human CXCR3A, has an activity of blocking a CXCR3A-dependent cell function, and does not specifically bind to a human vascular endothelial cell.

An antibody according to an embodiment includes:
a heavy chain CDR1 including an amino acid sequence represented by SEQ ID NO: 1, 11, 21, 31, 41, 51, 61, 71, 81, 91, 101, 111, 121, 131, 141, 151, 161, 171, 181, 191, 201, 211, 221, 231, 241, 251, 261, or 271;
a heavy chain CDR2 including an amino acid sequence represented by SEQ ID NO: 2, 12, 22, 32, 42, 52, 62, 72, 82, 92, 102, 112, 122, 132, 142, 152, 162, 172, 182, 192, 202, 212, 222, 232, 242, 252, 262, or 272;
a heavy chain CDR3 including an amino acid sequence represented by SEQ ID NO: 3, 13, 23, 33, 43, 53, 63, 73, 83, 93, 103, 113, 123, 133, 143, 153, 163, 173, 183, 193, 203, 213, 223, 233, 243, 253, 263, or 273;
a light chain CDR1 including an amino acid sequence represented by SEQ ID NO: 4, 14, 24, 34, 44, 54, 64, 74, 84, 94, 104, 114, 124, 134, 144, 154, 164, 174, 184, 194, 204, 214, 224, 234, 244, 254, 264, or 274;
a light chain CDR2 including an amino acid sequence represented by SEQ ID NO: 5, 15, 25, 35, 45, 55, 65, 75, 85, 95, 105, 115, 125, 135, 145, 155, 165, 175, 185, 195, 205, 215, 225, 235, 245, 255, 265, or 275; and
a light chain CDR3 including an amino acid sequence represented by SEQ ID NO: 6, 16, 26, 36, 46, 56, 66, 76, 86, 96, 106, 116, 126, 136, 146, 156, 166, 176, 186, 196, 206, 216, 226, 236, 246, 256, 266, or 276.

An antibody according to an embodiment satisfies any one of the following (A1) to (A28):
(A1) including a heavy chain CDR1 including an amino acid sequence represented by SEQ ID NO: 1, a heavy chain CDR2 including an amino acid sequence represented by SEQ ID NO: 2, and a heavy chain CDR3 including an amino acid sequence represented by SEQ ID NO: 3;
(A2) including a heavy chain CDR1 including an amino acid sequence represented by SEQ ID NO: 11, a heavy chain CDR2 including an amino acid sequence represented by SEQ ID NO: 12, and a heavy chain CDR3 including an amino acid sequence represented by SEQ ID NO: 13;
(A3) including a heavy chain CDR1 including an amino acid sequence represented by SEQ ID NO: 21, a heavy chain CDR2 including an amino acid sequence represented by SEQ ID NO: 22, and a heavy chain CDR3 including an amino acid sequence represented by SEQ ID NO: 23;
(A4) including a heavy chain CDR1 including an amino acid sequence represented by SEQ ID NO: 31, a heavy chain CDR2 including an amino acid sequence represented by SEQ ID NO: 32, and a heavy chain CDR3 including an amino acid sequence represented by SEQ ID NO: 33;
(A5) including a heavy chain CDR1 including an amino acid sequence represented by SEQ ID NO: 41, a heavy chain CDR2 including an amino acid sequence represented by SEQ ID NO: 42, and a heavy chain CDR3 including an amino acid sequence represented by SEQ ID NO: 43;
(A6) including a heavy chain CDR1 including an amino acid sequence represented by SEQ ID NO: 51, a heavy chain CDR2 including an amino acid sequence represented by SEQ ID NO: 52, and a heavy chain CDR3 including an amino acid sequence represented by SEQ ID NO: 53;
(A7) including a heavy chain CDR1 including an amino acid sequence represented by SEQ ID NO: 61, a heavy chain CDR2 including an amino acid sequence represented by SEQ ID NO: 62, and a heavy chain CDR3 including an amino acid sequence represented by SEQ ID NO: 63;
(A8) including a heavy chain CDR1 including an amino acid sequence represented by SEQ ID NO: 71, a heavy chain CDR2 including an amino acid sequence represented by SEQ ID NO: 72, and a heavy chain CDR3 including an amino acid sequence represented by SEQ ID NO: 73;
(A9) including a heavy chain CDR1 including an amino acid sequence represented by SEQ ID NO: 81, a heavy chain CDR2 including an amino acid sequence represented by SEQ ID NO: 82, and a heavy chain CDR3 including an amino acid sequence represented by SEQ ID NO: 83;
(A10) including a heavy chain CDR1 including an amino acid sequence represented by SEQ ID NO: 91, a heavy chain CDR2 including an amino acid sequence represented by SEQ ID NO: 92, and a heavy chain CDR3 including an amino acid sequence represented by SEQ ID NO: 93;
(A11) including a heavy chain CDR1 including an amino acid sequence represented by SEQ ID NO: 101, a heavy chain CDR2 including an amino acid sequence represented by SEQ ID NO: 102, and a heavy chain CDR3 including an amino acid sequence represented by SEQ ID NO: 103;
(A12) including a heavy chain CDR1 including an amino acid sequence represented by SEQ ID NO: 111, a heavy chain CDR2 including an amino acid sequence represented by SEQ ID NO: 112, and a heavy chain CDR3 including an amino acid sequence represented by SEQ ID NO: 113;
(A13) including a heavy chain CDR1 including an amino acid sequence represented by SEQ ID NO: 121, a heavy chain CDR2 including an amino acid sequence represented by SEQ ID NO: 122, and a heavy chain CDR3 including an amino acid sequence represented by SEQ ID NO: 123;
(A14) including a heavy chain CDR1 including an amino acid sequence represented by SEQ ID NO: 131, a heavy chain CDR2 including an amino acid sequence represented by SEQ ID NO: 132, and a heavy chain CDR3 including an amino acid sequence represented by SEQ ID NO: 133;
(A15) including a heavy chain CDR1 including an amino acid sequence represented by SEQ ID NO: 141, a heavy chain CDR2 including an amino acid sequence represented by SEQ ID NO: 142, and a heavy chain CDR3 including an amino acid sequence represented by SEQ ID NO: 143;
(A16) including a heavy chain CDR1 including an amino acid sequence represented by SEQ ID NO: 151, a heavy chain CDR2 including an amino acid sequence represented by SEQ ID NO: 152, and a heavy chain CDR3 including an amino acid sequence represented by SEQ ID NO: 153;
(A17) including a heavy chain CDR1 including an amino acid sequence represented by SEQ ID NO: 161, a heavy chain CDR2 including an amino acid sequence represented by SEQ ID NO: 162, and a heavy chain CDR3 including an amino acid sequence represented by SEQ ID NO: 163;
(A18) including a heavy chain CDR1 including an amino acid sequence represented by SEQ ID NO: 171, a heavy chain CDR2 including an amino acid sequence represented by SEQ ID NO: 172, and a heavy chain CDR3 including an amino acid sequence represented by SEQ ID NO: 173;
(A19) including a heavy chain CDR1 including an amino acid sequence represented by SEQ ID NO: 181, a heavy chain CDR2 including an amino acid sequence represented by SEQ ID NO: 182, and a heavy chain CDR3 including an amino acid sequence represented by SEQ ID NO: 183;
(A20) including a heavy chain CDR1 including an amino acid sequence represented by SEQ ID NO: 191, a heavy chain CDR2 including an amino acid sequence represented by SEQ ID NO: 192, and a heavy chain CDR3 including an amino acid sequence represented by SEQ ID NO: 193;
(A21) including a heavy chain CDR1 including an amino acid sequence represented by SEQ ID NO: 201, a heavy chain CDR2 including an amino acid sequence represented by SEQ ID NO: 202, and a heavy chain CDR3 including an amino acid sequence represented by SEQ ID NO: 203;
(A22) including a heavy chain CDR1 including an amino acid sequence represented by SEQ ID NO: 211, a heavy chain CDR2 including an amino acid sequence represented by SEQ ID NO: 212, and a heavy chain CDR3 including an amino acid sequence represented by SEQ ID NO: 213;
(A23) including a heavy chain CDR1 including an amino acid sequence represented by SEQ ID NO: 221, a heavy chain CDR2 including an amino acid sequence represented by SEQ ID NO: 222, and a heavy chain CDR3 including an amino acid sequence represented by SEQ ID NO: 223;
(A24) including a heavy chain CDR1 including an amino acid sequence represented by SEQ ID NO: 231, a heavy chain CDR2 including an amino acid sequence represented by SEQ ID NO: 232, and a heavy chain CDR3 including an amino acid sequence represented by SEQ ID NO: 233;
(A25) including a heavy chain CDR1 including an amino acid sequence represented by SEQ ID NO: 241, a heavy chain CDR2 including an amino acid sequence represented by SEQ ID NO: 242, and a heavy chain CDR3 including an amino acid sequence represented by SEQ ID NO: 243;
(A26) including a heavy chain CDR1 including an amino acid sequence represented by SEQ ID NO: 251, a heavy chain CDR2 including an amino acid sequence represented by SEQ ID NO: 252, and a heavy chain CDR3 including an amino acid sequence represented by SEQ ID NO: 253;
(A27) including a heavy chain CDR1 including an amino acid sequence represented by SEQ ID NO: 261, a heavy chain CDR2 including an amino acid sequence represented by SEQ ID NO: 262, and a heavy chain CDR3 including an amino acid sequence represented by SEQ ID NO: 263; and
(A28) including a heavy chain CDR1 including an amino acid sequence represented by SEQ ID NO: 271, a heavy chain CDR2 including an amino acid sequence represented by SEQ ID NO: 272, and a heavy chain CDR3 including an amino acid sequence represented by SEQ ID NO: 273.

An antibody according to an embodiment satisfies any one of the following (B1) to (B28):
(B1) including a light chain CDR1 including an amino acid sequence represented by SEQ ID NO: 4, a light chain CDR2 including an amino acid sequence represented by SEQ ID NO: 5, and a light chain CDR3 including an amino acid sequence represented by SEQ ID NO: 6;
(B2) including a light chain CDR1 including an amino acid sequence represented by SEQ ID NO: 14, a light chain CDR2 including an amino acid sequence represented by SEQ ID NO: 15, and a light chain CDR3 including an amino acid sequence represented by SEQ ID NO: 16;
(B3) including a light chain CDR1 including an amino acid sequence represented by SEQ ID NO: 24, a light chain CDR2 including an amino acid sequence represented by SEQ ID NO: 25, and a light chain CDR3 including an amino acid sequence represented by SEQ ID NO: 26;
(B4) including a light chain CDR1 including an amino acid sequence represented by SEQ ID NO: 34, a light chain CDR2 including an amino acid sequence represented by SEQ ID NO: 35, and a light chain CDR3 including an amino acid sequence represented by SEQ ID NO: 36;
(B5) including a light chain CDR1 including an amino acid sequence represented by SEQ ID NO: 44, a light chain CDR2 including an amino acid sequence represented by SEQ ID NO: 45, and a light chain CDR3 including an amino acid sequence represented by SEQ ID NO: 46;
(B6) including a light chain CDR1 including an amino acid sequence represented by SEQ ID NO: 54, a light chain CDR2 including an amino acid sequence represented by SEQ ID NO: 55, and a light chain CDR3 including an amino acid sequence represented by SEQ ID NO: 56;
(B7) including a light chain CDR1 including an amino acid sequence represented by SEQ ID NO: 64, a light chain CDR2 including an amino acid sequence represented by SEQ ID NO: 65, and a light chain CDR3 including an amino acid sequence represented by SEQ ID NO: 66;
(B8) including a light chain CDR1 including an amino acid sequence represented by SEQ ID NO: 74, a light chain CDR2 including an amino acid sequence represented by SEQ ID NO: 75, and a light chain CDR3 including an amino acid sequence represented by SEQ ID NO: 76;
(B9) including a light chain CDR1 including an amino acid sequence represented by SEQ ID NO: 84, a light chain CDR2 including an amino acid sequence represented by SEQ ID NO: 85, and a light chain CDR3 including an amino acid sequence represented by SEQ ID NO: 86;
(B10) including a light chain CDR1 including an amino acid sequence represented by SEQ ID NO: 94, a light chain CDR2 including an amino acid sequence represented by SEQ ID NO: 95, and a light chain CDR3 including an amino acid sequence represented by SEQ ID NO: 96;
(B11) including a light chain CDR1 including an amino acid sequence represented by SEQ ID NO: 104, a light chain CDR2 including an amino acid sequence represented by SEQ ID NO: 105, and a light chain CDR3 including an amino acid sequence represented by SEQ ID NO: 106;
(B12) including a light chain CDR1 including an amino acid sequence represented by SEQ ID NO: 114, a light chain CDR2 including an amino acid sequence represented by SEQ ID NO: 115, and a light chain CDR3 including an amino acid sequence represented by SEQ ID NO: 116;
(B13) including a light chain CDR1 including an amino acid sequence represented by SEQ ID NO: 124, a light chain CDR2 including an amino acid sequence represented by SEQ ID NO: 125, and a light chain CDR3 including an amino acid sequence represented by SEQ ID NO: 126;
(B14) including a light chain CDR1 including an amino acid sequence represented by SEQ ID NO: 134, a light chain CDR2 including an amino acid sequence represented by SEQ ID NO: 135, and a light chain CDR3 including an amino acid sequence represented by SEQ ID NO: 136;
(B15) including a light chain CDR1 including an amino acid sequence represented by SEQ ID NO: 144, a light chain CDR2 including an amino acid sequence represented by SEQ ID NO: 145, and a light chain CDR3 including an amino acid sequence represented by SEQ ID NO: 146;
(B16) including a light chain CDR1 including an amino acid sequence represented by SEQ ID NO: 154, a light chain CDR2 including an amino acid sequence represented by SEQ ID NO: 155, and a light chain CDR3 including an amino acid sequence represented by SEQ ID NO: 156;
(B17) including a light chain CDR1 including an amino acid sequence represented by SEQ ID NO: 164, a light chain CDR2 including an amino acid sequence represented by SEQ ID NO: 165, and a light chain CDR3 including an amino acid sequence represented by SEQ ID NO: 166;
(B18) including a light chain CDR1 including an amino acid sequence represented by SEQ ID NO: 174, a light chain CDR2 including an amino acid sequence represented by SEQ ID NO: 175, and a light chain CDR3 including an amino acid sequence represented by SEQ ID NO: 176;
(B19) including a light chain CDR1 including an amino acid sequence represented by SEQ ID NO: 184, a light chain CDR2 including an amino acid sequence represented by SEQ ID NO: 185, and a light chain CDR3 including an amino acid sequence represented by SEQ ID NO: 186;
(B20) including a light chain CDR1 including an amino acid sequence represented by SEQ ID NO: 194, a light chain CDR2 including an amino acid sequence represented by SEQ ID NO: 195, and a light chain CDR3 including an amino acid sequence represented by SEQ ID NO: 196;
(B21) including a light chain CDR1 including an amino acid sequence represented by SEQ ID NO: 204, a light chain CDR2 including an amino acid sequence represented by SEQ ID NO: 205, and a light chain CDR3 including an amino acid sequence represented by SEQ ID NO: 206;
(B22) including a light chain CDR1 including an amino acid sequence represented by SEQ ID NO: 214, a light chain CDR2 including an amino acid sequence represented by SEQ ID NO: 215, and a light chain CDR3 including an amino acid sequence represented by SEQ ID NO: 216;
(B23) including a light chain CDR1 including an amino acid sequence represented by SEQ ID NO: 224, a light chain CDR2 including an amino acid sequence represented by SEQ ID NO: 225, and a light chain CDR3 including an amino acid sequence represented by SEQ ID NO: 226;
(B24) including a light chain CDR1 including an amino acid sequence represented by SEQ ID NO: 234, a light chain CDR2 including an amino acid sequence represented by SEQ ID NO: 235, and a light chain CDR3 including an amino acid sequence represented by SEQ ID NO: 236;
(B25) including a light chain CDR1 including an amino acid sequence represented by SEQ ID NO: 244, a light chain CDR2 including an amino acid sequence represented by SEQ ID NO: 245, and a light chain CDR3 including an amino acid sequence represented by SEQ ID NO: 246;
(B26) including a light chain CDR1 including an amino acid sequence represented by SEQ ID NO: 254, a light chain CDR2 including an amino acid sequence represented by SEQ ID NO: 255, and a light chain CDR3 including an amino acid sequence represented by SEQ ID NO: 256;
(B27) including a light chain CDR1 including an amino acid sequence represented by SEQ ID NO: 264, a light chain CDR2 including an amino acid sequence represented by SEQ ID NO: 265, and a light chain CDR3 including an amino acid sequence represented by SEQ ID NO: 266; and
(B28) including a light chain CDR1 including an amino acid sequence represented by SEQ ID NO: 274, a light chain CDR2 including an amino acid sequence represented by SEQ ID NO: 275, and a light chain CDR3 including an amino acid sequence represented by SEQ ID NO: 276.

In an antibody according to an embodiment, the heavy chain CDRs 1 to 3 and the light chain CDRs 1 to 3 satisfy any one of the above (AB1) to (AB9) and (AB12) to (AB27).

An antibody according to an embodiment satisfies any one of the following (AB10), (AB11), and (AB28):
(AB10) including a heavy chain CDR1 including an amino acid sequence represented by SEQ ID NO: 91, a heavy chain CDR2 including an amino acid sequence represented by SEQ ID NO: 92, a heavy chain CDR3 including an amino acid sequence represented by SEQ ID NO: 93, a light chain CDR1 including an amino acid sequence represented by SEQ ID NO: 94, a light chain CDR2 including an amino acid sequence represented by SEQ ID NO: 95, and a light chain CDR3 including an amino acid sequence represented by SEQ ID NO: 96;
(AB11) including a heavy chain CDR1 including an amino acid sequence represented by SEQ ID NO: 101, a heavy chain CDR2 including an amino acid sequence represented by SEQ ID NO: 102, a heavy chain CDR3 including an amino acid sequence represented by SEQ ID NO: 103, a light chain CDR1 including an amino acid sequence represented by SEQ ID NO: 104, a light chain CDR2 including an amino acid sequence represented by SEQ ID NO: 105, and a light chain CDR3 including an amino acid sequence represented by SEQ ID NO: 106; and
(AB28) including a heavy chain CDR1 including an amino acid sequence represented by SEQ ID NO: 271, a heavy chain CDR2 including an amino acid sequence represented by SEQ ID NO: 272, a heavy chain CDR3 including an amino acid sequence represented by SEQ ID NO: 273, a light chain CDR1 including an amino acid sequence represented by SEQ ID NO: 274, a light chain CDR2 including an amino acid sequence represented by SEQ ID NO: 275, and a light chain CDR3 including an amino acid sequence represented by SEQ ID NO: 276.

In an antibody according to an embodiment, the heavy chain variable region and the light chain variable region satisfy any one of the above (C1) to (C9) and (C12) to (C27).

An antibody according to an embodiment satisfies any one of the following (C10), (C11), and (C28):
(C10) including a heavy chain variable region including an amino acid sequence represented by SEQ ID NO: 97 and a light chain variable region including an amino acid sequence represented by SEQ ID NO: 99;
(C11) including a heavy chain variable region including an amino acid sequence represented by SEQ ID NO: 107 and a light chain variable region including an amino acid sequence represented by SEQ ID NO: 109; and
(C28) including a heavy chain variable region including an amino acid sequence represented by SEQ ID NO: 277 and a light chain variable region including an amino acid sequence represented by SEQ ID NO: 279.

The heavy chain variable region (SEQ ID NO: 7) identified in (C1) includes the heavy chain CDRs 1 to 3 (SEQ ID NOs: 1 to 3) identified in (A1) or (AB1). The light chain variable region (SEQ ID NO: 9) identified in (C1) includes the light chain CDRs 1 to 3 (SEQ ID NOs: 4 to 6) identified in (B1) or (AB1). Examples of an antibody satisfying (A1), (B1), (AB1), or (C1) include "201001-1-F" described in Examples below.

The heavy chain variable region (SEQ ID NO: 17) identified in (C2) includes the heavy chain CDRs 1 to 3 (SEQ ID NOs: 11 to 13) identified in (A2) or (AB2). The light chain variable region (SEQ ID NO: 19) identified in (C2) includes the light chain CDRs 1 to 3 (SEQ ID NOs: 14 to 16) identified in (B2) or (AB2). Examples of an antibody satisfying (A2), (B2), (AB2), or (C2) include "201001-5-B" described in Examples below.

The heavy chain variable region (SEQ ID NO: 27) identified in (C3) includes the heavy chain CDRs 1 to 3 (SEQ ID NOs: 21 to 23) identified in (A3) or (AB3). The light chain variable region (SEQ ID NO: 29) identified in (C3) includes the light chain CDRs 1 to 3 (SEQ ID NOs: 24 to 26) identified in (B3) or (AB3). Examples of an antibody satisfying (A3), (B3), (AB3), or (C3) include "201001-2-C" described in Examples below.

The heavy chain variable region (SEQ ID NO: 37) identified in (C4) includes the heavy chain CDRs 1 to 3 (SEQ ID NOs: 31 to 33) identified in (A4) or (AB4). The light chain variable region (SEQ ID NO: 39) identified in (C4) includes the light chain CDRs 1 to 3 (SEQ ID NOs: 34 to 36) identified in (B4) or (AB4). Examples of an antibody satisfying (A4), (B4), (AB4), or (C4) include "201001-4-E" described in Examples below.

The heavy chain variable region (SEQ ID NO: 47) identified in (C5) includes the heavy chain CDRs 1 to 3 (SEQ ID NOs: 41 to 43) identified in (A5) or (AB5). The light chain variable region (SEQ ID NO: 49) identified in (C5) includes the light chain CDRs 1 to 3 (SEQ ID NOs: 44 to 46) identified in (B5) or (AB5). Examples of an antibody satisfying (A5), (B5), (AB5), or (C5) include "201001-1-C" described in Examples below.

The heavy chain variable region (SEQ ID NO: 57) identified in (C6) includes the heavy chain CDRs 1 to 3 (SEQ ID NOs: 51 to 53) identified in (A6) or (AB6). The light chain variable region (SEQ ID NO: 59) identified in (C6) includes the light chain CDRs 1 to 3 (SEQ ID NOs: 54 to 56) identified in (B6) or (AB6). Examples of an antibody satisfying (A6), (B6), (AB6), or (C6) include "201001-5-A" described in Examples below.

The heavy chain variable region (SEQ ID NO: 67) identified in (C7) includes the heavy chain CDRs 1 to 3 (SEQ ID NOs: 61 to 63) identified in (A7) or (AB7). The light chain variable region (SEQ ID NO: 69) identified in (C7) includes the light chain CDRs 1 to 3 (SEQ ID NOs: 64 to 66) identified in (B7) or (AB7). Examples of an antibody satisfying (A7), (B7), (AB7), or (C7) include "201009-1-D" described in Examples below.

The heavy chain variable region (SEQ ID NO: 77) identified in (C8) includes the heavy chain CDRs 1 to 3 (SEQ ID NOs: 71 to 73) identified in (A8) or (AB8). The light chain variable region (SEQ ID NO: 79) identified in (C8) includes the light chain CDRs 1 to 3 (SEQ ID NOs: 74 to 76) identified in (B8) or (AB8). Examples of an antibody satisfying (A8), (B8), (AB8), or (C8) include "201009-5-F" described in Examples below.

The heavy chain variable region (SEQ ID NO: 87) identified in (C9) includes the heavy chain CDRs 1 to 3 (SEQ ID NOs: 81 to 83) identified in (A9) or (AB9). The light chain variable region (SEQ ID NO: 89) identified in (C9) includes the light chain CDRs 1 to 3 (SEQ ID NOs: 84 to 86) identified in (B9) or (AB9). Examples of an antibody satisfying (A9), (B9), (AB9), or (C9) include "201112-1-C" described in Examples below.

The heavy chain variable region (SEQ ID NO: 97) identified in (C10) includes the heavy chain CDRs 1 to 3 (SEQ ID NOs: 91 to 93) identified in (A10) or (AB10). The light chain variable region (SEQ ID NO: 99) identified in (C10) includes the light chain CDRs 1 to 3 (SEQ ID NOs: 94 to 96) identified in (B10) or (AB10). Examples of an antibody satisfying (A10), (B10), (AB10), or (C10) include "13B4" described in Examples below.

The heavy chain variable region (SEQ ID NO: 107) identified in (C11) includes the heavy chain CDRs 1 to 3 (SEQ ID NOs: 101 to 103) identified in (A11) or (AB11). The light chain variable region (SEQ ID NO: 109) identified in (C11) includes the light chain CDRs 1 to 3 (SEQ ID NOs: 104 to 106) identified in (B11) or (AB11). Examples of an antibody satisfying (A11), (B11), (AB11), or (C11) include "11F11" described in Examples below.

The heavy chain variable region (SEQ ID NO: 117) identified in (C12) includes the heavy chain CDRs 1 to 3 (SEQ ID NOs: 111 to 113) identified in (A12) or (AB12). The light chain variable region (SEQ ID NO: 119) identified in (C12) includes the light chain CDRs 1 to 3 (SEQ ID NOs: 114 to 116) identified in (B12) or (AB12). Examples of an antibody satisfying (A12), (B12), (AB12), or (C12) include "201006-4-H" described in Examples below.

The heavy chain variable region (SEQ ID NO: 127) identified in (C13) includes the heavy chain CDRs 1 to 3 (SEQ ID NOs: 121 to 123) identified in (A13) or (AB13). The light chain variable region (SEQ ID NO: 129) identified in (C13) includes the light chain CDRs 1 to 3 (SEQ ID NOs: 124 to 126) identified in (B13) or (AB13). Examples of an antibody satisfying (A13), (B13), (AB13), or (C13) include "201006-4-F" described in Examples below.

The heavy chain variable region (SEQ ID NO: 137) identified in (C14) includes the heavy chain CDRs 1 to 3 (SEQ ID NOs: 131 to 133) identified in (A14) or (AB14). The light chain variable region (SEQ ID NO: 139) identified in (C14) includes the light chain CDRs 1 to 3 (SEQ ID NOs: 134 to 136) identified in (B14) or (AB14). Examples of an antibody satisfying (A14), (B14), (AB14), or (C14) include "201006-5-A" described in Examples below.

The heavy chain variable region (SEQ ID NO: 147) identified in (C15) includes the heavy chain CDRs 1 to 3 (SEQ ID NOs: 141 to 143) identified in (A15) or (AB15). The light chain variable region (SEQ ID NO: 149) identified in (C15) includes the light chain CDRs 1 to 3 (SEQ ID NOs: 144 to 146) identified in (B15) or (AB15). Examples of an antibody satisfying (A15), (B15), (AB15), or (C15) include "201006-4-A" described in Examples below.

The heavy chain variable region (SEQ ID NO: 157) identified in (C16) includes the heavy chain CDRs 1 to 3 (SEQ ID NOs: 151 to 153) identified in (A16) or (AB16). The light chain variable region (SEQ ID NO: 159) identified in (C16) includes the light chain CDRs 1 to 3 (SEQ ID NOs: 154 to 156) identified in (B16) or (AB16). Examples of an antibody satisfying (A16), (B16), (AB16), or (C16) include "201006-1-H" described in Examples below.

The heavy chain variable region (SEQ ID NO: 167) identified in (C17) includes the heavy chain CDRs 1 to 3 (SEQ ID NOs: 161 to 163) identified in (A17) or (AB17). The light chain variable region (SEQ ID NO: 169) identified in (C17) includes the light chain CDRs 1 to 3 (SEQ ID NOs: 164 to 166) identified in (B17) or (AB17). Examples of an antibody satisfying (A17), (B17), (AB17), or (C17) include "201006-3-D" described in Examples below.

The heavy chain variable region (SEQ ID NO: 177) identified in (C18) includes the heavy chain CDRs 1 to 3 (SEQ ID NOs: 171 to 173) identified in (A18) or (AB18). The light chain variable region (SEQ ID NO: 179) identified in (C18) includes the light chain CDRs 1 to 3 (SEQ ID NOs: 174 to 176) identified in (B18) or (AB18). Examples of an antibody satisfying (A18), (B18), (AB18), or (C18) include "201006-1-F" described in Examples below.

The heavy chain variable region (SEQ ID NO: 187) identified in (C19) includes the heavy chain CDRs 1 to 3 (SEQ ID NOs: 181 to 183) identified in (A19) or (AB19). The light chain variable region (SEQ ID NO: 189) identified in (C19) includes the light chain CDRs 1 to 3 (SEQ ID NOs: 184 to 186) identified in (B19) or (AB19). Examples of an antibody satisfying (A19), (B19), (AB19), or (C19) include "201006-4-G" described in Examples below.

The heavy chain variable region (SEQ ID NO: 197) identified in (C20) includes the heavy chain CDRs 1 to 3 (SEQ ID NOs: 191 to 193) identified in (A20) or (AB20). The light chain variable region (SEQ ID NO: 199) identified in (C20) includes the light chain CDRs 1 to 3 (SEQ ID NOs: 194 to 196) identified in (B20) or (AB20). Examples of an antibody satisfying (A20), (B20), (AB20), or (C20) include "201006-7-A" described in Examples below.

The heavy chain variable region (SEQ ID NO: 207) identified in (C21) includes the heavy chain CDRs 1 to 3 (SEQ ID NOs: 201 to 203) identified in (A21) or (AB21). The light chain variable region (SEQ ID NO: 209) identified in (C21) includes the light chain CDRs 1 to 3 (SEQ ID NOs: 204 to 206) identified in (B21) or (AB21). Examples of an antibody satisfying (A21), (B21), (AB21), or (C21) include "201006-2-D" described in Examples below.

The heavy chain variable region (SEQ ID NO: 217) identified in (C22) includes the heavy chain CDRs 1 to 3 (SEQ ID NOs: 211 to 213) identified in (A22) or (AB22). The light chain variable region (SEQ ID NO: 219) identified in (C22) includes the light chain CDRs 1 to 3 (SEQ ID NOs: 214 to 216) identified in (B22) or (AB22). Examples of an antibody satisfying (A22), (B22), (AB22), or (C22) include "201006-2-C" described in Examples below.

The heavy chain variable region (SEQ ID NO: 227) identified in (C23) includes the heavy chain CDRs 1 to 3 (SEQ ID NOs: 221 to 223) identified in (A23) or (AB23). The light chain variable region (SEQ ID NO: 229) identified in (C23) includes the light chain CDRs 1 to 3 (SEQ ID NOs: 224 to 226) identified in (B23) or (AB23). Examples of an antibody satisfying (A23), (B23), (AB23), or (C23) include "201006-3-H" described in Examples below.

The heavy chain variable region (SEQ ID NO: 237) identified in (C24) includes the heavy chain CDRs 1 to 3 (SEQ ID NOs: 231 to 233) identified in (A24) or (AB24). The light chain variable region (SEQ ID NO: 239) identified in (C24) includes the light chain CDRs 1 to 3 (SEQ ID NOs: 234 to 236) identified in (B24) or (AB24). Examples of an antibody satisfying (A24), (B24), (AB24), or (C24) include "201006-5-F" described in Examples below.

The heavy chain variable region (SEQ ID NO: 247) identified in (C25) includes the heavy chain CDRs 1 to 3 (SEQ ID NOs: 241 to 243) identified in (A25) or (AB25). The light chain variable region (SEQ ID NO: 249) identified in (C25) includes the light chain CDRs 1 to 3 (SEQ ID NOs: 244 to 246) identified in (B25) or (AB25). Examples of an antibody satisfying (A25), (B25), (AB25), or (C25) include "201006-7-G" described in Examples below.

The heavy chain variable region (SEQ ID NO: 257) identified in (C26) includes the heavy chain CDRs 1 to 3 (SEQ ID NOs: 251 to 253) identified in (A26) or (AB26). The light chain variable region (SEQ ID NO: 259) identified in (C26) includes the light chain CDRs 1 to 3 (SEQ ID NOs: 254 to 256) identified in (B26) or (AB26). Examples of an antibody satisfying (A26), (B26), (AB26), or (C26) include "201009-2-C" described in Examples below.

The heavy chain variable region (SEQ ID NO: 267) identified in (C27) includes the heavy chain CDRs 1 to 3 (SEQ ID NOs: 261 to 263) identified in (A27) or (AB27). The light chain variable region (SEQ ID NO: 269) identified in (C27) includes the light chain CDRs 1 to 3 (SEQ ID NOs: 264 to 266) identified in (B27) or (AB27). Examples of an antibody satisfying (A27), (B27), (AB27), or (C27) include "201009-3-H" described in Examples below.

The heavy chain variable region (SEQ ID NO: 277) identified in (C28) includes the heavy chain CDRs 1 to 3 (SEQ ID NOs: 271 to 273) identified in (A28) or (AB28). The light chain variable region (SEQ ID NO: 279) identified in (C28) includes the light chain CDRs 1 to 3 (SEQ ID NOs: 274 to 276) identified in (B28) or (AB28). Examples of an antibody satisfying (A28), (B28), (AB28), or (C28) include "201009-1-C" described in Examples below.

The amino acid sequences of SEQ ID NO: 1, SEQ ID NO: 31, SEQ ID NO: 41, SEQ ID NO: 51, SEQ ID NO: 61, SEQ ID NO: 71, SEQ ID NO: 111, SEQ ID NO: 121, SEQ ID NO: 131, SEQ ID NO: 161, and SEQ ID NO: 251 are the same. The amino acid sequences of SEQ ID NO: 81 and SEQ ID NO: 101 are the same. The amino acid sequences of SEQ ID NO: 171 and SEQ ID NO: 181 are the same. The amino acid sequences of SEQ ID NO: 191, SEQ ID NO: 201, and SEQ ID NO: 211 are the same. The amino acid sequences of SEQ ID NO: 221, SEQ ID NO: 231, SEQ ID NO: 241, and SEQ ID NO: 261 are the same.

The amino acid sequences of SEQ ID NO: 2, SEQ ID NO: 12, SEQ ID NO: 72, SEQ ID NO: 132, SEQ ID NO: 142, SEQ ID NO: 162, SEQ ID NO: 182, SEQ ID NO: 192, and SEQ ID NO: 222 are the same. The amino acid sequences of SEQ ID NO: 22, SEQ ID NO: 32, SEQ ID NO: 62, and SEQ ID NO: 202 are the same. The amino acid sequences of SEQ ID NO: 42 and SEQ ID NO: 52 are the same. The amino acid sequences of SEQ ID NO: 152 and SEQ ID NO: 212 are the same. The amino acid sequences of SEQ ID NO: 172, SEQ ID NO: 232, and SEQ ID NO: 262 are the same.

The amino acid sequences of SEQ ID NO: 3 and SEQ ID NO: 13 are the same. The amino acid sequences of SEQ ID NO: 63, SEQ ID NO: 123, SEQ ID NO: 153, SEQ ID NO: 163, SEQ ID NO: 173, SEQ ID NO: 183, SEQ ID NO: 193, SEQ ID NO: 203, SEQ ID NO: 213, and SEQ ID NO: 243 are the same. The amino acid sequences of SEQ ID NO: 83 and SEQ ID NO: 93 are the same. The amino acid sequences of SEQ ID NO: 113, SEQ ID NO: 133, SEQ ID NO: 143, SEQ ID NO: 223, SEQ ID NO: 233, SEQ ID NO: 253, and SEQ ID NO: 263 are the same.

The amino acid sequences of SEQ ID NO: 44 and SEQ ID NO: 54 are the same. The amino acid sequences of SEQ ID NO: 64 and SEQ ID NO: 124 are the same. The amino acid sequences of SEQ ID NO: 74, SEQ ID NO: 144, SEQ ID NO: 224, SEQ ID NO: 254, and SEQ ID NO: 264 are the same. The amino acid sequences of SEQ ID NO: 84, SEQ ID NO: 94, and SEQ ID NO: 104 are the same. The amino acid sequences of SEQ ID NO: 154 and SEQ ID NO: 164 are the same. The amino acid sequences of SEQ ID NO: 174, SEQ ID NO: 184, SEQ ID NO: 204, and SEQ ID NO: 214 are the same.

The amino acid sequences of SEQ ID NO: 5 and SEQ ID NO: 15 are the same. The amino acid sequences of SEQ ID NO: 25, SEQ ID NO: 145, SEQ ID NO: 175, SEQ ID NO: 185, and SEQ ID NO: 265 are the same. The amino acid sequences of SEQ ID NO: 45 and SEQ ID NO: 55 are the same. The amino acid sequences of SEQ ID NO: 65, SEQ ID NO: 125, and SEQ ID NO: 135 are the same. The amino acid sequences of SEQ ID NO: 75, SEQ ID NO: 225, and SEQ ID NO: 255 are the same. The amino acid sequences of SEQ ID NO: 85, SEQ ID NO: 95, and SEQ ID NO: 105 are the same. The amino acid sequences of SEQ ID NO: 155, SEQ ID NO: 165, and SEQ ID NO: 245 are the same. The amino acid sequences of SEQ ID NO: 195, SEQ ID NO: 205, and SEQ ID NO: 215 are the same.

The amino acid sequences of SEQ ID NO: 6, SEQ ID NO: 16, SEQ ID NO: 46, and SEQ ID NO: 56 are the same. The amino acid sequences of SEQ ID NO: 66, SEQ ID NO: 76, SEQ ID NO: 136, SEQ ID NO: 196, SEQ ID NO: 206, SEQ ID NO: 216, SEQ ID NO: 226, SEQ ID NO: 246, and SEQ ID NO: 266 are the same. The amino acid sequences of SEQ ID NO: 96 and SEQ ID NO: 106 are the same. The amino acid sequences of SEQ ID NO: 126, SEQ ID NO: 176, and SEQ ID NO: 186 are the same. The amino acid sequences of SEQ ID NO: 146, SEQ ID NO: 236, and SEQ ID NO: 256 are the same. The amino acid sequences of SEQ ID NO: 156 and SEQ ID NO: 166 are the same.

The above antibody may be a functional fragment of an antibody. Herein, the "functional fragment of an antibody" refers to a partial fragment of an antibody (i.e. immunoglobulin), which retains at least one action against an antigen. Examples of the partial fragment include F(ab')2, Fab, Fv, disulfide-bonded Fv, single chain antibodies (scFv, VH-VL), VH, and polymers thereof, as well as a fusion thereof with a heavy chain CH3 region, and also include various CDRs such as CDR1, CDR2, and CDR3, and a linked assembly of these CDRs, as well as a fusion of these CDRs or the CDR linked assembly with a heavy chain CH3 region. That means, the antibody according to the present disclosure also includes partial fragments of the antibody as described above. A partial fragment of an antibody is referred to as an "antibody fragment" in some cases.

The above antibody may be a multispecific antibody. A multispecific antibody according to this embodiment has at least a first specific binding property of specifically binding to the extracellular domain of human CXCR3A, having an activity of blocking a CXCR3A-dependent cell function, and not specifically binding to a human vascular endothelial cell, and a second specific binding property different from the first specific binding property. The second specific binding property may be directed to human CXCR3 or to other target molecules. Examples of the multispecific antibody include a diabody, which is a type of bispecific antibody.

In an embodiment, the above multispecific antibody includes two or more (e.g., 2, 3, 4, 5, or more) antigen-binding domains. This multispecific antibody can bind to two or more CXCR3 molecules, for example, via the same or different epitopes. This multispecific antibody can, for example, bind to CXCR3 and at least one other antigen with high affinity. The antigen-binding portion of the antibody may include one or more antibody fragments that retain the ability to bind specifically to an antigen. These fragments may include heavy chain and/or light chain variable regions from a parent antibody or a variant of the parent antibody.

The class (isotype) of the above antibody is not particularly limited. For example, the antibody may belong to any class such as IgG, IgM, IgA, IgD, and IgE. Furthermore, the subclass of the above antibody is not particularly limited. For example, if the antibody belongs to the IgG class, the subclass thereof may be any of IgG1, IgG2, IgG3, IgG4, and the like.

In an embodiment, the above antibody has an activity of blocking a CXCR3A-dependent cell function. A CXCR3A-dependent cell function is a function induced by activation of proteins (such as trimeric G proteins and β-arrestins) that couple to an intracellular domain of CXCR3 and mediate intracellular signaling. Activation of CXCR3A-dependent cell function is induced by stimulation with a CXCR3 ligand. Examples include fluctuations in intracellular calcium ions, fluctuations in intracellular cyclic adenosine monophosphate (cAMP), GTP binding to the low-molecular-weight G protein Rho, cell chemotaxis, and receptor internalization.

In an embodiment, the above antibody does not specifically bind to a human vascular endothelial cell. As shown in the examples below, human vascular endothelial cells predominantly express CXCR3B.

The extracellular domain of CXCR3A to which the above antibody specifically binds may be any of the N-terminal domain, the extracellular first loop domain, the extracellular second loop domain, and the extracellular third loop domain. The above antibody may bind to only one of these extracellular domains or to two or more of them.

The present disclosure encompasses antibodies that are "functionally equivalent" to the anti-human CXCR3 antibodies described above. The functionally equivalent antibodies include antibodies having the same epitope as of the anti-human CXCR3 antibodies. For example, the epitopes of the 28 types of anti-human CXCR3 antibodies specifically described in the Examples below are analyzed by an epitope mapping method using a partial peptide of human CXCR3 or the like. Then, a synthetic peptide including the identified epitope can be used as an antigen to obtain an anti-human CXCR3 antibody that binds to the same epitope as the above 28 anti-human CXCR3 antibodies. Furthermore, the amino acid sequences of the heavy chain variable region and the light chain variable region of the obtained anti-human CXCR3 antibody can be determined to identify the amino acid sequences of the heavy chain CDRs 1 to 3 and the light chain CDRs 1 to 3.

As an example of the antibodies functionally equivalent to the above antibodies, the present disclosure encompasses an antibody that specifically binds to an extracellular domain of human CXCR3A, has an activity of blocking a CXCR3A-dependent cell function, and does not specifically bind to a human vascular endothelial cell, which has an amino acid sequence defined in any one of (C1) to (C28) with 1 to 10 amino acids substituted, added, or deleted, or which has an amino acid sequence having at least 90% sequence identity with the amino acid sequence defined in any one of (C1) to (C28). The number of substituted, added, or deleted amino acids described above is preferably 1 to 8, more preferably 1 to 5, and particularly preferably 1 to 3. The amino acid sequence identity described above is preferably at least 92%, more preferably at least 95%, and particularly preferably at least 97%.

Examples of a method for determining whether the epitopes are identical between two antibodies include a method using a competition experiment. For example, if the binding between the above 28 anti-human CXCR3 antibodies or functional fragments thereof as first antibodies and the receptor is competitively inhibited by a second antibody to be tested, the second antibody is considered to bind to the same epitope as the first antibody. As an example of the antibodies that are functionally equivalent to the above antibodies, the present disclosure encompasses a second antibody that specifically binds to an extracellular domain of human CXCR3A, has an activity of blocking a CXCR3A-dependent cell function, does not specifically bind to a human vascular endothelial cell, and competitively inhibits the binding between the above antibody (first antibody) and the receptor.

The CDRs and other regions (such as framework regions and constant regions) disclosed in this specification may be selected independently and combined in various combinations with other CDRs or framework regions of a specific antibody. In certain embodiments, VH and/or VL, CDR, and framework sequences may be present in any combination in an antibody that specifically binds to an extracellular domain of human CXCR3A, has an activity of blocking a CXCR3A-dependent cell function, and does not specifically bind to a human vascular endothelial cell.

### <Humanized Antibody>

The present disclosure encompasses the above antibody that is a humanized antibody. A humanized antibody refers to an antibody in which the CDRs are derived from a non-human animal and other regions (e.g., framework regions and constant regions) are derived from a human. Methods for constructing and producing humanized antibodies are described later.

### <Nucleic Acid>

The present disclosure encompasses a nucleic acid (DNA) encoding the above antibody. The nucleic acid includes, for example, a first nucleic acid encoding the heavy chain variable region and/or a second nucleic acid encoding the light chain variable region.

The heavy chain variable region encoded by the first nucleic acid includes, for example, a heavy chain CDR1 including the amino acid sequence represented by SEQ ID NO: 1, 11, 21, 31, 41, 51, 61, 71, 81, 91, 101, 111, 121, 131, 141, 151, 161, 171, 181, 191, 201, 211, 221, 231, 241, 251, 261, or 271; a heavy chain CDR2 including the amino acid sequence represented by SEQ ID NO: 2, 12, 22, 32, 42, 52, 62, 72, 82, 92, 102, 112, 122, 132, 142, 152, 162, 172, 182, 192, 202, 212, 222, 232, 242, 252, 262, or 272; and a heavy chain CDR3 including the amino acid sequence represented by SEQ ID NO: 3, 13, 23, 33, 43, 53, 63, 73, 83, 93, 103, 113, 123, 133, 143, 153, 163, 173, 183, 193, 203, 213, 223, 233, 243, 253, 263, or 273. The heavy chain variable region encoded by the first nucleic acid includes, for example, the heavy chain CDRs 1 to 3 identified in any one of (A1) to (A28) above. The heavy chain variable region encoded by the first nucleic acid is, for example, one identified in any one of (C1) to (C28) above.

The light chain variable region encoded by the second nucleic acid includes, for example, a light chain CDR1 including the amino acid sequence represented by SEQ ID NO: 4, 14, 24, 34, 44, 54, 64, 74, 84, 94, 104, 114, 124, 134, 144, 154, 164, 174, 184, 194, 204, 214, 224, 234, 244, 254, 264, or 274; a light chain CDR2 including the amino acid sequence represented by SEQ ID NO: 5, 15, 25, 35, 45, 55, 65, 75, 85, 95, 105, 115, 125, 135, 145, 155, 165, 175, 185, 195, 205, 215, 225, 235, 245, 255, 265, or 275; and a light chain CDR3 including the amino acid sequence represented by SEQ ID NO: 6, 16, 26, 36, 46, 56, 66, 76, 86, 96, 106, 116, 126, 136, 146, 156, 166, 176, 186, 196, 206, 216, 226, 236, 246, 256, 266, or 276. The light chain variable region encoded by the second nucleic acid includes, for example, the light chain CDRs 1 to 3 identified in any one of (B1) to (B28) above. The light chain variable region encoded by the second nucleic acid is, for example, one identified in any one of (C1) to (C28) above.

The above nucleic acid may be incorporated into a vector. The vector is selected as appropriate depending on the type or the like of a host cell into which the vector is introduced. Examples of the vector include a gene therapy vector. In this case, the vector itself can be directly administered into a living body.

### <Method for Producing Antibody>

The above antibody can be produced using a genetic recombination method. Specifically, a recombinant cell that expresses the above nucleic acid can be constructed to obtain the above antibody from a culture of the cell.

### <Construction and Preparation of Humanized Antibody>

A method for constructing and producing a humanized antibody is described. As an example, a method for constructing and producing a humanized antibody having the heavy chain CDRs 1 to 3 and the light chain CDRs 1 to 3 identified in (AB1) above is described. As DNA encoding each CDR, DNA encoding the amino acid sequences represented in SEQ ID NOs: 1 to 6 is first prepared. The DNA can be prepared by using a known method such as PCR. The DNA can also be prepared by chemical synthesis.

Next, these DNA fragments are used to prepare DNA encoding a variable region in which the heavy chain CDRs 1 to 3 are grafted into a framework region of a VH of any human antibody. Similarly, DNA encoding a variable region in which the light chain CDRs 1 to 3 are grafted into a framework region of a VL of any human antibody is prepared. Each DNA prepared is inserted into a vector having a sequence encoding the CH or CL of a human antibody to construct a humanized antibody expression vector. The constructed expression vector is introduced into a host cell to obtain a recombinant cell that expresses the humanized antibody. Then, this recombinant cell is cultured to obtain the desired humanized antibody from a culture of the cell.

A Humanized antibody having the heavy chain CDRs 1 to 3 and light chain CDRs 1 to 3 identified in (AB2) to (AB28) above can also be constructed and produced by the same method.

A method for constructing and producing a chimeric antibody is described. As an example, a method for constructing and producing a chimeric antibody having the heavy chain variable region (VH) and light chain variable region (VL) identified in (C1) above is described. As DNA encoding the VH, DNA encoding the amino acid sequence represented by SEQ ID NO: 7 is first prepared. As DNA encoding the VL, DNA encoding the amino acid sequence represented by SEQ ID NO: 9 is prepared. The DNA can be prepared by using a known method such as PCR. The DNA can also be prepared by chemical synthesis.

The obtained DNA encoding the VH or VL is each inserted into a vector having a sequence encoding the CH or CL of a human antibody to construct a chimeric antibody expression vector. A vector having a sequence encoding the CH or CL of a human antibody is commercially available. The constructed expression vector is introduced into a host cell to obtain a recombinant cell that expresses the chimeric antibody. Then, this recombinant cell is cultured to obtain the desired chimeric antibody from a culture of the cell.

A chimeric antibody having the VH and VL identified in (C2) to (C28) above can also be constructed and produced by the same method.

### <Method for Producing Multispecific Antibody>

A method for producing a multispecific antibody includes linking the anti-human CXCR3 antibody of the present disclosure or a fragment thereof with another antibody or a fragment thereof. Another example includes co-expressing the anti-human CXCR3 antibody of the present disclosure or a fragment thereof and another antibody or a fragment thereof in a host cell. Other linking methods include chemical coupling, gene fusion, and non-covalent association.

Examples of practically applied multispecific antibodies (bispecific antibodies) include BLINCYTO^{®} which binds to CD3 and CD19. Other examples include HEMLIBRA^{®} which binds to coagulation factor IXa and coagulation factor X. Production techniques for multispecific antibodies are known in the art, and these known production techniques are applicable to the anti-human CXCR3 antibody of the present disclosure.

### <Purification Method>

A method for purifying the above antibody is not particularly limited, and any known method can be employed. For example, a culture supernatant of the recombinant cell is collected, and the antibody can be purified by combining known methods such as various types of chromatography, salting-out, dialysis, and membrane separation. For example, when the isotype of the antibody is IgG, the antibody can also be easily purified by affinity chromatography using protein A.

### <Modified Antibody>

The antibody of the present disclosure may be a modified antibody bonded with another molecule. Examples of another molecule include peptides, proteins, low molecular weight compounds, radioisotopes, and light absorbers. Examples of the method for bonding the antibody with another molecule include chemical coupling, gene fusion, and non-covalent association. If another molecule to be bonded is an antibody, the modified antibody can be a multispecific antibody. In this regard, a multispecific antibody can be regarded as a type of modified antibody.

In a preferred embodiment, the above modified antibody is an antibody drug conjugate (ADC). Examples of the drug to be conjugated include antiviral drugs; immunosuppressive drugs; immunomodulating drugs; biologically active proteins or polypeptides such as cytokines; radioisotopes; and light absorbers. These drugs can be conjugated directly or indirectly via a linker or a chelating agent to produce the antibody drug conjugate.

### <Medicament>

The present disclosure encompasses a medicament containing the above antibody as an active ingredient. The medicament may be a pharmaceutical composition including the above antibody and a pharmaceutically acceptable carrier. Preferably, the medicament blocks a CXCR3A-dependent cell function.

The mechanism by which the antibody blocks a CXCR3A-dependent cell function includes: binding of the antibody to a ligand-binding site on a cell surface, thereby competitively or non-competitively inhibiting ligand binding to the receptor; binding of the antibody to a site other than the ligand-binding site, thereby inhibiting activation by altering the structure of the receptor; and one in which binding of the antibody to the receptor induces receptor internalization, thereby reducing ligand responsiveness of the cell by decreasing an expression level of the receptor on the cell surface. An antibody that induces receptor internalization is also utilized for an ADC. In a preferred embodiment, the above antibody has internalization activity. In other words, it induces receptor internalization.

In a preferred embodiment, the medicament is for use in the treatment of a disease, disorder, or symptom involving impairment of a CXCR3-dependent cell function as a cause. Examples of the disease, disorder, or symptom involving impairment of a CXCR3-dependent cell function include: Th1-related diseases such as atherosclerosis, myocarditis, multiple sclerosis, asthma, chronic obstructive pulmonary disease, pulmonary fibrosis, type 1 diabetes, psoriasis, rheumatism, inflammatory bowel disease, systemic lupus erythematosus, and acute cardiac allograft rejection; infectious diseases such as influenza and COVID-19; and a tumor.

In particular, a drug that selectively inhibits CXCR3A is considered highly useful in a therapy aimed at suppressing infiltration, proliferation, and activation of inflammatory cells. An increase of a CXCR3A ligand in a patient may be disease-specific; for example, increased levels of CXCL10 have been reported in autoimmune colitis, type 1 diabetes, and acute lung injury; increased levels of CXCL9 and CXCL10 in multiple sclerosis; and increased levels of CXCL9, CXCL10, and CXCL11 in atherosclerosis (Meri K. Tulic et al., Nat Commun. 2019 May 16;10(1):2178.). Little is known about the role of CXCR3B-ligand interaction in an inflammatory disease, and for a drug that inhibits both CXCR3A and CXCR3B, the possibility of an unexpected side effect caused by CXCR3B inhibition cannot be ruled out.

Furthermore, in a certain tumor, a drug that selectively inhibits CXCR3A is considered highly promising. An association between CXCR3 expression and poor prognosis has been suggested in colorectal cancer, gastric cancer, pancreatic cancer, breast cancer, prostate cancer, renal cancer, ovarian cancer, melanoma, glioblastoma, and multiple myeloma (Xiaoming Wang et al., Front Oncol. 2022 Nov 21:12:1022688.). A CXCR3A-ligand interaction promotes tumor proliferation and metastasis, whereas a CXCR3B-ligand interaction promotes anti-tumor proliferation, inhibition of angiogenesis, and apoptosis. Therefore, a CXCR3 inhibitor specific to CXCR3A may suppress tumor growth and metastasis while maintaining the anti-tumor effect of CXCR3B, potentially offering a more potent anti-tumor effect. Although SCH546738 is known as an inhibitor that preferentially inhibits CXCR3A over CXCR3B, no CXCR3A-specific inhibitor has been reported (K. Boye et al., Sci Rep. 2017 Sep 6;7(1):10703.).

On the other hand, in vitiligo, a drug that inhibits both CXCR3A and CXCR3B may demonstrate higher therapeutic efficacy. In vitiligo, migration of a pathogenic T cell is stimulated via CXCR3A, and melanocyte apoptosis is promoted via CXCR3B (Meri K. Tulic et al., Nat Commun. 2019 May 16;10(1):2178.).

### <Administration Method>

The above medicament can be administered orally or parenterally, and systemically or locally. Examples of the dosage form include an injection dosage form, a transnasal dosage form, a transpulmonary dosage form, and a transdermal dosage form. In the case of the injection dosage form, the medicament can be administered systemically or locally, for example, by intravenous injection, intramuscular injection, intraperitoneal injection, or subcutaneous injection. The administration method can be selected as appropriate depending on the age and symptom of a patient. A dose of the antibody can be selected within a range of 0.0001 mg to 1000 mg per kilogram of body weight per administration. Alternatively, for example, the dose per patient can be selected within a range of 0.001 to 100,000 mg/body. However, the dose of the antibody is not limited to these ranges.

### <Formulation>

The above medicament can be formulated according to an ordinary method (e.g., Remington's Pharmaceutical Science, latest edition, Mark Publishing Company, Easton, U.S.A.). The medicament can contain a pharmaceutically acceptable carrier or additive. Examples of the carriers or the additives include, but are not limited to, surfactants (e.g., polyethylene glycol (PEG), or Tween), excipients, antioxidants (e.g., ascorbic acid), colorants, fragrant agents, preservatives, stabilizers, buffers (e.g., phosphoric acid, citric acid, orother organic acids), chelating agents (e.g., ethylenediaminetetraacetic acid (EDTA)), suspensions, isotonizing agents, binders, disintegrators, lubricants, fluidity accelerators, and flavoring agents, and other commonly used carriers or the like can be used as appropriate. Specific examples of the carrier or the additive include light anhydrous silicic acid, lactose, crystalline cellulose, mannitol, starch, carmellose calcium, carmellose sodium, hydroxypropyl cellulose, hydroxypropylmethyl cellulose, polyvinylacetal diethylaminoacetate, polyvinylpyrrolidone, gelatin, medium-chain fatty acid triglyceride, polyoxyethylene hardened castor oil 60, saccharose, carboxymethyl cellulose, corn starch, and inorganic salts. The medicament may contain another low molecular weight polypeptide; proteins such as serum albumin, gelatin, and immunoglobulin; and amino acids such as glycine, glutamine, asparagine, arginine, and lysine.

When the medicament is an aqueous solution for injection, it may be saline, an isotonic solution containing glucose or another auxiliary agent, such as D-sorbitol, D-mannose, D-mannitol, or sodium chloride, and it may be used in combination with an appropriate solubilizer, e.g., an alcohol (e.g., ethanol), a polyalcohol (e.g., propylene glycol, or PEG), a nonionic surfactant (polysorbate 80, or HCO-50), or the like. If necessary, the antibody can be encapsulated in a microcapsule (e.g., one made of hydroxymethyl cellulose, gelatin, or poly[methyl methacrylate]) or can be configured as a colloidal drug delivery system (e.g., liposomes, albumin microspheres, microemulsions, nanoparticles, or nanocapsules (e.g., see "Remington's Pharmaceutical Science 16th edition", Oslo Ed., 1980)).

Furthermore, a technique for sustained release of a drug is known and can be applied to the medicament (Langer et al., J. Biomed. Mater. Res. 15: 167-277 (1981); Langer, Chem. Tech. 12: 98-105 (1982); US Patent No. 3,773,919; EP Patent Publication No. 58,481; Sidman et al., Biopolymers 22: 547-556 (1983); EP Patent Publication No. 133,988).

### <Application for Gene Therapy>

The above nucleic acid may be incorporated into a gene therapy vector or mRNA that can be translated into a protein in vivo, to prepare a gene therapeutic drug. Examples of the method for administering the gene therapeutic drug (recombinant vector) include a direct administration method using a naked plasmid, as well as a method in which the drug is packaged into a liposome or the like for administration, a method in which the drug is incorporated into various viral vectors such as a retrovirus vector, an adenovirus vector, a vaccinia virus vector, a poxvirus vector, an adeno-associated virus vector, or a hemagglutinating virus of Japan (HVJ) vector (see Adolph, "Viral Genome Methods", CRC Press, Florida (1996)), and a method in which the drug is coated on a bead carrier such as a colloidal gold particle for administration (e.g., WO 93/17706).

The gene therapeutic drug may be administered by any method as long as the antibody is expressed in vivo and can exert its effect. Preferably, a sufficient amount of the drug is administered through an appropriate parenteral route, such as an intravenous route, an intraperitoneal route, a subcutaneous route, an intradermal route, an intra-adipose route, an intramammary route, an inhalation route, an intramuscular route, or the like by a method using injection, infusion, gas inductive particle bombardment (using e.g., an electron gun), or a mucosal route using collunarium or the like. Furthermore, the gene therapeutic drug may be administered ex vivo by a process in which the drug is introduced into a cell using liposome transfection, particle bombardment (U.S. Patent No. 4,945,050), or viral infection, and the cell is reintroduced into an animal.

Furthermore, the present disclosure also includes a treatment method and a therapeutic drug regarding a disease of a mammal suffering from a disease or an illness caused by CXCR3-dependent abnormal cell hyperfunction.

Herein, "treatment" means prevention or alleviation of progression and exacerbation of the pathological state of a disease in a mammal that is at risk of suffering from or has suffered from the disease. Thereby, the "treatment" is used to mean a therapeutic treatment aimed at preventing or alleviating progression and exacerbation of various symptoms and the like of the disease.

The term "disease" refers to all diseases that develop due to CXCR3-dependent abnormal cell hyperfunction, and conceptually includes, but is not limited to, atherosclerosis, myocarditis, multiple sclerosis, asthma, chronic obstructive pulmonary disease, pulmonary fibrosis, type 1 diabetes, psoriasis, rheumatism, inflammatory bowel disease, systemic lupus erythematosus, acute cardiac allograft rejection, influenza, COVID-19, or a tumor, all of which involve a Th1-related immune abnormality. It also encompasses a cardiovascular disorder, a neurological disorder, an inflammatory disease, an autoimmune disease, a metabolic disease, an infectious disease, a hematological cancer, or a solid cancer. The "mammal" to be treated means any animal classified as a mammal, and examples thereof include, but are not limited to, a human, as well as a pet animal such as a dog, a cat, or a rabbit, and a domestic animal such as cattle, a pig, a sheep, or a horse. A human is a particularly preferable "mammal".

### <Antibody-Immobilized Carrier>

The present disclosure encompasses a carrier to which the above antibody is immobilized (antibody-immobilized carrier). In a preferred embodiment, the antibody-immobilized carrier is used to remove a CXCR3-expressing cell from a body fluid by bringing the carrier into contact with the body fluid containing the CXCR3-expressing cell. An example of the body fluid includes blood. Only one type or two or more types of the antibody may be immobilized to the carrier.

As a specific form of the antibody-immobilized carrier according to the present disclosure, the antibody is immobilized to a water-insoluble carrier, which is charged into a container. Any material can be used as the water-insoluble carrier, but examples of a material suitable in terms of formability, sterilizability, and low cytotoxicity include: a synthetic polymer such as polyethylene, polypropylene, polystyrene, acrylic resin, nylon, polyester, polycarbonate, polyacrylamide, or polyurethane; a natural polymer such as agarose, cellulose, cellulose acetate, chitin, chitosan, or alginate; an inorganic material such as hydroxyapatite, glass, alumina, or titania; and a metal material such as stainless steel or titanium.

An example of the shape of the carrier includes a granular shape, a fibrous shape, a woven fabric shape, a non-woven fabric shape, a sponge-like porous shape, and a flat plate shape, but a granular shape, a fibrous shape, a woven fabric shape, a non-woven fabric shape, and a sponge-like porous shape are preferable in that they have a large surface area per volume.

A CXCR3-expressing cell removal kit can be prepared by combining the antibody-immobilized carrier of the present disclosure with another component. An example of another component includes an anticoagulant or an extracorporeal circulation circuit.

The present disclosure encompasses a method for treating a disease, disorder, or symptom, including administering an effective amount of the above antibody to a patient suffering from the disease, disorder, or symptom involving a CXCR3-dependent cell dysfunction. The present disclosure encompasses the above antibody for use in treating a disease, disorder, or symptom involving a CXCR3-dependent cell dysfunction. The present disclosure encompasses use of the above antibody for the manufacture of a medicament for the treatment of a disease, disorder, or symptom involving a CXCR3-dependent cell dysfunction. The disease, disorder, or symptom includes at least a cardiovascular disorder, a neurological disorder, an inflammatory disease, an autoimmune disease, a metabolic disease, an infectious disease, a hematological cancer, or a solid cancer.

### EXAMPLES

### [Example 1] Animal Immunization for Preparation of Anti-CXCR3 Antibody

### (1) Preparation of Plasmid for DNA Immunization

A template plasmid vector pCI-hEP4-GroEL was constructed as follows. An artificial gene with a signal sequence (SEQ ID NO: 294) added to the 5' end of human EP4 gene was synthesized based on the sequence information of human EP4 (NM_000958, SEQ ID NO: 293) registered in National Center for Biotechnology Information (NCBI) GenBank. This artificial gene was used as a template to prepare a human EP4 gene fragment (hereinafter referred to as "DNA fragment A") with the 5' end and the 3' end to which GCTAGC sequence and GTCGAC sequence were added respectively by PCR.

Genomic DNA was extracted and purified from Escherichia coli HMS174 (DE3) strain (Novagen). Subsequently, using the purified genomic DNA as a template, a DNA fragment containing the GroEL subunit gene (SEQ ID NO: 297; hereinafter referred to as "DNA fragment B") was amplified using a primer pair represented by SEQ ID NOs: 295 and 296 by PCR. In DNA fragment B, a SalI site was introduced at the 5' end, and a NotI site and a sequence encoding two stop codons (TAATAG) were introduced at the 3' end, all derived from the primers.

A mammalian expression vector pCI-Mammalian Expression Vector (Promega) was digested with restriction enzymes NheI and SalI, and the digested ends were dephosphorylated with bacterial alkaline phosphatase (BAP), and then DNA fragment A was inserted. Furthermore, this expression vector was digested with SalI and NotI, the digested ends were dephosphorylated with BAP, and then DNA fragment B was inserted to construct a template plasmid vector pCI-hEP4-GroEL.

An artificial gene with GCTAGC sequence added to the 5' end of human CXCR3A gene and with GTCGAC sequence added to the 3' end was synthesized based on the sequence information of human CXCR3A (AAO92295, SEQ ID NO: 281) registered in NCBI GenBank. This artificial gene was introduced into a cloning vector, and then the resulting vector was digested at the NheI and SalI sites to prepare a DNA fragment (hereinafter referred to as "DNA fragment C"). The template plasmid vector pCI-hEP4-GroEL was digested with restriction enzymes NheI and SalI, and DNA fragment C was inserted to construct an immunization vector pCI-hCXCR3A-GroEL. This vector expresses a fusion protein of human CXCR3A and GroEL.

### (2) DNA Immunization

The vector pCI-hCXCR3A-GroEL was dissolved at a concentration of 2 mg/mL in phosphate-buffered saline (PBS) to prepare an immunization composition. Thigh muscles of both legs of 8-week-old mice were each injected with 25 µL of this immunization composition to immunize the mice (Day 0). Thus, 50 µg of pCI-hCXCR3A-GroEL was administered to each leg, i.e., 100 µg of pCI-hCXCR3A-GroEL was administered to mouse per injection. After that, the same immunization was performed on day 14 and day 28. Blood was sampled from the immunized animals 21 and 35 days after the initial immunization, and serum was separated and collected, and stored at -30°C.

Thigh muscles of both legs of 8-week-old rats were each injected with 40 µL of the aforementioned immunization composition to immunize the rats (Day 0). Thus, 80 µg of pCI-hCXCR3A-GroEL was administered to each leg, i.e., 160 µg of pCI-hCXCR3A-GroEL was administered to rat per injection. After that, the same immunization was performed on day 14 and day 28. Blood was sampled from the immunized animals 21 and 35 days after the initial immunization, and serum was separated and collected, and stored at -30°C.

### (3) Preparation of Human CXCR3A-Stably Expressing Cells

An artificial gene was synthesized based on the sequence information of human CXCR3A (AAO92295, SEQ ID NO: 281) registered in NCBI GenBank, and introduced into pEF-FRT (Thermo Fisher Scientific) to construct pEF-FRT-hCXCR3A. pEF-FRT-hCXCR3A and pOG44 plasmid (Thermo Fisher Scientific) were co-introduced into CHO-FlpIn cells (Thermo Fisher Scientific) using Lipofectamine 2000 (Thermo Fisher Scientific). Culturing was performed in a medium containing hygromycin (Thermo Fisher Scientific), and cells that were hygromycin-resistant and stably expressed human CXCR3A were cloned. Hereinafter, this human CXCR3A-expressing cell is referred to as "human CXCR3A-stably expressing CHO cell".

### (4) Evaluation for Binding Property of Serum Antibody to Human CXCR3A by Flow Cytometry

The human CXCR3A-stably expressing CHO cells and CHO-FlpIn cells were washed with a fluorescence-activated cell sorting (FACS) buffer (PBS containing 1% fetal bovine serum (FBS)), and suspended in the FACS buffer at a cell concentration of 1×10⁷ cells/mL. To the cell suspension, Fc Block (Tonbo Biosciences) was added in an amount of 1/500 of the cell suspension, and the cells were blocked at 4°C. After the blocking, the cells were resuspended in the buffer at a concentration of 1 to 2×10⁵ cells/50 µL. This cell suspension and the immunized animal serum as a primary antibody sample were mixed, and the mixture was incubated at 4°C. After the incubation, the cells were washed twice with 100 µL of FACS buffer. As a secondary antibody, 50 µL of diluent of a phycoerythrin (PE)-labeled anti-mouse IgG antibody (SouthernBiotech) or an Alexa Fluor 488-labeled anti-rat IgG antibody (Abcam) was added to each well, and incubated at 4°C for 1 hour. The cells were washed twice with 100 µL of FACS buffer, then suspended in 80 µL of FACS buffer, and the fluorescence intensity on the cell surface was measured using flow cytometer iQue (Sartorius) to evaluate the binding property of the antibodies. The immunized animal serum reacted specifically with human CXCR3A-stably expressing CHO cells. This made it possible to confirm that immunization with pCI-hCXCR3A-GroEL had induced production of antibodies specifically recognizing human CXCR3A in immunized animals.

### (5) Boosting Using Transient Expression Cells, and Sampling

The human CXCR3A gene was introduced into a mammalian expression vector pCI-Mammalian Expression Vector (Promega) to construct pCI-hCXCR3A. HEK293FT cells were transfected with pCI-hCXCR3A using Lipofectamine 2000 to transiently express human CXCR3A. These cells were administered into the spleen of the individual (mouse or rat) in section (2), and 3 days later, spleen, inguinal lymph nodes, and iliac lymph nodes were sampled. Splenocytes and lymph node-derived cells were separated from each of the sampled tissues, dispensed in aliquots, and stored at -80°C until screening.

### [Example 2] Preparation of Antibody <1>

### (1) Selection of Specific Antibody-Producing Lymphocyte Using Microwell

Human CXCR3A-stably expressing CHO cells were suspended in F-12 culture medium (10% FBS, containing Penicillin/Streptomycin) to prepare a cell suspension at 3.5×10⁵ cells/500 µL. The cell suspension was filled into a microchamber for cell picking systems. The microchamber was subjected to centrifugation at 300 rpm for 1 minute five times so that one or two human CXCR3A-stably expressing CHO cells were placed in each microwell. After washing the microchamber with F-12 medium, 500 µL of F-12 medium was added thereto. Incubation was performed in a CO₂ incubator at 37°C for 1 hour to allow the human CXCR3A-stably expressing CHO cells to adhere to the bottom surface of the microwells. To the microwells, CytoRed solution (Dojindo Molecular Technologies, Inc.) adjusted to a concentration of 10 nM in F-12 culture medium was added, which was incubated at 37°C for another 1 hour to stain the cells. After washing three times with F-12 medium to remove excess CytoRed, the microchamber was filled with 1 mL of F-12 medium.

From the cells collected in Example 1 (5) after the DNA immunization, antibody-producing cells were concentrated using EasySep Mouse Biotin Positive Selection Kit (STEMCELL Technologies). In 500 µL of F-12 culture medium, 5×10⁴ to 2×10⁵ antibody-producing cells were suspended, and the cell suspension was filled into a microchamber. The microchamber was subjected to centrifugation at 300 rpm for 1 minute five times so that one or two antibody-producing cells were placed in each microwell. After washing the microchamber with the medium, an appropriate amount of the medium was added thereto, and the microchamber was incubated at 37°C for 30 minutes to allow the antibody-producing cells to secrete antibodies. After washing the microwells to remove the supernatant, Alexa Fluor 488-labeled anti-mouse IgG antibody (Abcam) or Alexa Fluor 488-labeled anti-rat IgG antibody (Abcam) diluted 500-fold with RPMI 1640 (containing 10% FBS) was added thereto, followed by incubation at 37°C for 30 minutes. After washing three times with FACS buffer (PBS containing 1% FBS), 1 mL of FACS buffer was added thereto. The microchamber was set in a cell picking system (AS ONE Corporation) to acquire information on transmitted light images and two types of fluorescence images of all the microwells. The fluorescence of CytoRed was detected at an excitation wavelength of 543 nm and a fluorescent wavelength of 593 nm. The fluorescence of Alexa Fluor 488 was detected at an excitation wavelength of 482 nm and a fluorescent wavelength of 536 nm. From microwells that could be determined to have antibodies binding to the human CXCR3A-stably expressing CHO cells based on scan images with transmitted light, CytoRed, and Alexa Fluor 488, antibody-producing cells were collected into a cell lysis solution using a capillary with a diameter of several µm to several tens of µm (AS ONE Corporation).

### (2) Isolation of Antibody Genes from Collected Antibody-Producing Cells

Antibody genes were obtained from the antibody-producing cells by MAGrahd method (Kurosawa N, Yoshioka M, Fujimoto R, Yamagishi F, Isobe M. "Rapid production of antigen-specific monoclonal antibodies from a variety of animals", BMC Biol. 2012; 10: 80). Specifically, cell-derived mRNA was captured on oligo dT magnets by mixing 5 µL of the cell lysate collected in section (1) with 5 µg of the oligo-dT magnets. The oligo-dT magnets were washed with a washing solution using a MAGrahd reactor tray and a neodymium magnet, and then cDNA was synthesized by a reverse transcription reaction. The magnets were further washed, and then a 5'-terminal translational reaction was performed. Using the synthesized cDNA, a gene of an antibody heavy chain variable region (VH region) and a gene of an antibody light chain variable region (VL region) were isolated and amplified by 5' RACE PCR method.

In addition, in order to enhance the specificity of the amplified products, PCR was performed twice. In the first PCR for the antibodies obtained from mice, a first forward primer (SEQ ID NO: 282) for commonly amplifying the VH and VL regions, a mouse VH first reverse primer (SEQ ID NO: 283) that specifically amplifies the VH region, and a mouse VL first reverse primer (SEQ ID NO: 284) that specifically amplifies the VL region were mixed and used. In the second PCR, the first PCR product was used as a template; for amplification of the VH region, a second forward primer (SEQ ID NO: 285) and a mouse VH second reverse primer (SEQ ID NO: 286) that specifically amplifies the VH region were used as primers, and for amplification of the VL region, a second forward primer (SEQ ID NO: 285) and a mouse VL second reverse primer (SEQ ID NO: 287) that specifically amplifies the VL region were used as primers.

Also, in the first PCR for the antibodies obtained from rats, a first forward primer (SEQ ID NO: 282) that commonly amplifies the VH and VL regions, a rat VH first reverse primer (SEQ ID NO: 288) that specifically amplifies the VH region, and a rat VL first reverse primer (SEQ ID NO: 289) that specifically amplifies the VL region were mixed and used. In the second PCR, the first PCR product was used as a template; for amplification of the VH region, a second forward primer (SEQ ID NO: 285) and a rat second reverse primer (SEQ ID NO: 290) that specifically amplifies the VH region were used as primers, and for amplification of the VL region, a second forward primer (SEQ ID NO: 285) and a rat VL second reverse primer (SEQ ID NO: 291) that specifically amplifies the VL region were used as primers.

As a result of agarose gel electrophoresis on the samples after the second PCR, amplification products corresponding to the VH region at a position of about 750 bp and to the VL region at a position of about 550 bp were confirmed, respectively.

### (3) Construction of Antibody Expression Units

Antibody expression units were constructed by TS-jPCR method (Yoshioka M, Kurosawa N, Isobe M. "Target-selective joint polymerase chain reaction: a robust and rapid method for high-throughput production of recombinant monoclonal antibodies from single cells.", BMC Biotechnol., 2011; 11: 75). Specifically, the VH region gene amplified in section (2), an antibody heavy chain constant region gene, and a gene including a promoter region necessary for gene expression were fused by PCR to construct an antibody expression unit that expresses a full-length antibody heavy chain. Similarly, the VL region gene amplified in section (2), an antibody light chain constant region gene, and a gene including a promoter region necessary for gene expression were fused by PCR to construct an antibody expression unit that expresses a full-length antibody light chain.

### (4) Introduction of Antibody Expression Units into Mammalian Cells

Expi293F cells (Thermo Fisher Scientific) were seeded in a 6-well cell culture plate at 7.5×10⁶ cells/3 mL/well. The two types of antibody expression units of heavy chain and light chain constructed in section (3) were co-introduced into the Expi293F cells using ExpiFectamine 293 Reagent (Thermo Fisher Scientific). On day 5 after the introduction, the cell supernatant was collected and used for evaluation of the binding property of the expressed antibody in the same way as in Example 1 (4). Antibody units that bound to human CXCR3A-stably expressing CHO cells but did not bind to CHO-FlpIn cells were determined as primary hit antibodies.

### [Example 3] Preparation of Antibody <2>

### (1) Preparation of Hybridomas and Screening for Binding Clones

As materials, the cells recovered in Example 1 (5) and SP2/0 myeloma cells were fused using a cell fusion apparatus NEPAGENE ECFG21 (Nepa Gene). The fused cells (hybridomas) were suspended in RPMI 1640 medium containing HAT Supplement (Invitrogen) and seeded in 96-well plates. After culturing for about 8 days, a part of the culture supernatant was collected. In the same manner as in Example 1 (4), screening was performed for hybridomas that produced antibodies specifically binding to human CXCR3A-stably expressing CHO cells.

### (2) Cloning of Hybridomas

The hybridomas confirmed to produce antibodies in section (1) were cloned using a limiting dilution method. Specifically, the hybridomas were seeded in 96-well plates at one cell or less per well and cultured. Two weeks later, screening was performed for antibodies specifically binding to the human CXCR3A-stably expressing CHO cells in the same manner as in Example 1 (4). Antibodies that bound to human CXCR3A-stably expressing CHO cells but did not bind to CHO-FlpIn cells were determined as primary hit antibodies.

After expansion culture of the hybridomas that produced the primary hit antibodies, the culture medium was replaced with CD Hybridoma Medium (Gibco) at a concentration of 5×10⁵ cells/mL, and the cells were cultured for 7 days. The culture supernatant was collected and then centrifuged to precipitate cell debris. The supernatant was passed through a 0.45 µm filter (Sartorius) and stored at 4°C until purification.

### (3) Isolation of Antibody Genes

The same procedure as in Example 2 (2) was performed.

### (4) Construction of Antibody Expression Units

The same procedure as in Example 2 (3) was performed.

### (5) Introduction of Antibody Expression Units into Mammalian Cells

The same procedure as in Example 2 (4) was performed.

### [Example 4] Determination of CDR Sequences in Antibody Heavy Chain Variable Region and Antibody Light Chain Variable Region

The second PCR products containing the antibody heavy chain variable region and the antibody light chain variable region obtained in Example 2 (2) or Example 3 (3) were purified using a PCR product purification kit (FastGene). For the sequencing of the antibodies obtained from mice, the direct sequencing method was performed using the mouse VH second reverse primer (SEQ ID NO: 286) as a sequencing primer for the antibody heavy chain variable region and using the mouse VL second reverse primer (SEQ ID NO: 287) as a sequencing primer for the antibody light chain variable region, respectively, to determine the sequences of the antibody heavy chain variable region and the antibody light chain variable region. Similarly, for the sequencing of the antibodies obtained from rats, the direct sequencing method was performed using the rat VH second reverse primer (SEQ ID NO: 290) as a sequencing primer for the antibody heavy chain variable region and using the rat VL second reverse primer (SEQ ID NO: 291) as a sequencing primer for the antibody light chain variable region, respectively, to determine the sequences of the antibody heavy chain variable region and the antibody light chain variable region. To determine the CDRs, the sequences were analyzed using Kabat numbering method and IMGT numbering method in combination, and a region that comprehensively includes the regions determined by both numbering methods was determined as a CDR.

Based on the sequence information of the determined antibody heavy chain variable region and antibody light chain variable region, 28 types of antibodies confirmed to have independent heavy chain variable region and light chain variable region sequences that did not overlap with others were selected. The names of the antibodies are presented below along with the table numbers and sequence numbers described later. Among these antibodies, 201112-1-C, 13B4, and 11F11 are derived from rats, and the others are derived from mice.
201001-1-F (Table 1-1; SEQ ID NOs: 1 to 10)
201001-5-B (Table 1-2; SEQ ID NOs: 11 to 20)
201001-2-C (Table 1-3; SEQ ID NOs: 21 to 30)
201001-4-E (Table 1-4; SEQ ID NOs: 31 to 40)
201001-1-C (Table 1-5; SEQ ID NOs: 41 to 50)
201001-5-A (Table 1-6; SEQ ID NOs: 51 to 60)
201009-1-D (Table 1-7; SEQ ID NOs: 61 to 70)
201009-5-F (Table 1-8; SEQ ID NOs: 71 to 80)
201112-1-C (Table 1-9; SEQ ID NOs: 81 to 90)
13B4 (Table 1-10; SEQ ID NOs: 91 to 100)
11F11 (Table 1-11; SEQ ID NOs: 101 to 110)
201006-4-H (Table 1-12; SEQ ID NOs: 111 to 120)
201006-4-F (Table 1-13; SEQ ID NOs: 121 to 130)
201006-5-A (Table 1-14; SEQ ID NOs: 131 to 140)
201006-4-A (Table 1-15; SEQ ID NOs: 141 to 150)
201006-1-H (Table 1-16; SEQ ID NOs: 151 to 160)
201006-3-D (Table 1-17; SEQ ID NOs: 161 to 170)
201006-1-F (Table 1-18; SEQ ID NOs: 171 to 180)
201006-4-G (Table 1-19; SEQ ID NOs: 181 to 190)
201006-7-A (Table 1-20; SEQ ID NOs: 191 to 200)
201006-2-D (Table 1-21; SEQ ID NOs: 201 to 210)
201006-2-C (Table 1-22; SEQ ID NOs: 211 to 220)
201006-3-H (Table 1-23; SEQ ID NOs: 221 to 230)
201006-5-F (Table 1-24; SEQ ID NOs: 231 to 240)
201006-7-G (Table 1-25; SEQ ID NOs: 241 to 250)
201009-2-C (Table 1-26; SEQ ID NOs: 251 to 260)
201009-3-H (Table 1-27; SEQ ID NOs: 261 to 270)
201009-1-C (Table 1-28; SEQ ID NOs: 271 to 280)

The amino acid sequences of the CDRs and variable regions of the respective antibodies, as well as the nucleotide sequences of the DNAs encoding the variable regions are summarized together with the SEQ ID NOs in Tables 1-1 to 1-28. In the tables, the underlined portions of the sequences of each variable region indicate the CDRs.

**[TABLE 1-1]**

| TABLE 1-1: 201001-1-F | | | |
|---|---|---|---|
| SEQ ID NO: | Region | Type | Sequence |
| 1 | Heavy chain CDR1 | AA | GFTFSSYAMS |
| 2 | Heavy chain CDR2 | AA | TISDGGGYTYYPDNVKG |
| 3 | Heavy chain CDR3 | AA | TREWGYNNYVRFAY |
| 4 | Light chain CDR1 | AA | RATSSIRYMH |
| 5 | Light chain CDR2 | AA | ATSNLAS |
| 6 | Light chain CDR3 | AA | QQWSSNPLT |
| 7 | Heavy chain variable region | AA | EVQLVESGGGLVKPGGSLKLSCAASGFTFSSYAMSWVRQTPEKRLEWVATISDGGG YTYYPDNVKGRFTISRDNAKNNLYLQMSHLKSADTALYYCTREWGYNNYVRFAYWG QGTLVTVSA |
| 8 | Heavy chain variable region | DNA | gaagtgcagctggtggagtctgggggaggcttagtgaagcctggagggtccctgaa actctcctgtgcagcctctggattcactttcagtagctatgccatgtcttgggttc gccagactccggaaaagaggctggagtgggtcgcaaccattagtgatggtggtggt tacacctactatccagacaatgtaaagggccgattcaccatctccagagacaatgc caagaacaacctgtacctgcaaatgagccatctgaagtctgcggacacagccttgt attactgtacaagagaatgggggatataataactacgtccggtttgcttactggggc caagggactctggtcactgtctctgca |
| 9 | Light chain variable region | AA | QIVLSQSPAILSASPGEKVTVTCRATSSIRYMHWYQQKPGSSPKPWIFATSNLASG VPARFSGSGSGTSYSLTISRVEAEDAATYYCQQWSSNPLTFGAGTKLELK |
| 10 | Light chain variable region | DNA | caaattgttctctcccagtctccagcaatcctgtctgcatctccaggggagaaggt cacagtgacttgcagggccacctcaagtataaggtacatgcactggtaccagcaga agccaggatcctcccccaaaccctggatttttgccacatccaacctggcttctgga gtccctgctcgcttcagtggcagtgggtctgggacctcttactctctcacaatcag cagagtggaggctgaagatgctgccacttattactgccagcagtggagtagtaacc cgctcacgttcggtgctgggaccaagctggagctgaaa |

**[TABLE 1-2]**

| TABLE 1-2: 201001-5-B | | | |
|---|---|---|---|
| SEQ ID NO: | Region | Type | Sequence |
| 11 | Heavy chain CDR1 | AA | GFTFSNYAMS |
| 12 | Heavy chain CDR2 | AA | TISDGGGYTYYPDNVKG |
| 13 | Heavy chain CDR3 | AA | TREWGYNNYVRFAY |
| 14 | Light chain CDR1 | AA | RASSRVTYMH |
| 15 | Light chain CDR2 | AA | ATSNLAS |
| 16 | Light chain CDR3 | AA | QQWSSNPLT |
| 17 | Heavy chain variable region | AA | EVQLVESGGGLVKPGGSLKLSCAASGFTFSNYAMSWVRQTPEKRLEWVATISDGGG YTYYPDNVKGRFTISRDNAKNNLYLQMSHLKSADTALYYCTREWGYNNYVRFAYWG QGTLVTVSA |
| 18 | Heavy chain variable region | DNA | gaagtgcaactggtggagtctgggggaggcttagtgaagcctggagggtccctgaa actctcctgtgcagcctctggattcactttcagtaactatgccatgtcttgggttc gccagactccggaaaagaggctggagtgggtcgcaaccattagtgatggtggtggt tacacctactatccagacaatgtaaagggccgattcaccatctccagagacaatgc caagaacaacctatacctacaaatgagccatctgaagtctgcggacacagccttgt attactgtacaagagaatggggatataataactacgtccggtttgcttactggggc caaggactctggtcactgtctctgca |
| 19 | Light chain variable region | AA | QIVLSQSPAILSASPGEKVTMTCRASSRVTYMHWYQQKPGSSPKPWIYATSNLASG VPARFSGSGSGTSYSLTISRVEAEDAATYYCQQWSSNPLTFGAGTKLELK |
| 20 | Light chain variable region | DNA | caaattgttctctcccagtctccagcaatcctgtctgcatctccaggggagaaggt cacaatgacttgcagggccagctcaagggtaacttacatgcactggtaccagcaga agccaggatcctcccccaaaccctggatttatgccacatccaacctggcttctgga gtcccagctcgcttcagtggcagtgggtctgggacctcttactctctcacaatcag cagagtggaggctgaagatgctgccacttattactgccagcagtggagtagtaacc cgctcacgttcggtgctgggaccaagctggagctgaaa |

**[TABLE 1-3]**

| TABLE 1-3: 201001-2-C | | | |
|---|---|---|---|
| SEQ ID NO: | Region | Type | Sequence |
| 21 | Heavy chain CDR1 | AA | GFTFSRYAMS |
| 22 | Heavy chain CDR2 | AA | TISDGGSYTYYPDNVKG |
| 23 | Heavy chain CDR3 | AA | ARDQGTYYRSYYGMDY |
| 24 | Light chain CDR1 | AA | SASSGVIYMH |
| 25 | Light chain CDR2 | AA | STSNLAS |
| 26 | Light chain CDR3 | AA | QQRSSYPFT |
| 27 | Heavy chain variable region | AA | EVQLVESGGGLVKPGGSLKLSCAASGFTFSRYAMSWVRQTPEKRLEWVATISDGGS YTYYPDNVKGRFTISRDNAKNNLYLQMSHLKSEDTAMYYCARDQGTYYRSYYGMDY WGQGTSVTVSS |
| 28 | Heavy chain variable region | DNA | gaagtgcagctggtggagtctgggggaggcttagtgaagcctggagggtccctgaa actctcctgtgcagcctctggattcactttcagtaggtatgccatgtcttgggttc gccagactccggaaaagaggctggagtgggtcgcaaccattagtgatggtggtagt tacacctactatccagacaatgtaaagggccgattcaccatctccagagacaatgc caagaacaacctgtacctgcaaatgagccatctgaagtctgaggacacagccatgt attactgtgcaagagatcaggggacctactataggtcttactatggtatggactac tggggtcaaggaacctcagtcaccgtctcctca |
| 29 | Light chain variable region | AA | QIVLTQSPAIMSASPGEKVTITCSASSGVIYMHWFQQKPGTSPKLWIYSTSNLASG VPARFSGSGSGTSYSLTISRMEAEDAATYYCQQRSSYPFTFGSGTKLEIK |
| 30 | Light chain variable region | DNA | caaattgttctcacccagtctccagcaatcatgtctgcatctccaggggagaaggt caccataacctgcagtgccagctcaggtgtaatttacatgcactggttccagcaga agccaggcacttctcccaaactctggatttatagcacatccaacctggcttctgga gtccctgctcgcttcagtggcagtggatctgggacctcttactctctcacaatcag ccgaatggaggctgaagatgctgccacttattactgccagcaaaggagtagttacc cattcacgttcggctcggggacaaagttggaaataaaa |

**[TABLE 1-4]**

| TABLE 1-4: 201001-4-E | | | |
|---|---|---|---|
| SEQ ID NO: | Region | Type | Sequence |
| 31 | Heavy chain CDR1 | AA | GFTFSSYAMS |
| 32 | Heavy chain CDR2 | AA | TISDGGSYTYYPDNVKG |
| 33 | Heavy chain CDR3 | AA | ARDHGYRYFFDY |
| 34 | Light chain CDR1 | AA | RASQNIGTSIH |
| 35 | Light chain CDR2 | AA | YASESIS |
| 36 | Light chain CDR3 | AA | QQSNSWPTT |
| 37 | Heavy chain variable region | AA | EVQLVESGGGLVKPGGSLKLSCAASGFTFSSYAMSWVRQTPEKRLEWVATISDGGS YTYYPDNVKGRFTISRDNAKNNLYLQMSHLKSEDTAMYYCARDHGYRYFFDYWGQG TTLTVSS |
| 38 | Heavy chain variable region | DNA | gaagtgcagctggtggagtctgggggaggcttagtgaagcctggagggtccctgaa actctcctgtgcagcctctggattcactttcagtagctatgccatgtcttgggttc gccagactccggaaaagaggctggagtgggtcgcaaccattagtgatggtggtagt tacacctactatccagacaatgtaaagggccgattcaccatctccagagacaatgc caagaacaacctgtacctgcaaatgagccatctgaagtctgaggacacagccatgt attactgtgcaagagatcacggctataggtacttctttgactactggggccaaggc accactctcacagtctcctca |
| 39 | Light chain variable region | AA | DILLTQSPAILSVSPGERVSFSCRASQNIGTSIHWYQQRTNGSPRLLIKYASESIS GIPSRFSGSGSGTDFTLSINSVESEDNADYYCQQSNSWPTTFGAGTKLELK |
| 40 | Light chain variable region | DNA | gacatcttgctgactcagtctccagccatcctgtctgtgagtccaggagaaagagt cagtttctcctgcagggccagtcagaacattggcacaagcatacactggtatcagc aaagaacaaatggttctccaaggcttctcataaagtatgcttctgagtctatctct gggatcccttccaggtttagcggcagtggatcagggacagattttactcttagcat caacagtgtggagtctgaagataatgcagattattactgtcaacaaagtaatagct ggccaaccacgttcggtgctgggaccaagctggagctgaaa |

**[TABLE 1-5]**

| TABLE 1-5: 201001-1-C | | | |
|---|---|---|---|
| SEQ ID NO: | Region | Type | Sequence |
| 41 | Heavy chain CDR1 | AA | GFTFSSYAMS |
| 42 | Heavy chain CDR2 | AA | TISNGGSYTYYPDNLKG |
| 43 | Heavy chain CDR3 | AA | ARGREGALLRYWNFDV |
| 44 | Light chain CDR1 | AA | SASSSVSSMH |
| 45 | Light chain CDR2 | AA | DTSKLAS |
| 46 | Light chain CDR3 | AA | QQWSSNPLT |
| 47 | Heavy chain variable region | AA | EVQLVESGGGLVKPGGSLKLSCAASGFTFSSYAMSWVRQTPEKRLEWVATISNGGS YTYYPDNLKGRFTISRDNAKNNLYLQMSHLKSEDTAMYFCARGREGALLRYWNFDV WGAGTTVTVSS |
| 48 | Heavy chain variable region | DNA | gaagtgcaactggtggagtctgggggaggcttagtgaagcctggagggtccctgaa actctcctgtgcagcctctggattcactttcagtagttatgccatgtcttgggttc gccagactccggaaaagaggctggagtgggtcgcaaccattagtaatggtggtagt tacacctactatccagacaatttaaagggccgattcaccatctccagagacaacgc caagaacaacctatacctgcaaatgagtcatctgaagtctgaggacacagccatgt atttctgtgcacgaggccgggaaggggccctattacgctactggaacttcgatgtc tggggcgcagggaccacggtcaccgtctcctca |
| 49 | Light chain variable region | AA | QIVLTQSPVIMSAFPGEKVTMTCSASSSVSSMHWYQQKSGTSPKRWIYDTSKLASG VPARFSGSGSGTSYSLTISSMEAEDAATYYCQQWSSNPLTFGAGTKLELK |
| 50 | Light chain variable region | DNA | caaattgttctcacccagtctccagttatcatgtctgcatttccaggggagaaggt caccatgacctgcagtgccagctcaagtgtaagttccatgcactggtaccagcaga agtcaggcacctcccccaaaagatggatttatgacacatccaaactggcttctgga gtccctgctcgcttcagtggcagtgggtctgggacctcttactctctcacaatcag cagcatggaggctgaagatgctgccacttattactgccagcagtggagtagtaacc cgctcacgttcggtgctgggaccaagctggagctgaaa |

**[TABLE 1-6]**

| TABLE 1-6: 201001-5-A | | | |
|---|---|---|---|
| SEQ ID NO: | Region | Type | Sequence |
| 51 | Heavy chain CDR1 | AA | GFTFSSYAMS |
| 52 | Heavy chain CDR2 | AA | TISNGGSYTYYPDNLKG |
| 53 | Heavy chain CDR3 | AA | TRGREGALLRYWNFDV |
| 54 | Light chain CDR1 | AA | SASSSVSSMH |
| 55 | Light chain CDR2 | AA | DTSKLAS |
| 56 | Light chain CDR3 | AA | QQWSSNPLT |
| 57 | Heavy chain variable region | AA | EVQLVESGGGLVKPGGSLKLSCAASGFTFSSYAMSWVRQTPEKRLEWVATISNGGS YTYYPDNLKGRFTISRDNAKNNLYLQMSHLKSEDTAMYYCTRGREGALLRYWNFDV WGAGTTVTVSS |
| 58 | Heavy chain variable region | DNA | gaagtgcaactggtggagtctgggggaggcttagtgaagcctggagggtccctgaa actctcctgtgcagcctctggattcactttcagtagttatgccatgtcttgggttc gccagactccggaaaagaggctggagtgggtcgcaaccattagtaatggtggtagt tacacctactatccagacaatttaaagggccgattcaccatctccagagacaacgc caagaacaacctgtacctgcaaatgagtcatctgaagtctgaggacacagccatgt attactgtacaagaggccgggaaggggccctattacgctactggaacttcgatgtc tggggcgcagggaccacggtcaccgtctcctca |
| 59 | Light chain variable region | AA | QIVLTQSPAIMSAFPGEKVTMTCSASSSVSSMHWYQQKSGTSPKRWIYDTSKLASG VPARFSGSGSGTSYSLTISSMEAEDAATYYCQQWSSNPLTFGAGTKLELK |
| 60 | Light chain variable region | DNA | caaattgttctcacccagtctccagcaatcatgtctgcatttccaggggagaaggt caccatgacctgcagtgccagctcaagtgtaagttccatgcactggtaccagcaga agtcaggcacctcccccaaaagatggatttatgacacatccaaactggcttctgga gtccctgctcgcttcagtggcagtgggtctgggacctcttactctctcacaatcag cagcatggaggctgaagatgctgccacttattactgccagcagtggagtagtaacc cgctcacgttcggtgctgggaccaagctggagctgaaa |

**[TABLE 1-7]**

| TABLE 1-7: 201009-1-D | | | |
|---|---|---|---|
| SEQ ID NO: | Region | Type | Sequence |
| 61 | Heavy chain CDR1 | AA | GFTFSSYAMS |
| 62 | Heavy chain CDR2 | AA | TISDGGSYTYYPDNVKG |
| 63 | Heavy chain CDR3 | AA | ARSGDGYFSSWFAY |
| 64 | Light chain CDR1 | AA | RASSNVICMY |
| 65 | Light chain CDR2 | AA | YTSNLAS |
| 66 | Light chain CDR3 | AA | QQFTSSPYT |
| 67 | Heavy chain variable region | AA | EVQLVESGGGLVKPGGSLKLSCAASGFTFSSYAMSWVRQTPEKRLEWVATISDGGS YTYYPDNVKGRFTISRDNAKNNLYLQMSHLKSEDTAMYYCARSGDGYFSSWFAYWG QGTLVTVSA |
| 68 | Heavy chain variable region | DNA | gaagtgcagctggtggagtctgggggaggcttagtgaagcctggagggtccctgaa actctcctgtgcagcctctggattcactttcagtagctatgccatgtcttgggttc gccagactccggaaaagaggctggagtgggtcgcaaccattagtgatggtggtagt tacacctactatccagacaatgtaaagggccgattcaccatctccagagacaatgc caagaacaacctgtacctgcaaatgagccatctgaagtctgaggacacagccatgt attactgtgcaagatcgggtgatggttacttctcctcctggtttgcttactggggc caagggactctggtcactgtctctgca |
| 69 | Light chain variable region | AA | ENVLTQSPAIMSATLGEKVTMSCRASSNVICMYWYQQKSGASPKLWIYYTSNLASG VPARFSASGSGASYSLTISSVEAEDAATYYCQQFTSSPYTFGSGTKLEIK |
| 70 | Light chain variable region | DNA | gaaaatgtgctcacccagtctccagcaatcatgtctgcaactctaggggagaaggt caccatgagctgcagggccagctcaaatgtgatttgcatgtactggtaccagcaga agtcaggtgcctcccccaaactatggatttattatacatccaacctggcttctgga gtcccagctcgcttcagtgccagtgggtctggggcctcttattctctcacaatcag cagcgtggaggctgaagatgctgccacgtattactgccagcagtttactagttccc cgtatacgttcggatcggggaccaagctggaaataaaa |

**[TABLE 1-8]**

| TABLE 1-8: 201009-5-F | | | |
|---|---|---|---|
| SEQ ID NO: | Region | Type | Sequence |
| 71 | Heavy chain CDR1 | AA | GFTFSSYAMS |
| 72 | Heavy chain CDR2 | AA | TISDGGGYTYYPDNVKG |
| 73 | Heavy chain CDR3 | AA | TRSGDDYYSSWFAY |
| 74 | Light chain CDR1 | AA | RASSNVKHMY |
| 75 | Light chain CDR2 | AA | KTSNLAS |
| 76 | Light chain CDR3 | AA | QQFTSSPYT |
| 77 | Heavy chain variable region | AA | EVHLVESGGGLVKPGGSLKLSCAASGFTFSSYAMSWVRQTPEKRLEWVATISDGGG YTYYPDNVKGRFTISRDNAKNDLYLQMSHLKSEDTAMYYCTRSGDDYYSSWFAYWG QGTLVTVSA |
| 78 | Heavy chain variable region | DNA | gaagtgcacctggtggaatctgggggaggcttagtgaagcctggagggtccctgaa actctcctgtgcagcctctggattcactttcagtagttatgccatgtcttgggttc gccagactccggaaaagaggctggagtgggtcgcaaccatcagtgatggtggtggt tacacctactatccagacaatgtaaagggccgattcaccatctccagagacaatgc caagaacgacctgtacctgcaaatgagccatctgaagtctgaggacacagccatgt attactgtacaagatcgggtgatgattactactcctcctggtttgcttactggggc caagggactctggtcactgtctctgca |
| 79 | Light chain variable region | AA | ENVLTQSPAIMSATLGEKVTMSCRASSNVKHMYWYQQKSGASPKLWIYKTSNLASG VPARFSGSGSGTSYSLTIISVEAEDAATYYCQQFTSSPYTFGSGTKLEIK |
| 80 | Light chain variable region | DNA | gaaaatgtgctcacccagtctccagcaatcatgtctgcaactctaggggagaaggt caccatgagctgcagggccagctcaaatgtaaagcacatgtactggtaccagcaga agtcaggtgcctcccccaaactatggatttataaaacatccaacctggcttctgga gtcccagctcgcttcagtggcagtgggtctgggacctcttattctctcacaatcat cagcgtggaggctgaagatgctgccacttattactgccagcagtttactagttccc cgtatacgttcggatcggggaccaagctggaaataaaa |

**[TABLE 1-9]**

| TABLE 1-9: 201112-1-C | | | |
|---|---|---|---|
| SEQ ID NO: | Region | Type | Sequence |
| 81 | Heavy chain CDR1 | AA | GYTFTDFAMQ |
| 82 | Heavy chain CDR2 | AA | WINTQTEKPTYAGDFKE |
| 83 | Heavy chain CDR3 | AA | ARGITMGY |
| 84 | Light chain CDR1 | AA | RSSQSLVHSNGNTYLH |
| 85 | Light chain CDR2 | AA | LVSNRFS |
| 86 | Light chain CDR3 | AA | FQRTHNPPT |
| 87 | Heavy chain variable region | AA | QIQLVQSGPELKKPGESLKISCKASGYTFTDFAMQWVKQAPGKGLQYLGWINTQTE KPTYAGDFKERFVFSLETSASTAYLQINNLKNEDTATYFCARGITMGYWGQGVMVT VSS |
| 88 | Heavy chain variable region | DNA | cagatccaattggtacagtctggacctgagctgaagaagcctggagagtcactgaa gatctcctgcaaggcttctggatatactttcacagactttgcaatgcagtgggtga aacaggctccaggaaagggcttgcagtatttgggctggatcaacacccaaactgag aagccaacatatgctggtgacttcaaagaacggtttgtcttctctttggaaacctc tgccagcactgcatatttgcagatcaacaacctcaaaaatgaggacacggctacat atttctgtgcaagagggattactatgggctactggggccaaggagtcatggtcaca gtctcctca |
| 89 | Light chain variable region | AA | DVVLTQTPVTLLISIGGQASISCRSSQSLVHSNGNTYLHWFLQKPGQSPQLLIFLV SNRFSGVPDRFSGSGSGTDFTLKISRVEPEDLGVYYCFQRTHNPPTFGSGTKLEIK |
| 90 | Light chain variable region | DNA | gatgttgtattgacacaaactccagtcaccctgcttatcagcattggaggtcaagc ctctatctcttgccggtctagtcagagcctggtacacagtaatggaaacacctact tgcattggttcctgcagaagccaggccagtctccacagcttctcatctttctggtt tccaacagattttctggggtgccagacaggttcagtggtagtgggtcagggacgga tttcaccctcaagatcagcagagtagagcctgaggacttgggagtttattactgct ttcaacgtacacataatcctcccacgttcggttctgggaccaagctagagatcaaa |

**[TABLE 1-10]**

| TABLE 1-10: 13B4 | | | |
|---|---|---|---|
| SEQ ID NO: | Region | Type | Sequence |
| 91 | Heavy chain CDR1 | AA | GYTFTDYAMQ |
| 92 | Heavy chain CDR2 | AA | WINTQTGKPTYAGDFKE |
| 93 | Heavy chain CDR3 | AA | ARGITMGY |
| 94 | Light chain CDR1 | AA | RSSQSLVHSNGNTYLH |
| 95 | Light chain CDR2 | AA | LVSNRFS |
| 96 | Light chain CDR3 | AA | LQRTHNPPT |
| 97 | Heavy chain variable region | AA | QIQLVQSGPELKKPGESLKISCKASGYTFTDYAMQWVKQAPGKGLQYLGWINTQTG KPTYAGDFKERFVFSLETSASTAYLQINNLKNEDTATYFCARGITMGYWGQGVMVT VSS |
| 98 | Heavy chain variable region | DNA | cagatccaattggtacagtctggacctgagctgaagaagcctggagagtcactgaa gatctcctgcaaggcttctggatataccttcacagactatgcaatgcagtgggtga aacaggccccaggaaagggcttgcaatatttgggctggatcaacacccaaactggg aagccaacatatgctggtgacttcaaagaacggtttgtcttctctttggaaacctc tgccagcactgcatatttgcagatcaacaacctcaaaaatgaggacacggctacat atttctgtgcaagagggattactatgggctactggggccaaggagtcatggtcaca gtctcctca |
| 99 | Light chain variable region | AA | DVVLTQTPVTLLISIGGQASISCRSSQSLVHSNGNTYLHWFLQKPGQSPQLLIFLV SNRFSGVPDRFSGSGSGTDFTLKISRVEPEDLGVYYCLQRTHNPPTFGSGTKLEIK |
| 100 | Light chain variable region | DNA | gatgttgtgttgacacaaactccagtcaccctgcttatcagcattggaggtcaagc ctctatctcttgccggtctagtcagagcctggtccacagtaatggaaacacctact tgcattggttcctacagaagccaggccagtctccacagcttctcatctttctggtt tccaacagattttctggggtgccagacaggttcagtggtagtgggtcagggacgga tttcaccctcaagatcagcagagtagagcctgaggacttgggagtttattactgcc tgcaacgtacacataatcctcccacgttcggttctgggaccaagctggagatcaaa |

**[TABLE 1-11]**

| TABLE 1-11: 11F11 | | | |
|---|---|---|---|
| SEQ ID NO: | Region | Type | Sequence |
| 101 | Heavy chain CDR1 | AA | GYTFTDFAMQ |
| 102 | Heavy chain CDR2 | AA | WINTQTGKPTYADDFKE |
| 103 | Heavy chain CDR3 | AA | GRGITIGY |
| 104 | Light chain CDR1 | AA | RSSQSLVHSNGNTYLH |
| 105 | Light chain CDR2 | AA | LVSNRFS |
| 106 | Light chain CDR3 | AA | LQRTHNPPT |
| 107 | Heavy chain variable region | AA | QIQLVQSGPELKKPGESLKISCKASGYTFTDFAMQWVKQAPGKGLQYMGWINTQTG KPTYADDFKERFVFSLETSASTAYLQINNLKNEDTATYFCGRGITIGYWGQGVMVT VSS |
| 108 | Heavy chain variable region | DNA | cagatccagttggtacagtctggacctgagctgaagaagcctggagagtcactgaa gatctcctgcaaggcttctgggtataccttcacagactttgcaatgcagtgggtga aacaggctccaggaaagggcttgcagtatatgggctggatcaacacccaaactggg aagccaacatatgctgatgacttcaaagaacggtttgtcttctctttggaaacctc tgccagtactgcatatttgcagatcaacaacctcaaaaatgaggacacggctacat atttctgtggaagagggattactataggctactggggccaaggagtcatggtcaca gtctcctca |
| 109 | Light chain variable region | AA | DVVLTQTPVTLLISIGGQASISCRSSQSLVHSNGNTYLHWFLQKPGQSPQLLIYLV SNRFSGVPDRFSGSGSGTDFTLKISRVEPEDLGVYYCLQRTHNPPTFGSGTKLEIK |
| 110 | Light chain variable region | DNA | gatgttgtgttgacacaaactccagtcaccctgcttatcagcattggaggtcaagc ctctatctcttgccggtctagtcagagcctggtacacagtaatggaaacacctact tgcattggttcctgcagaagccaggccagtctccacagcttctcatctatctggtt tccaacagattttctggggtgccagacaggttcagtggtagtgggtcagggacgga tttcaccctcaagatcagcagagtagagcctgaggacttgggagtttattactgct tacaacgtacacataatcctcccacgttcggttctgggaccaagctggagatcaaa |

**[TABLE 1-12]**

| TABLE 1-12: 201006-4-H | | | |
|---|---|---|---|
| SEQ ID NO: | Region | Type | Sequence |
| 111 | Heavy chain CDR1 | AA | GFTFSSYAMS |
| 112 | Heavy chain CDR2 | AA | TISDGGSYTYYPDKVKG |
| 113 | Heavy chain CDR3 | AA | GRDRQLRFNWAWFAY |
| 114 | Light chain CDR1 | AA | KSSQSLLSSSNQKNYLA |
| 115 | Light chain CDR2 | AA | FASTRES |
| 116 | Light chain CDR3 | AA | QQHYSSPRT |
| 117 | Heavy chain variable region | AA | EVQLVESGGGLVKPGGSLKLSCAASGFTFSSYAMSWVRQTPEKRLEWVATISDGGS YTYYPDKVKGRFTISRDNAKNNLYLQMSHLKSEDTAMYYCGRDRQLRFNWAWFAYW GQGTLVTVSA |
| 118 | Heavy chain variable region | DNA | gaagtgcagctggtggagtctgggggaggcttagtgaagcctggagggtccctgaa actctcctgtgcagcctctggattcactttcagtagctatgccatgtcttgggttc gccagactccggaaaagaggctggagtgggtcgcaaccattagtgatggtggtagt tacacctactatccagacaaagtaaagggccgattcaccatctccagagacaatgc caagaacaacctgtacctgcaaatgagccatctgaagtctgaggacacagccatgt attactgtggaagagatcgacaactacgttttaattgggcctggtttgcttactgg ggccaagggactctggtcactgtctctgca |
| 119 | Light chain variable region | AA | DIVMTQSPSSLAMSVGQKVTMSCKSSQSLLSSSNQKNYLAWYQQKPGQSPKLLVYF ASTRESGVPDRFIGSGSGTDFTLTISSVQAEDLALYYCQQHYSSPRTFGGGTRLEI K |
| 120 | Light chain variable region | DNA | gacattgtgatgacacagtctccatcctccctggctatgtcagtaggacagaaggt cactatgagctgcaagtccagtcagagccttttaagtagtagcaatcaaaagaact atttggcctggtaccagcagaaaccaggacagtctcctaaacttctggtatacttt gcatccactagggaatctggggtccctgatcgcttcataggcagtggatctgggac agatttcactcttaccatcagcagtgtgcaggctgaagacctggcactttattact gtcagcaacattatagctctcctcggacgttcggtggaggcaccaggctggaaatc aaa |

**[TABLE 1-13]**

| TABLE 1-13: 201006-4-F | | | |
|---|---|---|---|
| SEQ ID NO: | Region | Type | Sequence |
| 121 | Heavy chain CDR1 | AA | GFTFSSYAMS |
| 122 | Heavy chain CDR2 | AA | TISDGGSYTYYPDSVKG |
| 123 | Heavy chain CDR3 | AA | ARSGDGYFSSWFAY |
| 124 | Light chain CDR1 | AA | RASSNVICMY |
| 125 | Light chain CDR2 | AA | YTSNLAS |
| 126 | Light chain CDR3 | AA | QQFTSSPYP |
| 127 | Heavy chain variable region | AA | EVHLVESGGGSVKPGGSLKLSCAASGFTFSSYAMSWVRQTPEKRLEWVATISDGGS YTYYPDSVKGRFTISRDNAKNNLYLQMSHLKSEDTAMYFCARSGDGYFSSWFAYWG QGTLVTVSA |
| 128 | Heavy chain variable region | DNA | gaagtgcatctggtggagtctgggggaggctcagtgaagcctggagggtccctgaa actctcctgtgcagcctctggattcactttcagtagttatgccatgtcttgggttc gccagactccggaaaagaggctggagtgggtcgcaaccattagtgatggtggcagt tacacctactatccagacagtgttaagggccgattcaccatttccagagacaatgc caagaacaacctataccttcaaatgagccatctgaagtctgaggacacagccatgt atttctgtgcaagatcgggtgatggttacttctcctcttggtttgcttactggggc caagggactctggtcactgtctctgca |
| 129 | Light chain variable region | AA | ENVLTQSPAIMSATLGEKVTMSCRASSNVICMYWYQQKSGASPKLWIYYTSNLASG VPDRFSGSGSGTSYSLTISSVEAEDAATYYCQQFTSSPYPFGSGTKLEIK |
| 130 | Light chain variable region | DNA | gaaaatgtgctcacccagtctccagcaatcatgtctgcaactctaggggagaaggt caccatgagctgcagggccagctcaaatgtaatctgtatgtactggtatcagcaga agtcaggtgcctcccccaaactatggatttattacacatccaacctggcttctgga gtcccagatcgcttcagtggcagtgggtctgggacctcttattctctcacaatcag cagcgtggaggctgaagatgctgccacttattactgccagcagtttactagttccc cgtatccgttcggatcggggaccaagctggaaataaaa |

**[TABLE 1-14]**

| TABLE 1-14: 201006-5-A | | | |
|---|---|---|---|
| SEQ ID NO: | Region | Type | Sequence |
| 131 | Heavy chain CDR1 | AA | GFTFSSYAMS |
| 132 | Heavy chain CDR2 | AA | TISDGGGYTYYPDNVKG |
| 133 | Heavy chain CDR3 | AA | ARSGDGYYSSWFAY |
| 134 | Light chain CDR1 | AA | RASSNVKCVY |
| 135 | Light chain CDR2 | AA | YTSNLAS |
| 136 | Light chain CDR3 | AA | QQFTSSPYT |
| 137 | Heavy chain variable region | AA | EVQLVESGGGLVKPGGSLKLSCAASGFTFSSYAMSWVRQTPEKRLEWVATISDGGG YTYYPDNVKGRFTISRDNAKNNLYLQMSHLKSEDTAMYYCARSGDGYYSSWFAYWG QGTLVTVSA |
| 138 | Heavy chain variable region | DNA | gaagtgcagctggtggagtctgggggaggcttagtgaagcctggagggtccctgaa actctcctgtgcagcctctggattcactttcagtagctatgccatgtcttgggttc gtcagactccggaaaagaggctggagtgggtcgcaaccattagtgatggtggtggt tacacctactatccagacaatgtaaagggccgattcaccatctccagagacaatgc caagaacaacctgtacctgcaaatgagccatctgaagtctgaggacacagccatgt attactgtgcaagatcgggtgatggttactactcctcctggtttgcttactggggc caagggactctggtcactgtctctgca |
| 139 | Light chain variable region | AA | ENVLTQSPAIMSATLGEKVTMSCRASSNVKCVYWYQQKSGASPKLWIYYTSNLASG VPARFSGSGSGTSYSLTISSVEAEDAATYYCQQFTSSPYTFGSGTKLEIK |
| 140 | Light chain variable region | DNA | gaaaatgtgctcacccagtctccagcaatcatgtctgcaactcttggggagaaggt caccatgagctgcagggccagctcaaatgtaaagtgcgtgtactggtaccagcaga agtcaggtgcctcccccaaactatggatttattacacatccaacctggcttctgga gtcccagctcgcttcagtggcagtgggtctgggacctcttattctctcacaatcag cagcgtggaggctgaagatgctgccacttattactgccagcagtttactagttccc cgtatacgttcggatcggggaccaagctggaaataaaa |

**[TABLE 1-15]**

| TABLE 1-15: 201006-4-A | | | |
|---|---|---|---|
| SEQ ID NO: | Region | Type | Sequence |
| 141 | Heavy chain CDR1 | AA | GFIFSSYAMS |
| 142 | Heavy chain CDR2 | AA | TISDGGGYTYYPDNVKG |
| 143 | Heavy chain CDR3 | AA | ARSGDGYYSSWFAY |
| 144 | Light chain CDR1 | AA | RASSNVKHMY |
| 145 | Light chain CDR2 | AA | STSNLAS |
| 146 | Light chain CDR3 | AA | QQFTSSPYA |
| 147 | Heavy chain variable region | AA | EVQLVDSGGDLVKPGGSLKLSCAASGFIFSSYAMSWVRQTPEKRLEWVATISDGGG YTYYPDNVKGRFTISRDNAKNNLYLQMSHLKSEDTAMYFCARSGDGYYSSWFAYWG QGTLVTVSA |
| 148 | Heavy chain variable region | DNA | gaagtgcagctggtggactctgggggagacttagtgaagcctggagggtccctgaa actctcctgtgcagcctctggattcattttcagtagctatgccatgtcttgggttc gccagactccggaaaagaggctggagtgggtcgcaaccattagtgatggtggtggt tacacctactatccagacaatgtaaagggccgattcaccatctccagagacaatgc caagaacaacctgtacctgcaaatgagccatctgaagtctgaggacacagccatgt atttctgtgcaagatcgggtgatggttactactcctcctggtttgcttactggggc caagggactctggtcactgtctctgca |
| 149 | Light chain variable region | AA | ENVLTQSPAIMSATLGEKVTMSCRASSNVKHMYWYQQKSGASPKLWIYSTSNLASG VPPRFSGSGSGTSYSLTISSVEAEDAATYYCQQFTSSPYAFGSGTKLEIK |
| 150 | Light chain variable region | DNA | gaaaatgtgctcacccagtctccagcaatcatgtctgcaactctaggggagaaggt caccatgagctgcagggccagctcaaatgtaaagcacatgtattggtaccagcaga agtcaggtgcctcccccaaactctggatttattccacatccaacctggcttctgga gtcccacctcgcttcagtggcagtgggtctgggacctcttattctctcacaatcag cagcgtggaggctgaagatgctgccacttattactgccagcagtttactagttccc cgtatgcgttcggatcggggaccaagctggaaataaaa |

**[TABLE 1-16]**

| TABLE 1-16: 201006-1-H | | | |
|---|---|---|---|
| SEQ ID NO: | Region | Type | Sequence |
| 151 | Heavy chain CDR1 | AA | GFTFSIYAMS |
| 152 | Heavy chain CDR2 | AA | TISDGGGYTYYPDNLKG |
| 153 | Heavy chain CDR3 | AA | ARSGDGYFSSWFAY |
| 154 | Light chain CDR1 | AA | RANSNVIYIY |
| 155 | Light chain CDR2 | AA | STSSLAS |
| 156 | Light chain CDR3 | AA | QHFTSSPYT |
| 157 | Heavy chain variable region | AA | EVQLVESGGGLVKPGGSLKLSCAASGFTFSIYAMSWVRQTPEKRLEWVATISDGGG YTYYPDNLKGRFTISRDNAKNNLYLQMSHLRSEDTAIYYCARSGDGYFSSWFAYWG QGTLVTVSA |
| 158 | Heavy chain variable region | DNA | gaagtgcagctggtggagtctgggggaggcttagtgaagcctggagggtccctgaa actctcctgtgcagcctctggattcactttcagtatctatgccatgtcttgggttc gccagactccggaaaagaggctggagtgggtcgcaaccattagtgatggtggtggt tatacctactatccagacaatttaaagggccgattcaccatctccagagacaatgc caagaacaacctatacctgcaaatgagccatctgaggtctgaggacacagccattt attactgtgcaagatcgggtgatggttatttctcctcctggtttgcttactggggc caagggactctggtcactgtctctgca |
| 159 | Light chain variable region | AA | EIVLTQSPAIMSATLGEKVTMSCRANSNVIYIYWYQRKSGASPKLWVYSTSSLASG VPPRFTGSGSGTSYSLTISSVEAEDAATYYCQHFTSSPYTFGSGTKLEIK |
| 160 | Light chain variable region | DNA | gaaattgtgctcacccagtctccagcaatcatgtctgcaactctaggggagaaggt caccatgagctgcagggccaactcaaatgtaatctacatttactggtatcagcgga agtcaggtgcctcccccaaactgtgggtttattccacttccagcctggcttctgga gtcccacctcgcttcactggcagtgggtctgggacctcttattctctcacaatcag cagcgtggaggctgaagatgctgccacttattactgccagcattttactagttccc cgtatacgttcggatcggggaccaagctggaaataaaa |

**[TABLE 1-17]**

| TABLE 1-17: 201006-3-D | | | |
|---|---|---|---|
| SEQ ID NO: | Region | Type | Sequence |
| 161 | Heavy chain CDR1 | AA | GFTFSSYAMS |
| 162 | Heavy chain CDR2 | AA | TISDGGGYTYYPDNVKG |
| 163 | Heavy chain CDR3 | AA | ARSGDGYFSSWFAY |
| 164 | Light chain CDR1 | AA | RANSNVIYIY |
| 165 | Light chain CDR2 | AA | STSSLAS |
| 166 | Light chain CDR3 | AA | QHFTSSPYT |
| 167 | Heavy chain variable region | AA | EVQLVESGGGLVKPGGSLKLSCAASGFTFSSYAMSWVRQTPEKRLEWVATISDGGG YTYYPDNVKGRFTISRDNAKNNLYLQMSHLRSEDTAIYYCARSGDGYFSSWFAYWG QGTLVTVSA |
| 168 | Heavy chain variable region | DNA | gaagtgcagctggtggagtctgggggaggcttagtgaagcctggagggtccctgaa actctcctgtgcagcctctggattcactttcagtagctatgccatgtcttgggttc gccagactccggaaaagaggctggagtgggtcgcaaccattagtgatggtggtggt tatacctactatccagacaatgtaaagggccgattcaccatctccagagacaatgc caagaacaacctatacctgcaaatgagccatctgaggtctgaggacacagccattt attactgtgcaagatcgggtgatggttatttctcctcctggtttgcttactggggc caagggactctggtcactgtctctgca |
| 169 | Light chain variable region | AA | EIVLTQSPAIMSATLGEKVTMSCRANSNVIYIYWYQRKSGASPKLWVYSTSSLASG VPPRFTGSGSGTSYSLTISSVEAEDAATYYCQHFTSSPYTFGSGTKLEIK |
| 170 | Light chain variable region | DNA | gaaattgtgctcacccagtctccagcaatcatgtctgcaactctaggggagaaggt caccatgagctgcagggccaactcaaatgtaatctacatttactggtatcagcgga agtcaggtgcctcccccaaactgtgggtttattccacttccagcctggcttctgga gtcccacctcgcttcactggcagtgggtctgggacctcttattctctcacaatcag cagcgtggaggctgaagatgctgccacttattactgccagcattttactagttccc cgtatacgttcggatcggggaccaagctggaaataaaa |

**[TABLE 1-18]**

| TABLE 1-18: 201006-1-F | | | |
|---|---|---|---|
| SEQ ID NO: | Region | Type | Sequence |
| 171 | Heavy chain CDR1 | AA | GFTFRTYAMS |
| 172 | Heavy chain CDR2 | AA | TISDGGGYTYYPDNIKG |
| 173 | Heavy chain CDR3 | AA | ARSGDGYFSSWFAY |
| 174 | Light chain CDR1 | AA | RASSNVKYIY |
| 175 | Light chain CDR2 | AA | STSNLAS |
| 176 | Light chain CDR3 | AA | QQFTSSPYP |
| 177 | Heavy chain variable region | AA | EVQLVESGGGLVKPGGSLKLSCAASGFTFRTYAMSWVRQTPEKRLEWVATISDGGG YTYYPDNIKGRFTISRDNAKNNLYLQMSHLKSEDTALYYCARSGDGYFSSWFAYWG QGTLVTVSV |
| 178 | Heavy chain variable region | DNA | gaagtgcagctggtggagtctgggggaggcttagtgaagcctggagggtccctgaa actctcctgtgcagcctctggattcactttcagaacctatgccatgtcttgggttc gccagactccggaaaagagactggagtgggtcgcaaccattagtgatggtggtggt tacacctactatccagacaatataaagggccgattcaccatctccagagacaatgc caaaaacaacctgtacctgcaaatgagccatctgaagtctgaggacacagccttgt attactgtgcaagatcgggtgatggttacttctcctcctggtttgcttactggggc caagggactctggtcactgtctctgta |
| 179 | Light chain variable region | AA | ENVLTQSPAIMSATLGEKVTMSCRASSNVKYIYWYQQKSGASPKLWIYSTSNLASG VPARFSGSGSGTSYSLTISSVEAEDAATYYCQQFTSSPYPFGSGTKLEIK |
| 180 | Light chain variable region | DNA | gaaaatgtgctcacccagtctccagcaatcatgtctgcaactctaggggagaaggt caccatgagctgcagggccagctcaaatgtaaagtacatatactggtaccagcaga agtcaggtgcctcccccaaactatggatttattccacatccaacctggcttctgga gtcccagctcgcttcagtggcagtgggtctgggacctcttattctctcacaatcag cagcgtggaggctgaagatgctgccacttattactgccagcagtttactagttccc cgtatccgttcggatcggggaccaagctggaaataaaa |

**[TABLE 1-19]**

| TABLE 1-19: 201006-4-G | | | |
|---|---|---|---|
| SEQ ID NO: | Region | Type | Sequence |
| 181 | Heavy chain CDR1 | AA | GFTFRTYAMS |
| 182 | Heavy chain CDR2 | AA | TISDGGGYTYYPDNVKG |
| 183 | Heavy chain CDR3 | AA | ARSGDGYFSSWFAY |
| 184 | Light chain CDR1 | AA | RASSNVKYIY |
| 185 | Light chain CDR2 | AA | STSNLAS |
| 186 | Light chain CDR3 | AA | QQFTSSPYP |
| 187 | Heavy chain variable region | AA | EVQLVESGGGLVKPGGSLKLSCAASGFTFRTYAMSWVRQTPEKRLEWVATISDGGG YTYYPDNVKGRFTISRDNAKNNLYLQMSHLKSEDTALYYCARSGDGYFSSWFAYWG QGTLVTVSV |
| 188 | Heavy chain variable region | DNA | gaagtgcagctggtggagtctgggggaggcttagtgaagcctggagggtccctgaa actctcctgtgcagcctctggattcactttcagaacctatgccatgtcttgggttc gccagactccggaaaagaggctggagtgggtcgcaaccattagtgatggtggtggt tacacctactatccagacaatgtaaagggccgattcaccatctccagagacaatgc caaaaacaacctgtacctgcaaatgagccatctgaagtctgaggacacagccttgt attactgtgcaagatcgggtgatggttacttctcctcctggtttgcttactggggc caagggactctggtcactgtctctgta |
| 189 | Light chain variable region | AA | ENVLTQSPAIMSATLGEKVTMSCRASSNVKYIYWYQQKSGASPKLWIYSTSNLASG VPARFSGSGSGTSYSLTISSVEAEDAATYYCQQFTSSPYPFGSGTKLEIK |
| 190 | Light chain variable region | DNA | gaaaatgtgctcacccagtctccagcaatcatgtctgcaactctaggggagaaggt caccatgagctgcagggccagctcaaatgtaaagtacatatactggtaccagcaga agtcaggtgcctcccccaaactatggatttattccacatccaacctggcttctgga gtcccagctcgcttcagtggcagtgggtctgggacctcttattctctcacaatcag cagcgtggaggctgaagatgctgccacttattactgccagcagtttactagttccc cgtatccgttcggatcggggaccaagctggaaataaaa |

**[TABLE 1-20]**

| TABLE 1-20: 201006-7-A | | | |
|---|---|---|---|
| SEQ ID NO: | Region | Type | Sequence |
| 191 | Heavy chain CDR1 | AA | GFTFNTYAMS |
| 192 | Heavy chain CDR2 | AA | TISDGGGYTYYPDNVKG |
| 193 | Heavy chain CDR3 | AA | ARSGDGYFSSWFAY |
| 194 | Light chain CDR1 | AA | RASANVKYIY |
| 195 | Light chain CDR2 | AA | STSKLSS |
| 196 | Light chain CDR3 | AA | QQFTSSPYT |
| 197 | Heavy chain variable region | AA | EVQLVESGGGLVKPGGSLKLSCAASGFTFNTYAMSWVRQTPEKRLEWVATISDGGG YTYYPDNVKGRFTISRDNGKNNLYLQMSHVKSEDTAMYYCARSGDGYFSSWFAYWG QGTLVTVSA |
| 198 | Heavy chain variable region | DNA | gaagtgcagctggtggagtctgggggaggcttagtgaagcctggagggtccctgaa actctcctgtgcagcctctggattcactttcaatacctatgccatgtcttgggttc gccagactccggaaaagaggctagagtgggtcgcaaccattagtgatggtggtggt tacacctactatccagacaatgtaaagggccgattcaccatctccagagacaatgg caagaacaacctgtacctgcaaatgagccatgtgaagtctgaggacacagccatgt attactgtgcaagatcgggtgatggttacttctcctcctggtttgcttactggggc caagggactctggtcactgtctctgca |
| 199 | Light chain variable region | AA | EDVLIQSPAIMSATLGEKVTMSCRASANVKYIYWYQQKSGASPKLWIYSTSKLSSG VPVRFSGSGSGTSYSLTISSVEAEDAATYYCQQFTSSPYTFGSGTKLEIK |
| 200 | Light chain variable region | DNA | gaagatgtgctcatccagtctccagcaatcatgtctgcaactctaggggagaaggt caccatgagctgcagggccagcgcaaatgtaaagtacatatactggtaccagcaga agtcaggtgcctcccccaaactatggatttattccacatccaaactgtcttctgga gtcccagttcgcttcagtggcagtgggtctgggacctcttattctctcacaatcag cagcgtggaggctgaagatgctgccacttattactgccagcagtttactagttccc cgtatacgttcggatcggggaccaagttggaaataaaa |

**[TABLE 1-21]**

| TABLE 1-21: 201006-2-D | | | |
|---|---|---|---|
| SEQ ID NO: | Region | Type | Sequence |
| 201 | Heavy chain CDR1 | AA | GFTFNTYAMS |
| 202 | Heavy chain CDR2 | AA | TISDGGSYTYYPDNVKG |
| 203 | Heavy chain CDR3 | AA | ARSGDGYFSSWFAY |
| 204 | Light chain CDR1 | AA | RASSNVKYIY |
| 205 | Light chain CDR2 | AA | STSKLSS |
| 206 | Light chain CDR3 | AA | QQFTSSPYT |
| 207 | Heavy chain variable region | AA | EVQLVESGGGLVKPGGSLKLSCAASGFTFNTYAMSWVRQTPEKRLEWVATISDGGS YTYYPDNVKGRFTISRDNAKNNLYLQMSHLKSEDTAMYYCARSGDGYFSSWFAYWG QGTLVTVSA |
| 208 | Heavy chain variable region | DNA | gaagtgcagctggtggagtctgggggaggcttagtgaagcctggagggtccctgaa actctcctgtgcagcctctggattcactttcaatacctatgccatgtcttgggttc gccagactccggaaaagaggctggagtgggtcgcaaccattagtgatggtggtagt tacacctactatccagacaatgtaaagggccgattcaccatctccagagacaatgc caagaacaacctgtacctgcaaatgagccatctgaagtctgaggacacagccatgt attactgtgcaagatcgggtgatggttacttctcctcctggtttgcttactggggc caagggactctggtcactgtctctgca |
| 209 | Light chain variable region | AA | EDVLTQSPAIMSATLGEKVTMSCRASSNVKYIYWYQQKSGASPKLWIYSTSKLSSG VPVRFSGSGSGTSYSLTISSVEAEDAATYYCQQFTSSPYTFGSGTKLEIK |
| 210 | Light chain variable region | DNA | gaagatgtgctcacccagtctccagcaatcatgtctgcaactctaggggagaaggt caccatgagctgcagggccagctcaaatgtaaaatacatatactggtaccagcaga agtcaggtgcctcccccaaactatggatttattccacatccaaactgtcttctgga gtcccagttcgcttcagtggcagtgggtctgggacctcttattctctcacaatcag cagcgtggaggctgaagatgctgccacttattactgccagcagtttactagttccc cgtatacgttcggatcggggaccaagctggaaataaaa |

**[TABLE 1-22]**

| TABLE 1-22: 201006-2-C | | | |
|---|---|---|---|
| SEQ ID NO: | Region | Type | Sequence |
| 211 | Heavy chain CDR1 | AA | GFTFNTYAMS |
| 212 | Heavy chain CDR2 | AA | TISDGGGYTYYPDNLKG |
| 213 | Heavy chain CDR3 | AA | ARSGDGYFSSWFAY |
| 214 | Light chain CDR1 | AA | RASSNVKYIY |
| 215 | Light chain CDR2 | AA | STSKLSS |
| 216 | Light chain CDR3 | AA | QQFTSSPYT |
| 217 | Heavy chain variable region | AA | EVQLVESGGDLVKPGGSLKLSCAASGFTFNTYAMSWVRQTPEKRLEWVATISDGGG YTYYPDNLKGRFTVSRDNAKNNLYLQMSHLKSEDTAIYFCARSGDGYFSSWFAYWG QGTLVTVSA |
| 218 | Heavy chain variable region | DNA | gaagtgcagctggtggagtctgggggagacttagtgaagcctggagggtccctgaa actctcctgtgcagcctctggattcactttcaatacctatgccatgtcttgggttc gccagactccggaaaagaggctggagtgggtcgcaaccattagtgatggtggtggt tacacctactatccagacaatttaaagggccgattcaccgtctccagagacaatgc caagaacaacctgtacctgcaaatgagccatctgaagtctgaggacacagccattt atttctgtgcacgatcgggtgatggttacttctcctcctggtttgcttactggggc caggggactctggtcactgtctctgca |
| 219 | Light chain variable region | AA | ENVLTQSPAIMSATLGEKVTMSCRASSNVKYIYWYQQKSGGSPKLWIYSTSKLSSG VPVRFSGSGSGTSYSLTISSVEAEDAATYYCQQFTSSPYTFGSGTKLEIK |
| 220 | Light chain variable region | DNA | gaaaatgtgctcacccagtctccagcaatcatgtctgcaactctaggggagaaggt caccatgagctgcagggccagctcaaatgtaaagtacatatactggtaccagcaga agtcaggtggctcccccaaactatggatttattccacatccaaactgtcttctgga gtcccagttcgcttcagtggcagtgggtctgggacctcttattctctcacaatcag cagcgtggaggctgaagatgctgccacttattactgccagcagtttactagttccc cgtatacgttcggatcggggaccaagctggaaataaaa |

**[TABLE 1-23]**

| TABLE 1-23: 201006-3-H | | | |
|---|---|---|---|
| SEQ ID NO: | Region | Type | Sequence |
| 221 | Heavy chain CDR1 | AA | GFTFSTYAMS |
| 222 | Heavy chain CDR2 | AA | TISDGGGYTYYPDNVKG |
| 223 | Heavy chain CDR3 | AA | ARSGDGYYSSWFAY |
| 224 | Light chain CDR1 | AA | RASSNVKHMY |
| 225 | Light chain CDR2 | AA | KTSNLAS |
| 226 | Light chain CDR3 | AA | QQFTSSPYT |
| 227 | Heavy chain variable region | AA | EVQLVESGGGLVKPGGSLKLSCAASGFTFSTYAMSWVRQTPEKRLEWVATISDGGG YTYYPDNVKGRFTISRDNAKNDLYLQMSHLKSEDTAMYYCARSGDGYYSSWFAYWG QGTLVTVSA |
| 228 | Heavy chain variable region | DNA | gaagtgcagctggtggaatctgggggaggcttagtgaagcctggagggtccctgaa actctcctgtgcagcctctggattcactttcagtacttatgccatgtcttgggttc gccagactccggaaaagaggctggagtgggtcgcaaccatcagtgatggtggtggt tacacctactatccagacaatgtaaagggccgattcaccatctccagagacaatgc caagaacgacctgtacctgcaaatgagccatctgaagtctgaggacacagccatgt attactgtgcaagatcgggtgatggttactactcctcctggtttgcttactggggc caagggactctggtcactgtctctgca |
| 229 | Light chain variable region | AA | ENVLTQSPAIMSATLGEKVTMSCRASSNVKHMYWYQQKSGASPKLWIYKTSNLASG VPARFSGSGSGTSYSLTISSVEAEDAATYYCQQFTSSPYTFGSGTKLEIK |
| 230 | Light chain variable region | DNA | gaaaatgtgctcacccagtctccagcaatcatgtctgcaactctaggggagaaggt caccatgagctgcagggccagctcaaatgtaaagcacatgtactggtaccagcaga agtcaggtgcctcccccaaattatggatttataaaacatccaacctggcttctgga gtcccagctcgcttcagtggcagtgggtctgggacctcttattctctcacaatcag cagcgtggaggctgaagatgctgccacttattactgccagcagtttactagttccc cgtatacgttcggatcggggaccaagctggaaataaaa |

**[TABLE 1-24]**

| TABLE 1-24: 201006-5-F | | | |
|---|---|---|---|
| SEQ ID NO: | Region | Type | Sequence |
| 231 | Heavy chain CDR1 | AA | GFTFSTYAMS |
| 232 | Heavy chain CDR2 | AA | TISDGGGYTYYPDNIKG |
| 233 | Heavy chain CDR3 | AA | ARSGDGYYSSWFAY |
| 234 | Light chain CDR1 | AA | RASSNVKYMY |
| 235 | Light chain CDR2 | AA | STSKLAS |
| 236 | Light chain CDR3 | AA | QQFTSSPYA |
| 237 | Heavy chain variable region | AA | EVQLVESGGGLVKPGGSLKLSCAASGFTFSTYAMSWVRQTPEKRLEWVATISDGGG YTYYPDNIKGRFTISRDNAKNNLYLQMSHLKSDDTAMYYCARSGDGYYSSWFAYWG QGTLVTVSA |
| 238 | Heavy chain variable region | DNA | gaagtgcagctggtggagtctgggggaggcttagtgaagcctggagggtccctgaa actctcctgtgcagcctctggattcactttcagtacctatgccatgtcttgggttc gccagactccggaaaagaggctggagtgggtcgcaaccattagtgatggtggtggt tacacctactatccagacaatataaagggccgattcaccatctccagagacaatgc caagaacaacctgtacctgcaaatgagccatctgaagtctgacgacacagccatgt attactgtgcaagatcgggtgatggttactactcctcctggtttgcttactggggc caagggactctggtcactgtctctgca |
| 239 | Light chain variable region | AA | ENVLTQSPAIMSATLGEKVTMSCRASSNVKYMYWYQQKSGGSPKLWIYSTSKLASG VPARFSGSGSGTSYSLTISSVEAEDAATYYCQQFTSSPYAFGSGTKLEIK |
| 240 | Light chain variable region | DNA | gaaaatgtgctcacccagtctccagcaatcatgtctgcaactctaggggagaaggt caccatgagctgcagggccagctcaaatgtaaagtacatgtactggtaccagcaga agtcaggtggctcccccaaactctggatttattccacatccaaattggcgtctgga gtcccagctcgcttcagtggcagtgggtctgggacctcttattctctcacaatcag cagcgtggaggctgaagatgctgccacttattactgccagcagtttactagttccc cgtatgcgttcggatcggggaccaagctggaaataaaa |

**[TABLE 1-25]**

| TABLE 1-25: 201006-7-G | | | |
|---|---|---|---|
| SEQ ID NO: | Region | Type | Sequence |
| 241 | Heavy chain CDR1 | AA | GFTFSTYAMS |
| 242 | Heavy chain CDR2 | AA | TISDGGGYTYYPDSVKG |
| 243 | Heavy chain CDR3 | AA | ARSGDGYFSSWFAY |
| 244 | Light chain CDR1 | AA | RASSNVIYMY |
| 245 | Light chain CDR2 | AA | STSSLAS |
| 246 | Light chain CDR3 | AA | QQFTSSPYT |
| 247 | Heavy chain variable region | AA | EVQLVESGGGLVKPGGSLKLSCAASGFTFSTYAMSWVRQTPEKRLEWVATISDGGG YTYYPDSVKGRFTISRDNAKNDLYLQMSHLRSEDTAIYYCARSGDGYFSSWFAYWG QGTLVTVSA |
| 248 | Heavy chain variable region | DNA | gaagtgcagctggtggagtctgggggaggcttagtgaagcctggagggtccctgaa actctcctgtgcagcctctggattcactttcagtacctatgccatgtcttgggttc gccagactccggaaaagaggctggagtgggtcgcaaccattagtgatggtggtggt tatacctactatccagacagtgtaaagggccgattcaccatctccagagacaatgc caagaacgacctgtacctgcaaatgagccatctgaggtctgaggacacagccattt attactgtgcaagatcgggtgatggttacttctcctcctggtttgcttactggggc caagggactctggtcactgtctctgca |
| 249 | Light chain variable region | AA | ENVITQSPAIMSATLGEKVTMSCRASSNVIYMYWYQQKSGASPKLWIYSTSSLASG VPPRFSGSGSGTSYSLTISSVEAEDAATYYCQQFTSSPYTFGSGTKLEIK |
| 250 | Light chain variable region | DNA | gaaaatgtgatcacccagtctccagcaatcatgtctgcaactctaggggagaaggt caccatgagctgcagggccagctcaaatgtaatctacatgtactggtaccagcaga agtcaggtgcctcccccaaactgtggatttattccacttccagcctggcttctgga gtcccacctcgcttcagtggcagtgggtctgggacctcttattctctcacaatcag cagcgtggaggctgaagatgctgccacttattactgccagcagtttactagttccc cgtatacgttcggatcggggaccaagctggaaataaaa |

**[TABLE 1-26]**

| TABLE 1-26: 201009-2-C | | | |
|---|---|---|---|
| SEQ ID NO: | Region | Type | Sequence |
| 251 | Heavy chain CDR1 | AA | GFTFSSYAMS |
| 252 | Heavy chain CDR2 | AA | TISDGGSYTYYPDNIKG |
| 253 | Heavy chain CDR3 | AA | ARSGDGYYSSWFAY |
| 254 | Light chain CDR1 | AA | RASSNVKHMY |
| 255 | Light chain CDR2 | AA | KTSNLAS |
| 256 | Light chain CDR3 | AA | QQFTSSPYA |
| 257 | Heavy chain variable region | AA | EVQLVESGGGLVKPGGSLKLSCAASGFTFSSYAMSWVRQTPEKRLEWVATISDGGS YTYYPDNIKGRFTISRDNAKNDLYLQMSHLKSEDTAMYYCARSGDGYYSSWFAYWG QGTLVTVSA |
| 258 | Heavy chain variable region | DNA | gaagtgcagctggtggaatctgggggaggcttagtgaagcctggagggtccctgaa actctcctgtgcagcctctggattcactttcagtagttatgccatgtcttgggttc gccagactccggaaaagaggctggagtgggtcgcaaccatcagtgatggtggtagt tacacctactatccagacaatataaagggccgattcaccatctccagagacaatgc caagaacgacctgtacctgcaaatgagccatctgaagtctgaggacacagccatgt attactgtgcaagatcgggtgatggttactactcctcctggtttgcttactggggc caaggactctggtcactgtctctgca |
| 259 | Light chain variable region | AA | ENVLTQSPAIMSATLGEKVTMSCRASSNVKHMYWYQQKSGASPKLWIYKTSNLASG VPARFSGSGSGTSYSLTISSVEAEDAATYYCQQFTSSPYAFGSGTKLEIK |
| 260 | Light chain variable region | DNA | gaaaatgtgctcacccagtctccagcaatcatgtctgcaactctaggggagaaggt caccatgagctgcagggccagctcaaatgtaaagcacatgtactggtaccagcaga agtcaggtgcctcccccaaactatggatttataaaacatccaacctggcttctgga gtcccagctcgcttcagtggcagtgggtctgggacctcttattctctcacaatcag cagcgtggaggctgaagatgctgccacttattactgccagcagtttactagttccc cgtatgcgttcggatcggggaccaagctggaaataaaa |

**[TABLE 1-27]**

| TABLE 1-27: 201009-3-H | | | |
|---|---|---|---|
| SEQ ID NO: | Region | Type | Sequence |
| 261 | Heavy chain CDR1 | AA | GFTFSTYAMS |
| 262 | Heavy chain CDR2 | AA | TISDGGGYTYYPDNIKG |
| 263 | Heavy chain CDR3 | AA | ARSGDGYYSSWFAY |
| 264 | Light chain CDR1 | AA | RASSNVKHMY |
| 265 | Light chain CDR2 | AA | STSNLAS |
| 266 | Light chain CDR3 | AA | QQFTSSPYT |
| 267 | Heavy chain variable region | AA | EVQLVESGGGLVKPGGALKLSCAVSGFTFSTYAMSWVRQTPEKRPEWVATISDGGG YTYYPDNIKGRFTISRDNAKNNLYLQINHLKSEDTAMYYCARSGDGYYSSWFAYWG QGTLVTVSA |
| 268 | Heavy chain variable region | DNA | gaagtgcagctggtggagtctgggggaggcttagtgaagcctggagggccctgaa actctcctgtgcagtctctggattcactttcagtacctatgccatgtcttgggttc gccagactccggaaaagagaccggagtgggtcgcaaccattagtgatggtggtggt tacacctactatccagacaatataaagggccgattcaccatctccagagacaatgc caagaacaacctgtacctgcaaattaaccatctgaagtctgaggacacagccatgt attactgtgcaagatcgggtgatggttactactcctcctggtttgcttactggggc caaggactctggtcactgtctctgca |
| 269 | Light chain variable region | AA | ENVLTQSPAIMSAILGEKVTMSCRASSNVKHMYWYQQKSGASPKLWIYSTSNLASG VPARFSGSGSGTSYSLTISSVEAEDAATYYCQQFTSSPYTFGSGTKLEIK |
| 270 | Light chain variable region | DNA | gaaaatgtgctcacccagtctccagcaatcatgtctgcaattctaggggagaaggt caccatgagctgcagggccagctcaaatgtaaagcacatgtactggtaccagcaga agtcaggtgcctcccccaagctatggatttattccacatccaacctggcttctgga gtcccagctcgcttcagtggcagtgggtctgggacctcttattctctcacaatcag cagcgtggaggctgaagatgctgccacttattactgccagcagtttactagttccc cgtatacgttcggatcggggaccaagctggaaataaaa |

**[TABLE 1-28]**

| TABLE 1-28: 201009-1-C | | | |
|---|---|---|---|
| SEQ ID NO: | Region | Type | Sequence |
| 271 | Heavy chain CDR1 | AA | GFTFNTFAMY |
| 272 | Heavy chain CDR2 | AA | RIRTKSNNYATYYADSVKD |
| 273 | Heavy chain CDR3 | AA | VTEYSTYVWFAY |
| 274 | Light chain CDR1 | AA | KASENVRTYVS |
| 275 | Light chain CDR2 | AA | GASNRYT |
| 276 | Light chain CDR3 | AA | GQTYNYPFT |
| 277 | Heavy chain variable region | AA | EVQLAESGGRLVQPKGSLKLSCATSGFTFNTFAMYWIRQAPGKGLEWVARIRTKSN NYATYYADSVKDRFTISRDDSQSMLYLQMNNLKTEDTAMYYCVTEYSTYVWFAYWG QGTLVTVSA |
| 278 | Heavy chain variable region | DNA | gaggtgcagcttgctgagtctggaggaagattggtgcagcctaaagggtcattgaa actctcatgtgcaacctctggattcaccttcaatacctttgccatgtactggatcc gccaggctccaggaaagggtttggaatgggttgctcgcataagaactaaaagtaat aattatgcaacatattatgccgattcagtgaaagacagattcaccatctccagaga tgattcacaaagcatgctctatctgcaaatgaacaacctgaaaactgaggacacag ccatgtattactgtgtgacagaatatagtacctacgtctggtttgcttactggggc caaggactctggtcactgtctctgca |
| 279 | Light chain variable region | AA | NIVMTQSPKSMSMSVGERVTLSCKASENVRTYVSWYQQKPEQSPKLLIYGASNRYT GVPDRFTGSGSATDFTLTISSVQAEDLADYYCGQTYNYPFTFGTGTKLEIK |
| 280 | Light chain variable region | DNA | aacattgtaatgacccaatctcccaaatccatgtccatgtcagtaggagagagggt caccttgagctgcaaggccagtgaaaatgtgcgtacttatgtatcctggtatcaac agaaaccagagcagtctcctaaactactgatatacggggcatccaaccgatacact ggggtccccgatcgcttcacaggcagtggatctgcgacagatttcactctgaccat cagcagtgtgcaggctgaagaccttgcagattattactgtggacagacttacaact atccattcacgttcggcacggggacaaaattggaaataaaa |

### [Example 5] Antibody Screening Based on Evaluation for Intracellular Ca²⁺ Signaling Inhibitory Activity

Purified antibodies were obtained from culture supernatants of clones selected as the primary hit antibodies in Example 2 (4) or Example 3 (5) by Protein A affinity purification. The purified primary hit antibodies were subjected to the following tests.

Human CXCR3A-stably expressing CHO cells were seeded in a 96-well microplate at an initial cell concentration of 2×10⁴ cells/100 µL per well and cultured for 2 days. After 2 days, the culture medium was replaced with a solution containing 3 µM Cal-520 (AAT Bioquest), 0.05% of Pluronic-F127, and 2.5 mM of probenecid (Thermo Fisher Scientific). After one hour, the primary hit antibodies diluted to a concentration within a range of 100 nM to 10 nM, or control antibodies were added to each well and incubated for 15 minutes. As the control antibodies, a mouse isotype control antibody (FUJIFILM Wako Pure Chemical Corporation) or a rat isotype control antibody (FUJIFILM Wako Pure Chemical Corporation) was used. After the incubation, the assay plate was set in a Ca²⁺ signal analyzer FDSSµCELL (Hamamatsu Photonics K.K.) to measure the inhibitory activity against a transient increase in the intracellular Ca²⁺ concentration when each cell was stimulated with 100 nM IP-10 (PeproTech). The inhibitory activity was calculated as a relative value by performing standardization in which a case "without antibody and with IP-10" was defined as an inhibition rate of 0% and a case "without antibody and without IP-10" was defined as an inhibition rate of 100%. As representative examples, the inhibitory activities of 28 types of antibodies having different CDRs are shown in Table 2. The notation "N/A" in the table indicates that the measurement has not been performed.

The same test was repeated, and the following eight types of antibodies: 13B4 and 11F11 obtained from rats, and 201006-3-H, 201006-5-F, 201006-7-G, 201009-2-C, 201009-3-H, and 201009-1-C obtained from mice, were selected as antibodies exhibiting stable intracellular Ca²⁺ signaling inhibitory activities.

### [Table 2]

**TABLE 2**

| Antibody | Intracellular Ca²⁺ signaling inhibition (%) (100 nM IP-10) | | |
|---|---|---|---|
| | 100 nM | 33 nM | 10 nM |
| 201001-1-F | 99 | N/A | 15 |
| 201001-5-B | 96 | N/A | -8 |
| 201001-2-C | 99 | N/A | 10 |
| 201001-4-E | 39 | N/A | -29 |
| 201001-1-C | 92 | N/A | 5 |
| 201001-5-A | 72 | N/A | -24 |
| 201009-1-D | 86 | N/A | -6 |
| 201009-5-F | 93 | N/A | 20 |
| 201112-1-C | N/A | 84 | N/A |
| 13B4 | 95 | N/A | 67 |
| 11F11 | 98 | N/A | 61 |
| 201006-4-H | 50 | N/A | -12 |
| 201006-4-F | 86 | N/A | -13 |
| 201006-5-A | 26 | N/A | -11 |
| 201006-4-A | 83 | N/A | 2 |
| 201006-1-H | 97 | N/A | -13 |
| 201006-3-D | 92 | N/A | 17 |
| 201006-1-F | 96 | N/A | -4 |
| 201006-4-G | 83 | N/A | 0 |
| 201006-7-A | 98 | N/A | 21 |
| 201006-2-D | 96 | N/A | 0 |
| 201006-2-C | 96 | N/A | -2 |
| 201006-3-H | 96 | N/A | 25 |
| 201006-5-F | 97 | N/A | 6 |
| 201006-7-G | 97 | N/A | 16 |
| 201009-2-C | 97 | N/A | 30 |
| 201009-3-H | 98 | N/A | 30 |
| 201009-1-C | 98 | N/A | 3 |

### [Example 6] Quantification of CXCR3A and CXCR3B Gene Expression in Vascular Endothelial Cells

RNA was extracted from pellets of human umbilical cord vascular endothelial cells (Lonza) and human microvascular endothelial cells (Cell Systems) using RNeasy Mini Kit (QIAGEN) according to the protocol included with the kit, and genomic DNA was removed from the RNA using DNase I (QIAGEN). For the human renal adenocarcinoma cell line ACHN, which is known to express both CXCR3A and CXCR3B, RNA subjected to the same treatment was obtained (Takanobu Utsumi, Takahito Suyama, Yusuke Imamura, Miki Fuse, Shinichi Sakamoto, Naoki Nihei, Takeshi Ueda, Hiroyoshi Suzuki, Naohiko Seki, Tomohiko Ichikawa, "The association of CXCR3 and renal cell carcinoma metastasis" J Urol. 2014 Aug; 192 (2): 567-74.).

cDNA was synthesized from the RNA using High capacity RNA-to-cDNA kit (Applied Biosystems). CXCR3A and CXCR3B genes contained in 100 ng of the cDNA were detected by quantitative real-time PCR. 18S was used as an internal standard gene, and TaqMan Gene Expression Assays (Applied Biosystems) was used as 18S detection primers. As detection primers for CXCR3A and CXCR3B, TaqMan Assays described in the literature was used (Laura Lasagni, Michela Francalanci, Francesco Annunziato, Elena Lazzeri, Stefano Giannini, Lorenzo Cosmi, Costanza Sagrinati, Benedetta Mazzinghi, Claudio Orlando, Enrico Maggi, Fabio Marra, Sergio Romagnani, Mario Serio, Paola Romagnani, "An Alternatively Spliced Variant of CXCR3 Mediates the Inhibition of Endothelial Cell Growth Induced by IP-10, Mig, and I-TAC, and Acts as Functional Receptor for Platelet Factor 4." J Exp Med. 2003 Jun 2; 197 (11): 1537-49.).

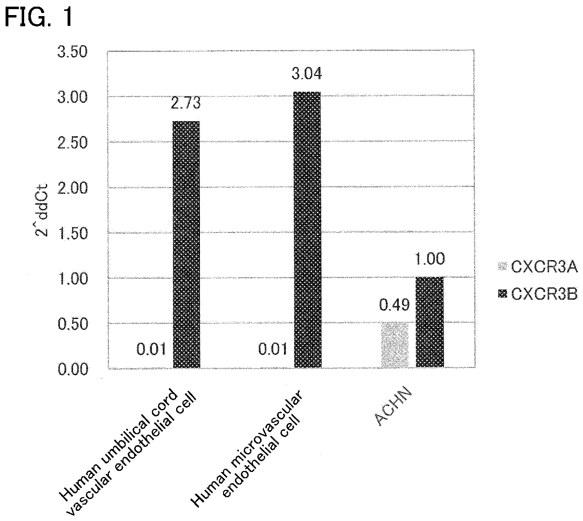

The obtained Ct values were analyzed by the ΔΔCt method to calculate relative expression levels of CXCR3A and CXCR3B in each cell, with the CXCR3B expression level in ACHN being defined as 1. As shown in Fig. 1, it was confirmed that CXCR3B was predominantly expressed compared to CXCR3A in the human umbilical cord vascular endothelial cells and the human microvascular endothelial cells.

### [Example 7] Evaluation for Binding Property to Vascular Endothelial Cells by Flow Cytometry

Among 28 types of anti-CXCR3 antibodies listed in Table 2, 19 types of antibodies were subjected to the following tests.

Human CXCR3A-stably expressing CHO cells, as well as human umbilical cord vascular endothelial cells (Lonza) and human microvascular endothelial cells (Cell Systems) that were confirmed in Example 6 to predominantly express CXCR3B, were washed with FACS buffer (PBS containing 1% FBS) and then suspended in FACS buffer at a cell concentration of 1×10⁷ cells/mL. To the cell suspension, Fc Block (Tonbo Biosciences) in an amount of 1/500 of the cell suspension, or 1:1-diluted goat serum was added, and blocking was performed at 4°C for 30 minutes. After the blocking, the cells were resuspended at a concentration of 1×10⁵ cells/50 µL. Then, 50 µL of this cell suspension was mixed with 50 µL of various diluted antibodies, and the mixture was incubated at 4°C for 1 hour. As the aforementioned antibodies, 19 types of anti-CXCR3 antibodies, CXCR3B-specific antibodies (Proteintech), prior art antibodies, or control antibodies were used. As the prior art antibodies, anti-CXCR3 antibody #49801 (R&D Systems) or anti-CXCR3 antibody 4Hu3 (described in Patent Document 2) was used. As the control antibodies, a mouse isotype control antibody (FUJIFILM Wako Pure Chemicals Corporation), a rat isotype control antibody (FUJIFILM Wako Pure Chemicals Corporation), or a human isotype control antibody (GenScript) was used. After the incubation, the cells were washed twice with 100 µL of FACS buffer. As the secondary antibodies, 50 µL of a diluent of PE-labeled anti-mouse IgG antibody (SouthernBiotech), Alexa Fluor 488-labeled anti-rat IgG antibody (Abcam), APC-labeled anti-rat IgG antibody (Abcam), or APC-labeled anti-human IgG antibody (Invitrogen) was added to each well, which was incubated at 4°C for 1 hour. The cells were washed twice with 100 µL of FACS buffer, then suspended in 50 µL of FACS buffer, and the fluorescence intensity on the cell surface was measured using a flow cytometer Novocyte (Agilent Technologies) to evaluate antibody binding properties.

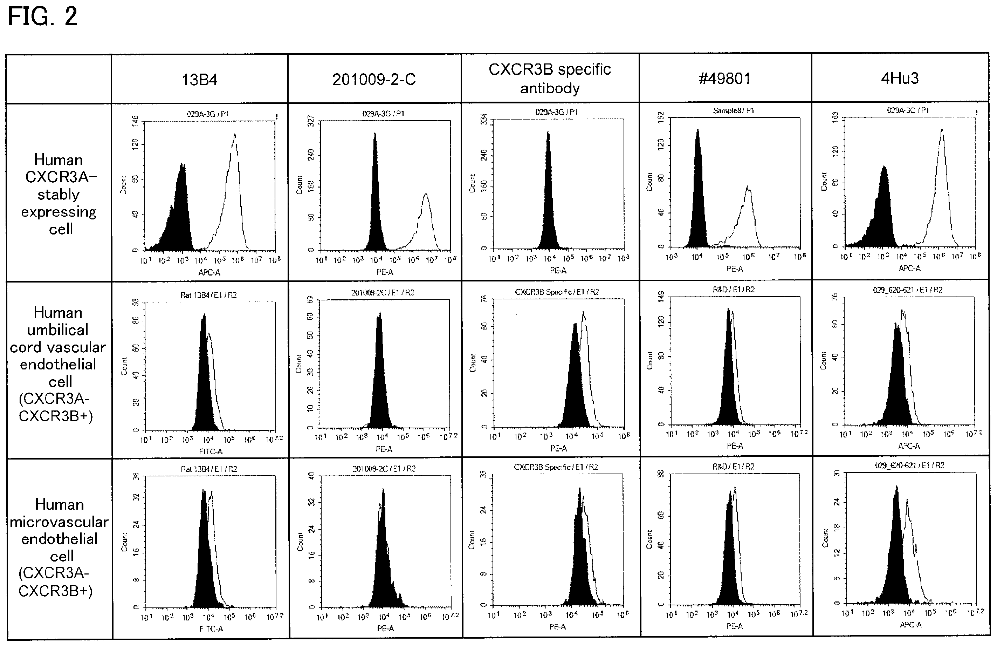

Fig. 2 presents histograms as representative examples, in which 13B4, 201009-2-C, a CXCR3B-specific antibody, or prior art antibodies (collectively, anti-chemokine antibodies) are compared to the control antibody (isotype antibody). In Fig. 2, the blackened graph areas represent isotype antibodies, and the blanked graph areas represent anti-chemokine antibodies. A case where a histogram shifted to the right was judged as "bound". The CXCR3B-specific antibody was confirmed to bind to two types of human vascular endothelial cells but not to human CXCR3A-stably expressing CHO cells. The 13B4 and the prior art antibodies were confirmed to bind to human CXCR3A-stably expressing CHO cells and two types of human vascular endothelial cells. The 201009-2-C bound only to the human CXCR3A-stably expressing CHO cells and did not bind to human vascular endothelial cells.

The binding specificities of the antibodies to the cells are presented in Table 3. Cases showing a rightward shift in the histogram were indicated by "+", and cases showing no such shift were indicated by "-". It was confirmed that 16 types of antibodies including 201009-2-C bound specifically to human CXCR3A but did not bind to human vascular endothelial cells that predominantly express human CXCR3B.

### [Table 3]

**TABLE 3**

| Antibody | Cell binding | | |
|---|---|---|---|
| | Human CXCR3A - stably expressing cell | Human umbilical cord vascular endothelial cell | Human microvascular endothelial cell |
| 201001-1-F | + | - | - |
| 201001-5-B | + | - | - |
| 201001-2-C | + | - | - |
| 201001-1-C | + | - | - |
| 201009-5-F | + | - | - |
| 13B4 | + | + | + |
| 11F11 | + | + | + |
| 201006-1-H | + | - | - |
| 201006-3-D | + | - | - |
| 201006-1-F | + | - | - |
| 201006-7-A | + | - | - |
| 201006-2-D | + | - | - |
| 201006-2-C | + | - | - |
| 201006-3-H | + | - | - |
| 201006-5-F | + | - | - |
| 201006-7-G | + | - | - |
| 201009-2-C | + | - | - |
| 201009-3-H | + | - | - |
| 201009-1-C | + | + | + |
| CXCR3B specific antibody | - | + | + |
| #49801 | + | + | + |
| 4Hu3 | + | + | + |

### [Example 8] Preparation of Antibody

### (1) cDNA Cloning

The heavy chains and light chains of the eight types of antibody genes selected in Example 5: 13B4, 11F11, 201006-3-H, 201006-5-F, 201006-7-G, 201009-2-C, 201009-3-H, and 201009-1-C were cloned into a pET vector by a homologous sequence-selective recombination cloning (Kurosawa N, Yoshioka M, and Isobe M., Target-selective homologous recombination cloning for high-throughput generation of monoclonal antibodies from single plasma cells, BMC Biotechnol., Apr 13, 2011; 11: 39).

### (2) Introduction of Antibody-Expressing Plasmid into Mammalian Cells

ExpiCHO cells (Thermo Fisher Scientific) were seeded into a 125 mL Erlenmeyer flask (vent cap) for cell culture at a concentration of 1.8×10⁸ cells/30 mL/flask. Two types of plasmids: heavy chain and light chain antibody-expressing plasmids constructed in section (1) were co-introduced into ExpiCHO cells using Expifectamine CHO Transfection Kit (Thermo Fisher Scientific). On day 5 after the introduction, the cell supernatants were collected and purified by protein A affinity purification to obtain purified antibodies.

### [Example 9] Evaluation for Binding Property to CXCR3A by Flow Cytometry

The eight types of antibodies obtained in Example 8: 13B4, 11F11, 201006-3-H, 201006-5-F, 201006-7-G, 201009-2-C, 201009-3-H, and 201009-1-C were subjected to the following tests.

The human CXCR3A-stably expressing CHO cells were washed with FACS buffer (PBS containing 1% FBS) and suspended in FACS buffer at a cell concentration of 1×10⁷ cells/mL. To this suspension, Fc Block (Tonbo Biosciences) was added in an amount of 1/500 of the cell suspension, and blocking was performed at 4°C for 30 minutes. After the blocking, the cells were suspended at a concentration of 1×10⁵ cells/50 µL. A 50 µL aliquot of this cell suspension was mixed with 50 µL of each of the eight types of diluted anti-CXCR3 antibodies or control antibodies, and the mixture was incubated at 4°C for 1 hour. As control antibodies, a mouse isotype control antibody (FUJIFILM Wako Pure Chemical Corporation) or a rat isotype control antibody (FUJIFILM Wako Pure Chemical Corporation) was used. After the incubation, the cells were washed twice with 100 µL of FACS buffer. As a secondary antibody, 50 µL of a diluent of a PE-labeled anti-mouse IgG antibody (SouthernBiotech) or an Alexa Fluor 488-labeled anti-rat IgG antibody (Abcam) was added to each well, and incubated at 4°C for 1 hour. The cells were washed twice with 100 µL of FACS buffer, then suspended in 50 µL of FACS buffer, and the fluorescence intensity on the cell surface was measured using a flow cytometer Novocyte (Agilent Technologies) to evaluate the binding property of the antibodies.

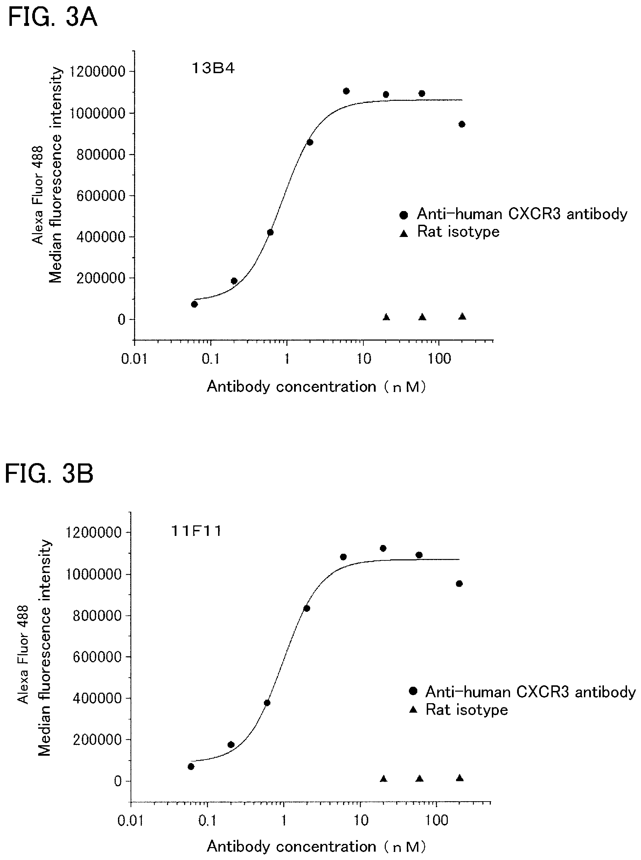

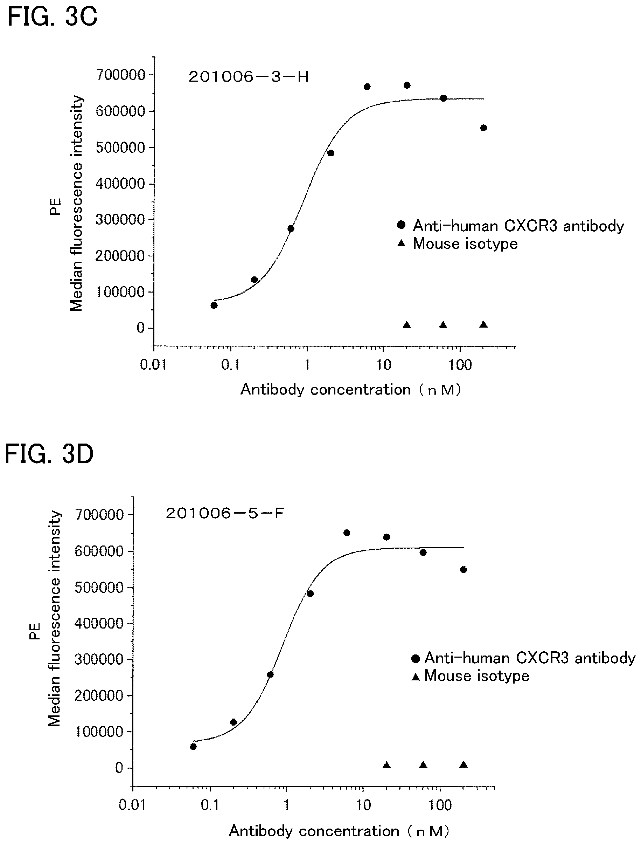

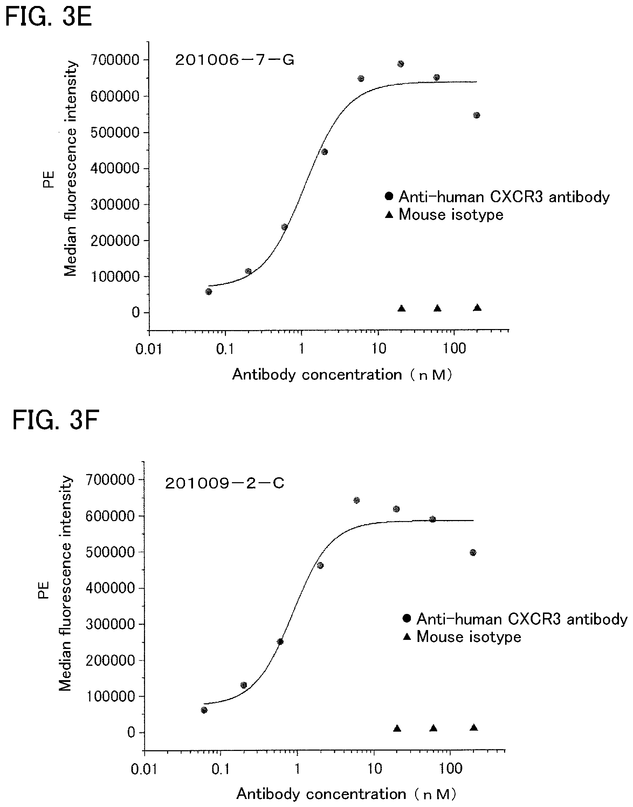

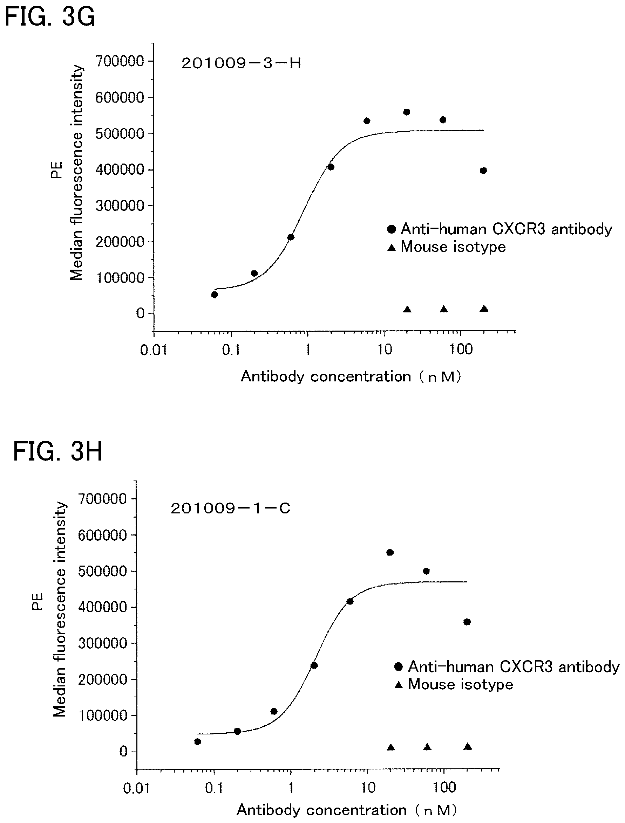

A graph was created, in which the concentration of the antibody was plotted on the horizontal axis, and the median fluorescence intensity of the flow cytometer histogram was plotted on the vertical axis. All of the eight antibodies were confirmed to dose-dependently bind to the human CXCR3A-stably expressing CHO cells. The results of the binding of each antibody to the human CXCR3A-stably expressing CHO cells are presented in Figs. 3A to 3H. The 50% binding concentration (EC₅₀) and the 80% binding concentration (EC₈₀) were calculated and summarized in Table 4.

### [Table 4]

**TABLE 4**

| Antibody | Binding to human CXCR3A | |
|---|---|---|
| | EC₅₀ (nM) | EC₈₀ (nM) |
| 13B4 | 0.9 | 1.9 |
| 11F11 | 1.0 | 2.1 |
| 201006-3-H | 0.9 | 2.1 |
| 201006-5-F | 0.9 | 1.9 |
| 201006-7-G | 1.1 | 2.7 |
| 201009-2-C | 0.9 | 1.9 |
| 201009-3-H | 0.9 | 1.9 |
| 201009-1-C | 2.1 | 4.3 |

### [Example 10] Evaluation for Intracellular Ca²⁺ Signaling Inhibition

The eight types of antibodies obtained in Example 8: 13B4, 11F11, 201006-3-H, 201006-5-F, 201006-7-G, 201009-2-C, 201009-3-H, 201009-1-C were subjected to the following tests.

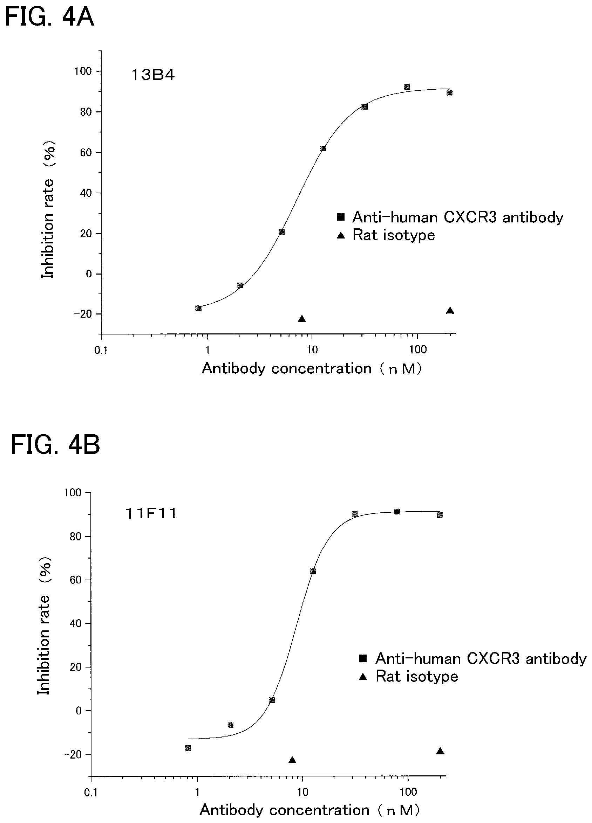

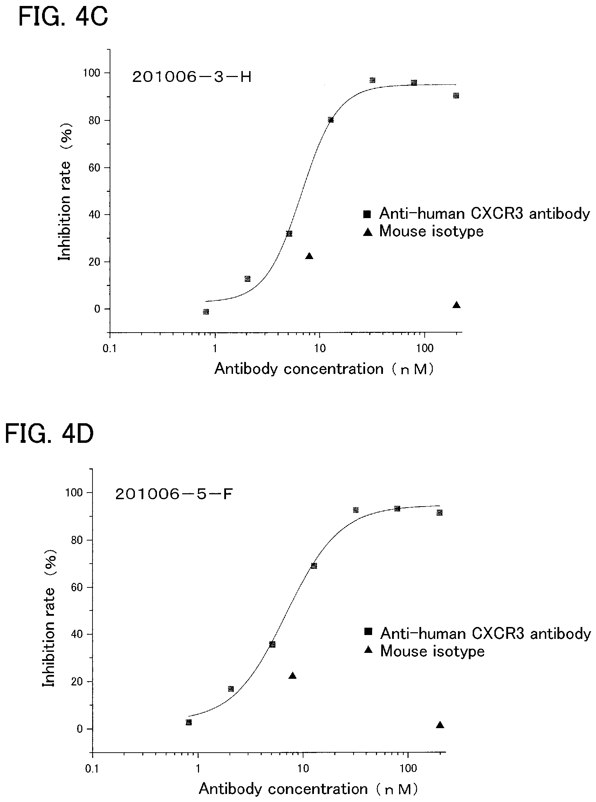

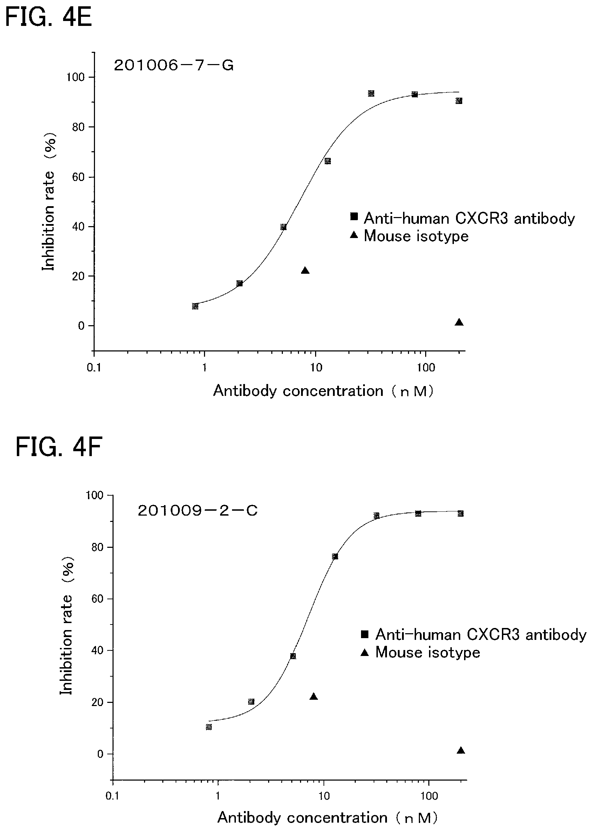

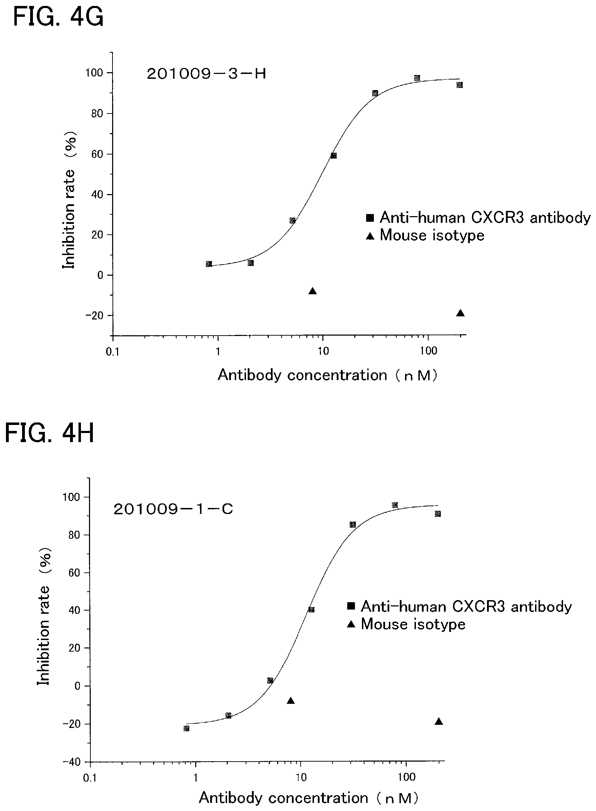

Human CXCR3A-stably expressing CHO cells were seeded in a 96-well microplate at an initial cell concentration of 2×10⁴ cells/100 µL per well and cultured for 2 days. After 2 days, the culture medium was replaced with a solution containing 3 µM Cal-520 (AAT Bioquest), 0.05% Pluronic-F127, and 2.5 mM probenecid (Thermo Fisher Scientific). After 1 hour, the eight types of antibodies diluted to a concentration within a range of 200 nM to 0.8 nM, or control antibodies were added to each well and incubated for 15 minutes. As the control antibodies, a mouse isotype control antibody (FUJIFILM Wako Pure Chemical Corporation) or a rat isotype control antibody (FUJIFILM Wako Pure Chemical Corporation) was used. After the incubation, the assay plate was placed in a Ca²⁺ signal analyzer FDSSµCELL (Hamamatsu Photonics K.K.) to measure the inhibitory activity against the transient increase in the intracellular Ca²⁺ concentration induced by stimulation of each cell with 100 nM IP-10 (PeproTech). The inhibitory activity was calculated as a relative value based on standardization in which a case of "without antibody and with IP-10" was defined as an inhibition rate of 0% and a case of "without antibody and without IP-10" was defined as an inhibition rate of 100%. Graphs with each antibody concentration plotted on the horizontal axis and each inhibition rate (%) plotted on the vertical axis are presented in Figs. 4A to 4H.

The graphs show that all of the eight antibodies dose-dependently inhibited IP-10-induced intracellular Ca²⁺ signaling in the human CXCR3A-stably expressing CHO cells, and had an activity of blocking the CXCR3-dependent cell functions. Table 5 presents the 50% inhibitory concentration (IC₅₀) of each antibody. Among the eight antibodies, six antibodies that showed excellent activities in Examples 9 and 10: 13B4, 11F11, 201006-3-H, 201006-5-F, 201006-7-G, and 201009-2-C were selected and used for the following analyses.

### [Table 5]

**TABLE 5**

| Antibody | Intracellular Ca²⁺ signaling inhibitory activity IC₅₀ (nM) (100 nM IP-10) |
|---|---|
| 13B4 | 7.1 |
| 11F11 | 8.9 |
| 201006-3-H | 6.7 |
| 201006-5-F | 7.0 |
| 201006-7-G | 7.1 |
| 201009-2-C | 7.1 |
| 201009-3-H | 9.9 |
| 201009-1-C | 11.4 |

### [Example 11] Binding Specificity Evaluation for Antibodies

To evaluate the binding specificity of the antibodies against CXCR3A, the six antibodies selected in Example 10: 13B4, 11F11, 201006-3-H, 201006-5-F, 201006-7-G, and 201009-2-C were subjected to the following tests.

Human CXCR3A-stably expressing CHO cells, and other chemokine receptor-expressing cells such as human CCR7-stably expressing CHO cells (in-house production) and human CCR6-stably expressing CHO cells (in-house production) were washed with FACS buffer (PBS containing 1% FBS), and suspended in FACS buffer at a cell concentration of 1×10⁷ cells/mL. To the cell suspension, Fc Block (Tonbo Biosciences) was added in an amount of 1/500 of the cell suspension, and the cells were blocked at 4°C for 30 minutes. After the blocking, the cells were resuspended in the buffer at a concentration of 1×10⁵ cells/50 µL.
A 50 µL aliquot of this cell suspension was mixed with 50 µL each of the six types of diluted anti-CXCR3 antibodies, an anti-CCR7 antibody (in-house production), an anti-CCR6 antibody (in-house production), or control antibodies, and the mixture was incubated at 4°C for 1 hour. As the control antibodies, a mouse isotype control antibody (FUJIFILM Wako Pure Chemical Corporation), a rat isotype control antibody (FUJIFILM Wako Pure Chemical Corporation), or a human isotype control antibody (GenScript) was used. After the incubation, the cells were washed twice with 100 µL of FACS buffer. As secondary antibodies, 50 µL of a diluent of PE-labeled anti-mouse IgG antibody (SouthernBiotech), APC-labeled anti-rat IgG antibody (Abcam), or APC-labeled anti-human IgG antibody (Invitrogen) was added to each well, and incubated at 4°C for 1 hour. The cells were washed twice with 100 µL of FACS buffer, then suspended in 50 µL of FACS buffer, and the fluorescence intensity on the cell surface was measured using a flow cytometer Novocyte (Agilent Technologies) to evaluate the binding property of the antibodies.

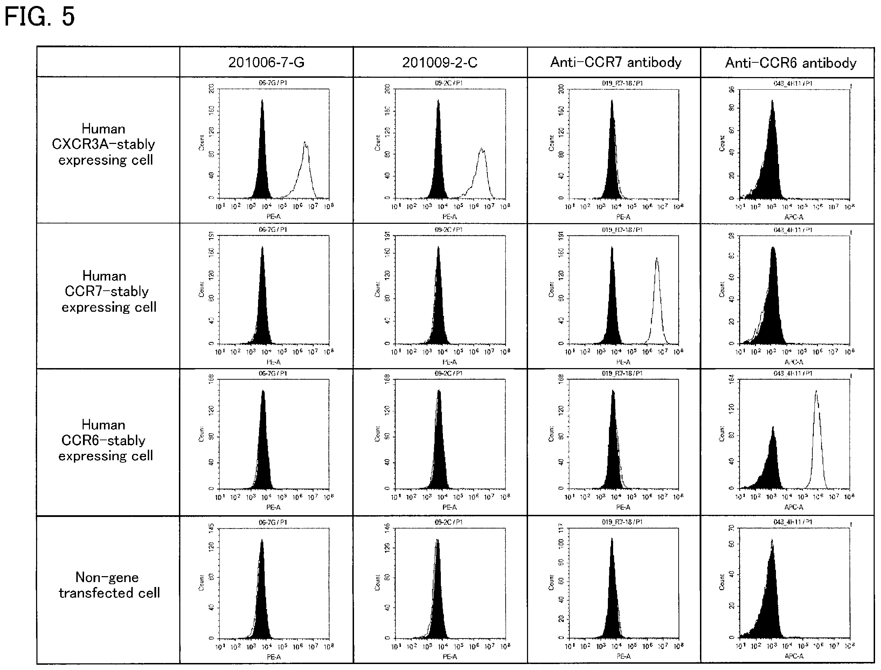

Fig. 5 presents histograms as representative examples, in which 201006-7-G, 201009-2-C, anti-human CCR7 antibodies or anti-human CCR6 antibody (hereinafter collectively referred to as anti-chemokine antibodies) are compared to the control antibody (isotype antibody). In Fig. 5, the blackened graph areas represent isotype antibodies, and the blanked graph areas represent anti-chemokine antibodies. Cases showing a rightward shift in the histogram were judged as "bound". The binding of 201006-7-G and 201009-2-C was observed only in human CXCR3A-stably expressing CHO cells. These results showed that 201006-7-G and 201009-2-C do not bind to other chemokine receptors but specifically bind to human CXCR3A. Also, 13B4, 11F11, 201006-3-H, and 201006-5-F showed results similar to those in Fig. 5. The binding specificities of the six CXCR3 antibodies are presented in Table 6. Cases showing a rightward shift in the histogram were indicated by "+", and cases showing no shift were indicated by "-".

### [Table 6]

**TABLE 6**

| Antibody | Binding specificity | | |
|---|---|---|---|
| | Human CXCR3A | Human CCR7 | Human CCR6 |
| 13B4 | + | - | - |
| 11F11 | + | - | - |
| 201006-3-H | + | - | - |
| 201006-5-F | + | - | - |
| 201006-7-G | + | - | - |
| 201009-2-C | + | - | - |

### [Example 12] Evaluation for Binding Property to Human Peripheral Blood-Derived T Cell by Flow Cytometry

The six antibodies selected in Example 10: 13B4, 11F11, 201006-3-H, 201006-5-F, 201006-7-G, and 201009-2-C were subjected to the following tests.

Frozen human peripheral blood mononuclear cells (Cellular Technology Limited) were thawed and cultured in TexMACS medium (Miltenyi Biotec) containing 1% T cell TransAct, human and 10 ng/mL IL-2 for 3 days to activate the cells. The cells were expansively cultured while renewing the medium every 2 or 3 days (TexMACS medium containing 10 ng/mL IL-2). T cells after an elapse of 10 days of the expansion culture were used for the test. Human T cells were suspended in goat serum (Thermo Fisher Scientific) diluted 1:1 in FACS buffer (PBS containing 1% FBS) at 1×10⁷ cells/mL, and blocked at 4°C for 30 minutes. After the blocking, the cells were resuspended at a cell concentration of 4×10⁶ cells/mL. A 25 µL aliquot of this cell suspension was mixed with 25 µL each of the six types of diluted antibodies or control antibodies, and the mixture was incubated at 4°C for 1 hour. As the control antibodies, a mouse isotype control antibody (FUJIFILM Wako Pure Chemical Corporation) or a rat isotype control antibody (FUJIFILM Wako Pure Chemical Corporation) was used. After the incubation, the cells were washed twice with 120 µL of FACS buffer. As secondary antibodies, 25 µL of a diluent of PE-labeled anti-mouse IgG antibody (SouthernBiotech) or Alexa Fluor 488-labeled anti-rat IgG antibody (Abcam) was added to each well, which was incubated at 4°C for 1 hour. The cells were washed twice with FACS buffer, then suspended in 50 µL of FACS buffer, and the fluorescence intensity on the cell surface was measured using a flow cytometer Novocyte (Agilent Technologies) to evaluate antibody binding properties.

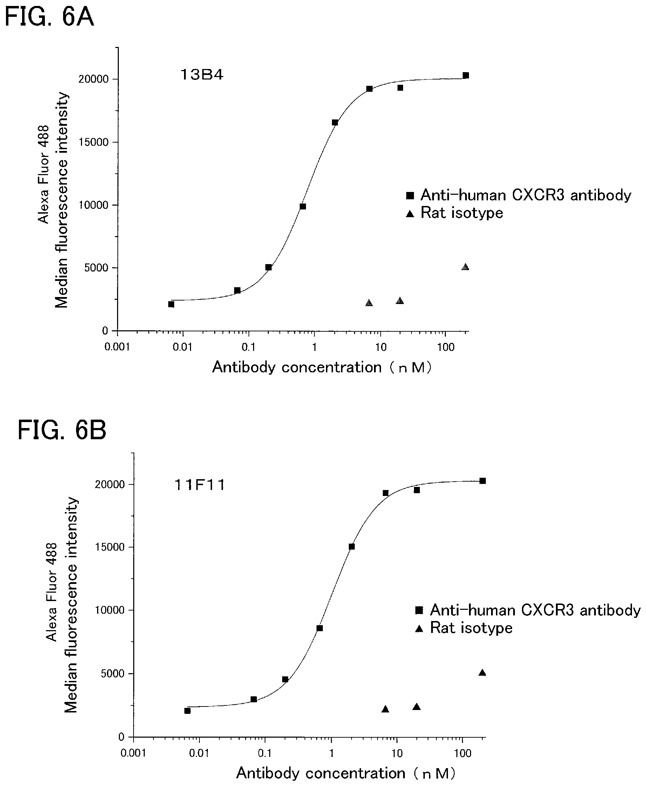

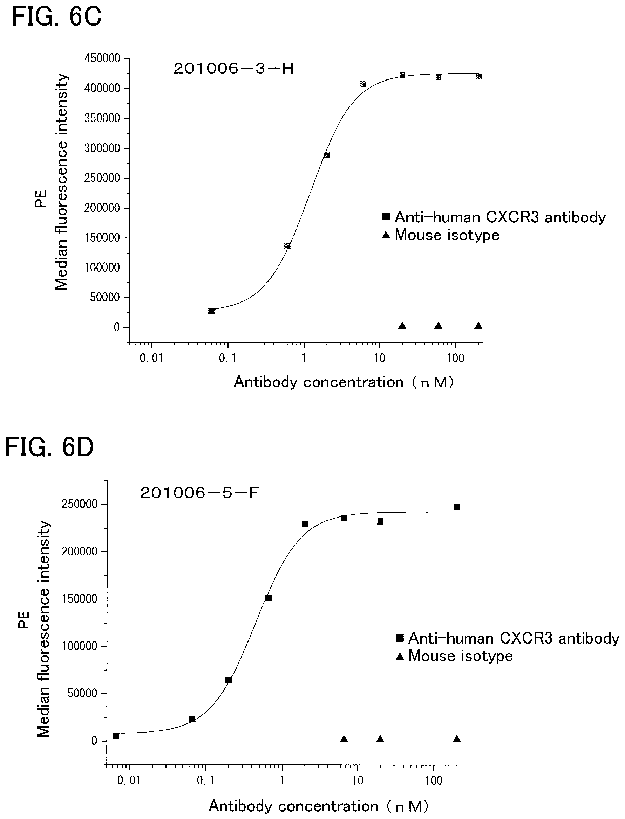

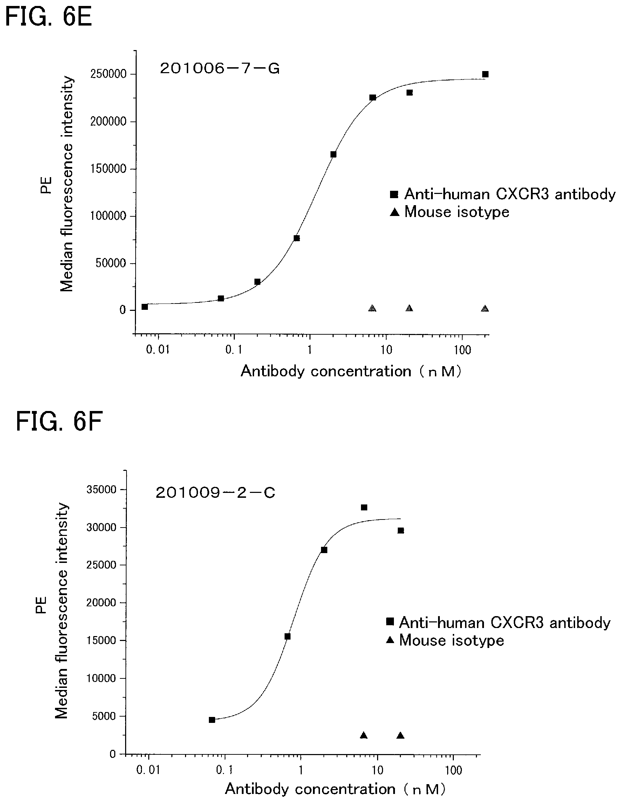

A graph was created, in which the concentration of the antibody was plotted on the horizontal axis, and the median fluorescence intensity of the flow cytometer histogram was plotted on the vertical axis. All of the six antibodies were confirmed to dose-dependently bind to the human T cells. The results of the binding of each antibody to the human T cells are presented in Figs. 6A to 6F. The 50% binding concentration (EC₅₀) was calculated and summarized in Table 7.

### [Table 7]

**TABLE 7**

| Antibody | Cell binding EC₅₀ (nM) |
|---|---|
| 13B4 | 0.77 |
| 11F11 | 1.02 |
| 201006-3-H | 1.19 |
| 201006-5-F | 0.45 |
| 201006-7-G | 1.21 |
| 201009-2-C | 0.68 |

### [Example 13] Evaluation for Human Peripheral Blood-Derived T Cell Migration Inhibitory Activity of Antibodies

The six antibodies selected in Example 10: 13B4, 11F11, 201006-3-H, 201006-5-F, 201006-7-G, and 201009-2-C were subjected to the following tests.

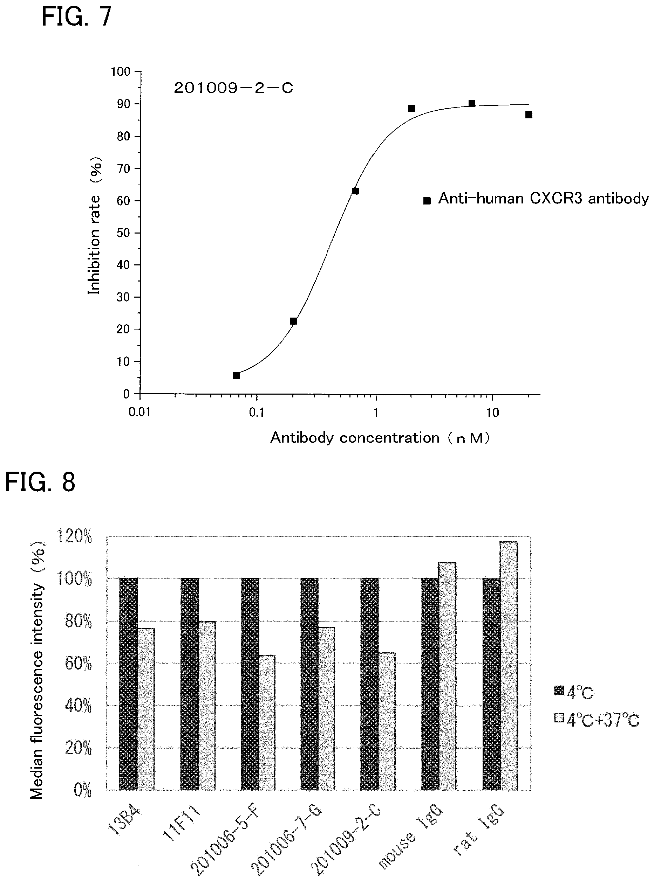

Frozen human peripheral blood mononuclear cells (Cellular Technology Limited) were cultured in the same way as in Example 12. T cells after an elapse of 10 days of the expansion culture were used for the test. The human T cells were washed with the assay buffer (RPMI-1640 + 0.1% BSA). Serially diluted antibodies were added to the cells at a cell density of 2.0 to 2.4×10⁶/mL, which were allowed to stand at 37°C for 60 minutes. IP-10 (2.4 nM; PeproTech) was added into wells of a receiver plate for an insert-integrated 96-well Transwell with a 3.0 µm polycarbonate membrane (Corning). The insert of Transwell was placed onto the receiver plate, and then 75 µL of the cell suspension described above was seeded into the insert and incubated at 37°C for 90 minutes. After the incubation, the insert was removed, and the number of cells that had migrated was counted using a flow cytometer Novocyte (Agilent Technologies). The inhibitory activity was calculated as a relative value based on standardization in which a case of "without antibody and with IP-10" was defined as an inhibition rate of 0% and a case of "without antibody and without IP-10" was defined as an inhibition rate of 100%. Fig. 7 presents the inhibitory effect of 201009-2-C on IP-10-dependent T cell migration as a representative example. The antibody concentration was plotted on the horizontal axis, and the inhibition rate (%) was plotted on the vertical axis. The T cell migration inhibition rates of the antibodies are summarized in Table 8.

All of the six antibodies were confirmed to dose-dependently inhibit the IP-10-induced cell migration in human T cells, and were shown to have an activity of blocking CXCR3-dependent cell function.

### [Table 8]

**TABLE 8**

| Antibody | Cell migration inhibition (%) (2.4 nM IP-10) | | |
|---|---|---|---|
| | 20 nM | 6.6 nM | 0.66 nM |
| 13B4 | 82 | 77 | 56 |
| 11F11 | 84 | 78 | 50 |
| 201006-3-H | 79 | 75 | 31 |
| 201006-5-F | 80 | 75 | 57 |
| 201006-7-G | 80 | 77 | 29 |
| 201009-2-C | 87 | 78 | 43 |

### [Example 14] Evaluation of Antibody Internalization by Flow Cytometry

The five types of antibodies: 13B4, 11F11, 201006-5-F, 201006-7-G, and 201009-2-C were subjected to the following tests.

Frozen human peripheral blood mononuclear cells (Cellular Technology Limited) were cultured in the same way as in Example 12. T cells after an elapse of 10 days of the expansion culture were used for the test. Human T cells were suspended in goat serum (Thermo Fisher Scientific) diluted 1:1 in FACS buffer (PBS containing 1% FBS) at 1×10⁷ cells/mL, and blocked at 4°C for 30 minutes. After the blocking, the cells were resuspended at a cell concentration of 1×10⁵ cells/50 µL. This cell suspension was mixed with each of the five antibodies or control antibodies at an antibody concentration of 1 nM, and the mixture was incubated at 4°C for 30 minutes. As the control antibodies, a mouse isotype control antibody (FUJIFILM Wako Pure Chemical Corporation) or a rat isotype control antibody (FUJIFILM Wako Pure Chemical Corporation) was used. After the incubation, the cells were washed twice with cold FACS buffer. For the purpose of enhancing internalization of the antibody, a portion of the washed cells was incubated at 37°C for 30 minutes. The remaining cells were allowed to stand at 4°C for 30 minutes. As secondary antibodies, 50 µL of a diluent of a PE-labeled anti-mouse IgG antibody (SouthernBiotech) or an Alexa Fluor 488-labeled anti-rat IgG antibody (Abcam PLC) was added to each well, which was incubated at 4°C for 15 minutes. The cells were washed twice with cold FACS buffer, then suspended in 50 µL of cold FACS buffer, and the fluorescence intensity on the cell surface was measured using a flow cytometer Novocyte (Agilent Technologies).

Internalization of the antibodies was indirectly assessed based on the expression level of the receptor on the cell surface. Specifically, the median fluorescence intensity of cells reacted with the antibody at 4°C was normalized as 100%, and the median fluorescence intensity of cells reacted with the antibody at 37°C was expressed as a relative value. Fig. 8 presents the median fluorescence intensity (%) under the various conditions. As indicated by the decrease in the median fluorescence intensity (%) in the cells reacted at 37°C compared to those at 4°C, it was confirmed that the five types of antibodies were internalized into the cells together with the receptors.

### [Example 15] Preparation of Humanized Anti-CXCR3 Antibody

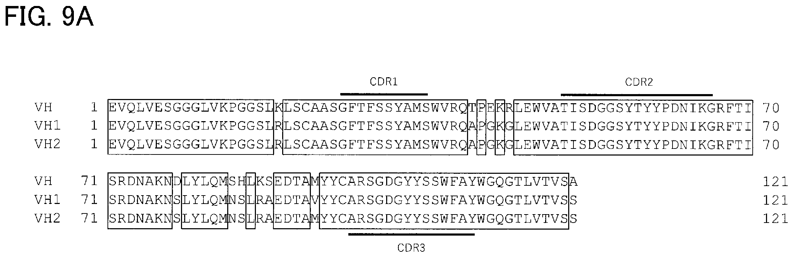

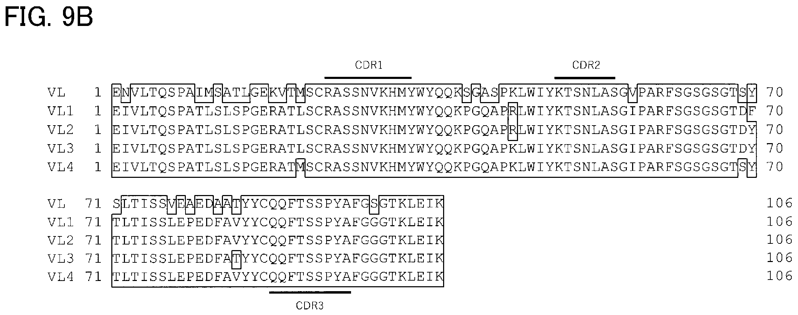

A case of 201009-2-C will be described as an example. To prepare a humanized antibody grafted with CDRs of 201009-2-C, a human framework was selected on the basis of the homology between 201009-2-C and human germline VH and VL genes. In order to support a predicted antibody conformation of 201009-2-C, multiple antibodies with variants of the selected human germline VH and VL sequences: "VH1+VL1", "VH1+VL2", "VH1+VL3", "VH1+VL4", "VH2+VL1", "VH2+VL2", "VH2+VL3", and "VH2+VL4" were designed based on a computer modeling. Fig. 9A presents the alignment of VH1 (SEQ ID NO: 298), VH2 (SEQ ID NO: 299), and 201009-2-C VH region (SEQ ID NO: 257). Fig. 9B presents the alignment of VL1 (SEQ ID NO: 300), VL2 (SEQ ID NO: 301), VL3 (SEQ ID NO: 302), VL4 (SEQ ID NO: 303), and 201009-2-C VL region (SEQ ID NO: 259).

Artificial genes were synthesized, in which the human IgG1 heavy chain constant region (SEQ ID NO: 304) was connected to the VH region and the κ chain constant region (SEQ ID NO: 305) was connected to the VL region in each antibody. As a comparison, an artificial gene of a chimeric anti-CXCR3 antibody "VH+VL-Chimera" was also synthesized, in which the human IgG1 heavy chain constant region was connected to the VH region and the κ chain constant region was connected to the VL region in 201009-2-C. The antibody genes were introduced into CHO cells, and, from the CHO cell culture, purified antibodies were produced from the culture liquids by affinity chromatography and subjected to the following tests. As a result of assaying purities by SDS-PAGE, all the purified antibodies were confirmed to have a purity of 90% or higher.

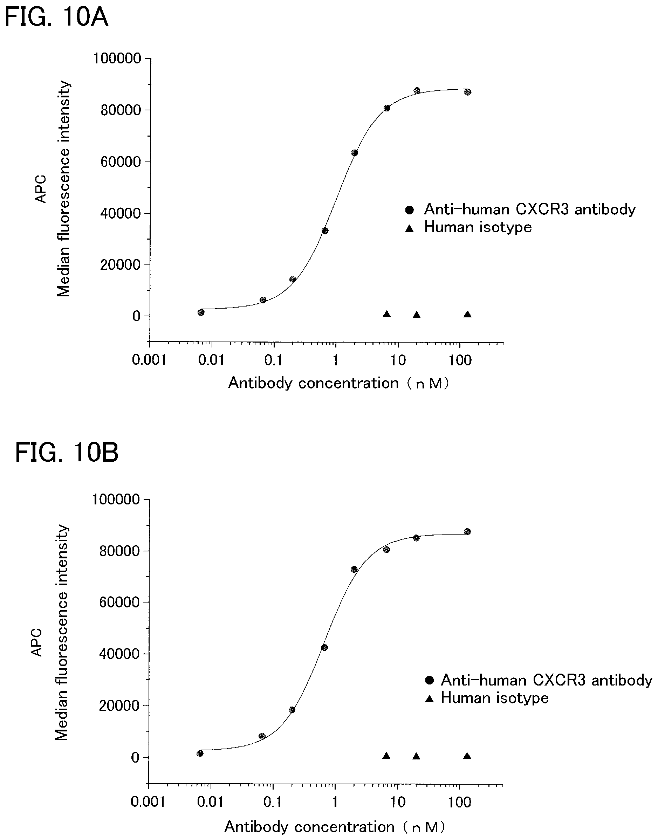

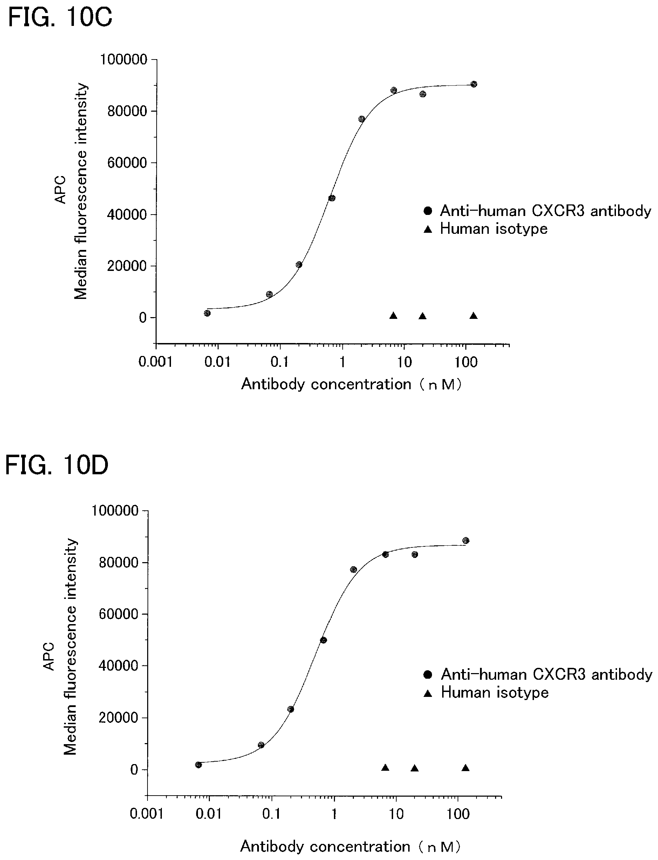

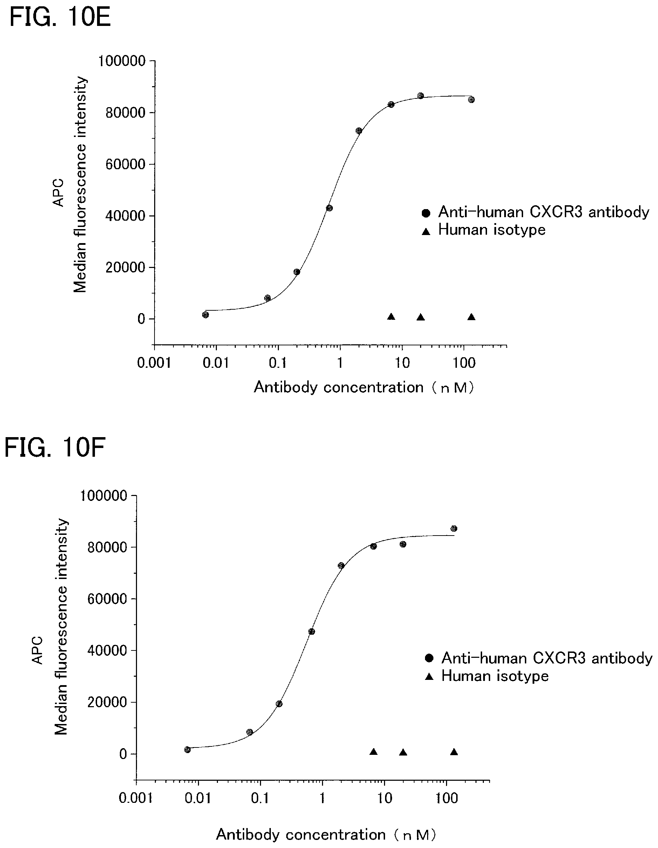

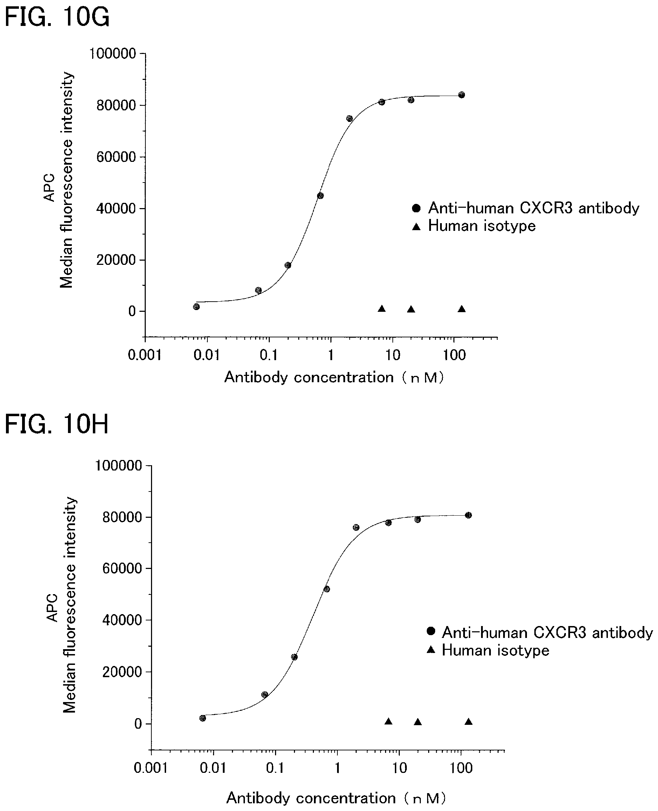

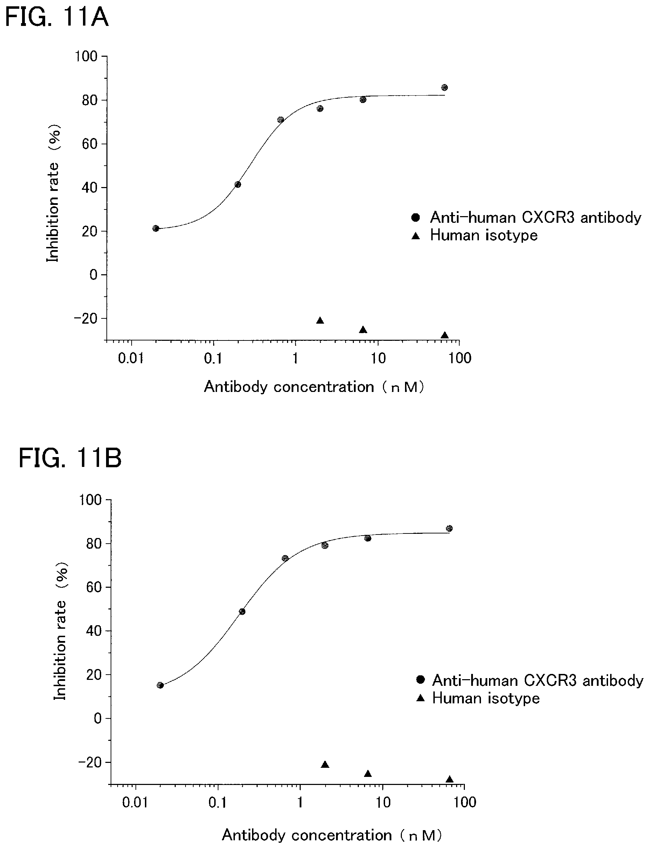

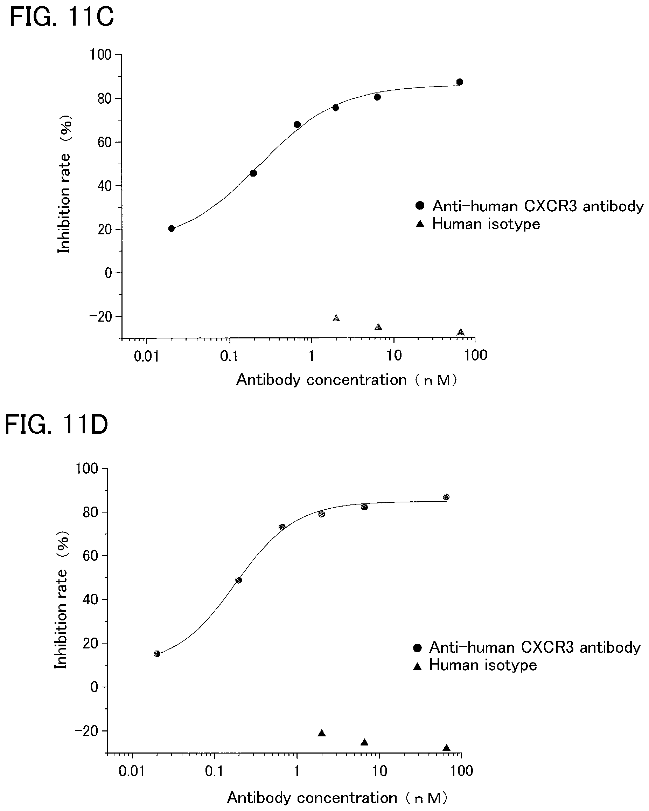

Subsequently, the binding activity of the antibody to human T cells was measured according to the method in Example 12. A graph was created, in which the concentration of the antibody was plotted on the horizontal axis, and the median fluorescence intensity of the flow cytometer histogram was plotted on the vertical axis. The results of the binding of each antibody to human T cells are presented in Figs. 10A to 10H, and the calculated 50% binding concentrations (EC₅₀) are summarized in Table 9. Also, the human T cell migration inhibitory activity of each antibody was evaluated according to the method in Example 13. The dose dependencies of the T cell migration inhibition rates of each antibody are summarized in Table 10. For the activities of "VH1+VL1", "VH1+VL4", "VH2+VL1", and "VH2+VL4", graphs were created, in which the concentration of each antibody was plotted on the horizontal axis and the inhibition rate (%) was plotted on the vertical axis, as presented in Figs. 11A to 11D. The calculated 50% inhibitory concentrations (IC₅₀) are summarized in Table 11. Since the binding activity and inhibitory activity of the VH+VL-chimera were approximately equivalent to those of 201009-2-C, it was confirmed that conversion into the human constant regions did not affect the binding activity and inhibitory activity. Furthermore, since the binding activity and migration inhibitory activity of the eight types of antibodies designed as humanized CXCR3 antibodies were equivalent to those of VH+VL-Chimera, it was confirmed that the binding activity and inhibitory activity were maintained in all the designs after the humanization.

### [Table 9]

**TABLE 9**

| Antibody | Cell binding EC₅₀ (nM) |
|---|---|
| VH1+VL1 | 1.01 |
| VH1+VL2 | 0.67 |
| VH1+VL3 | 0.62 |
| VH1+VL4 | 0.50 |
| VH2+VL1 | 0.66 |
| VH2+VL2 | 0.56 |
| VH2+VL3 | 0.59 |
| VH2+VL4 | 0.40 |
| VH+VL chimera | 0.56 |
| 201009-2-C | 0.74 |

### [Table 10]

**TABLE 10**

| Antibody | Cell migration inhibition (%) (4.6 nM IP-10) | | |
|---|---|---|---|
| | 66 nM | 6.6 nM | 0.66 nM |
| VH1+VL1 | 83 | 78 | 65 |
| VH1+VL2 | 79 | 73 | 52 |
| VH1+VL3 | 76 | 77 | 58 |
| VH1+VL4 | 83 | 83 | 72 |
| VH2+VL1 | 92 | 89 | 71 |
| VH2+VL2 | 90 | 81 | 66 |
| VH2+VL3 | 90 | 82 | 60 |
| VH2+VL4 | 93 | 89 | 76 |
| VH+VL chimera | 92 | 90 | 83 |
| 201009-2-C | 94 | 91 | 77 |

### [Table 11]

**TABLE 11**

| Antibody | Cell migration inhibition IC₅₀ (nM) |
|---|---|
| VH1+VL1 | 0.27 |
| VH1+VL4 | 0.20 |
| VH2+VL1 | 0.24 |
| VH2+VL4 | 0.15 |
| VH+VL chimera | 0.18 |

## Claims

1. An antibody that specifically binds to human CXCR3, wherein
the antibody specifically binds to an extracellular domain of human CXCR3A,
the antibody comprises an activity of blocking a CXCR3A-dependent cell function,
the antibody does not specifically bind to a human vascular endothelial cell, and
the antibody satisfies any one of the following (AB1) to (AB9) and (AB12) to (AB27):
(AB26) comprising a heavy chain CDR1 including an amino acid sequence represented by SEQ ID NO: 251, a heavy chain CDR2 including an amino acid sequence represented by SEQ ID NO: 252, a heavy chain CDR3 including an amino acid sequence represented by SEQ ID NO: 253, a light chain CDR1 including an amino acid sequence represented by SEQ ID NO: 254, a light chain CDR2 including an amino acid sequence represented by SEQ ID NO: 255, and a light chain CDR3 including an amino acid sequence represented by SEQ ID NO: 256;
(AB23) comprising a heavy chain CDR1 including an amino acid sequence represented by SEQ ID NO: 221, a heavy chain CDR2 including an amino acid sequence represented by SEQ ID NO: 222, a heavy chain CDR3 including an amino acid sequence represented by SEQ ID NO: 223, a light chain CDR1 including an amino acid sequence represented by SEQ ID NO: 224, a light chain CDR2 including an amino acid sequence represented by SEQ ID NO: 225, and a light chain CDR3 including an amino acid sequence represented by SEQ ID NO: 226;
(AB24) comprising a heavy chain CDR1 including an amino acid sequence represented by SEQ ID NO: 231, a heavy chain CDR2 including an amino acid sequence represented by SEQ ID NO: 232, a heavy chain CDR3 including an amino acid sequence represented by SEQ ID NO: 233, a light chain CDR1 including an amino acid sequence represented by SEQ ID NO: 234, a light chain CDR2 including an amino acid sequence represented by SEQ ID NO: 235, and a light chain CDR3 including an amino acid sequence represented by SEQ ID NO: 236;
(AB25) comprising a heavy chain CDR1 including an amino acid sequence represented by SEQ ID NO: 241, a heavy chain CDR2 including an amino acid sequence represented by SEQ ID NO: 242, a heavy chain CDR3 including an amino acid sequence represented by SEQ ID NO: 243, a light chain CDR1 including an amino acid sequence represented by SEQ ID NO: 244, a light chain CDR2 including an amino acid sequence represented by SEQ ID NO: 245, and a light chain CDR3 including an amino acid sequence represented by SEQ ID NO: 246;
(AB27) comprising a heavy chain CDR1 including an amino acid sequence represented by SEQ ID NO: 261, a heavy chain CDR2 including an amino acid sequence represented by SEQ ID NO: 262, a heavy chain CDR3 including an amino acid sequence represented by SEQ ID NO: 263, a light chain CDR1 including an amino acid sequence represented by SEQ ID NO: 264, a light chain CDR2 including an amino acid sequence represented by SEQ ID NO: 265, and a light chain CDR3 including an amino acid sequence represented by SEQ ID NO: 266;
(AB1) comprising a heavy chain CDR1 including an amino acid sequence represented by SEQ ID NO: 1, a heavy chain CDR2 including an amino acid sequence represented by SEQ ID NO: 2, a heavy chain CDR3 including an amino acid sequence represented by SEQ ID NO: 3, a light chain CDR1 including an amino acid sequence represented by SEQ ID NO: 4, a light chain CDR2 including an amino acid sequence represented by SEQ ID NO: 5, and a light chain CDR3 including an amino acid sequence represented by SEQ ID NO: 6;
(AB2) comprising a heavy chain CDR1 including an amino acid sequence represented by SEQ ID NO: 11, a heavy chain CDR2 including an amino acid sequence represented by SEQ ID NO: 12, a heavy chain CDR3 including an amino acid sequence represented by SEQ ID NO: 13, a light chain CDR1 including an amino acid sequence represented by SEQ ID NO: 14, a light chain CDR2 including an amino acid sequence represented by SEQ ID NO: 15, and a light chain CDR3 including an amino acid sequence represented by SEQ ID NO: 16;
(AB3) comprising a heavy chain CDR1 including an amino acid sequence represented by SEQ ID NO: 21, a heavy chain CDR2 including an amino acid sequence represented by SEQ ID NO: 22, a heavy chain CDR3 including an amino acid sequence represented by SEQ ID NO: 23, a light chain CDR1 including an amino acid sequence represented by SEQ ID NO: 24, a light chain CDR2 including an amino acid sequence represented by SEQ ID NO: 25, and a light chain CDR3 including an amino acid sequence represented by SEQ ID NO: 26;
(AB5) comprising a heavy chain CDR1 including an amino acid sequence represented by SEQ ID NO: 41, a heavy chain CDR2 including an amino acid sequence represented by SEQ ID NO: 42, a heavy chain CDR3 including an amino acid sequence represented by SEQ ID NO: 43, a light chain CDR1 including an amino acid sequence represented by SEQ ID NO: 44, a light chain CDR2 including an amino acid sequence represented by SEQ ID NO: 45, and a light chain CDR3 including an amino acid sequence represented by SEQ ID NO: 46;
(AB8) comprising a heavy chain CDR1 including an amino acid sequence represented by SEQ ID NO: 71, a heavy chain CDR2 including an amino acid sequence represented by SEQ ID NO: 72, a heavy chain CDR3 including an amino acid sequence represented by SEQ ID NO: 73, a light chain CDR1 including an amino acid sequence represented by SEQ ID NO: 74, a light chain CDR2 including an amino acid sequence represented by SEQ ID NO: 75, and a light chain CDR3 including an amino acid sequence represented by SEQ ID NO: 76;
(AB16) comprising a heavy chain CDR1 including an amino acid sequence represented by SEQ ID NO: 151, a heavy chain CDR2 including an amino acid sequence represented by SEQ ID NO: 152, a heavy chain CDR3 including an amino acid sequence represented by SEQ ID NO: 153, a light chain CDR1 including an amino acid sequence represented by SEQ ID NO: 154, a light chain CDR2 including an amino acid sequence represented by SEQ ID NO: 155, and a light chain CDR3 including an amino acid sequence represented by SEQ ID NO: 156;
(AB17) comprising a heavy chain CDR1 including an amino acid sequence represented by SEQ ID NO: 161, a heavy chain CDR2 including an amino acid sequence represented by SEQ ID NO: 162, a heavy chain CDR3 including an amino acid sequence represented by SEQ ID NO: 163, a light chain CDR1 including an amino acid sequence represented by SEQ ID NO: 164, a light chain CDR2 including an amino acid sequence represented by SEQ ID NO: 165, and a light chain CDR3 including an amino acid sequence represented by SEQ ID NO: 166;
(AB18) comprising a heavy chain CDR1 including an amino acid sequence represented by SEQ ID NO: 171, a heavy chain CDR2 including an amino acid sequence represented by SEQ ID NO: 172, a heavy chain CDR3 including an amino acid sequence represented by SEQ ID NO: 173, a light chain CDR1 including an amino acid sequence represented by SEQ ID NO: 174, a light chain CDR2 including an amino acid sequence represented by SEQ ID NO: 175, and a light chain CDR3 including an amino acid sequence represented by SEQ ID NO: 176;
(AB20) comprising a heavy chain CDR1 including an amino acid sequence represented by SEQ ID NO: 191, a heavy chain CDR2 including an amino acid sequence represented by SEQ ID NO: 192, a heavy chain CDR3 including an amino acid sequence represented by SEQ ID NO: 193, a light chain CDR1 including an amino acid sequence represented by SEQ ID NO: 194, a light chain CDR2 including an amino acid sequence represented by SEQ ID NO: 195, and a light chain CDR3 including an amino acid sequence represented by SEQ ID NO: 196; and
(AB21) comprising a heavy chain CDR1 including an amino acid sequence represented by SEQ ID NO: 201, a heavy chain CDR2 including an amino acid sequence represented by SEQ ID NO: 202, a heavy chain CDR3 including an amino acid sequence represented by SEQ ID NO: 203, a light chain CDR1 including an amino acid sequence represented by SEQ ID NO: 204, a light chain CDR2 including an amino acid sequence represented by SEQ ID NO: 205, and a light chain CDR3 including an amino acid sequence represented by SEQ ID NO: 206;
(AB22) comprising a heavy chain CDR1 including an amino acid sequence represented by SEQ ID NO: 211, a heavy chain CDR2 including an amino acid sequence represented by SEQ ID NO: 212, a heavy chain CDR3 including an amino acid sequence represented by SEQ ID NO: 213, a light chain CDR1 including an amino acid sequence represented by SEQ ID NO: 214, a light chain CDR2 including an amino acid sequence represented by SEQ ID NO: 215, and a light chain CDR3 including an amino acid sequence represented by SEQ ID NO: 216;
(AB4) comprising a heavy chain CDR1 including an amino acid sequence represented by SEQ ID NO: 31, a heavy chain CDR2 including an amino acid sequence represented by SEQ ID NO: 32, a heavy chain CDR3 including an amino acid sequence represented by SEQ ID NO: 33, a light chain CDR1 including an amino acid sequence represented by SEQ ID NO: 34, a light chain CDR2 including an amino acid sequence represented by SEQ ID NO: 35, and a light chain CDR3 including an amino acid sequence represented by SEQ ID NO: 36;
(AB6) comprising a heavy chain CDR1 including an amino acid sequence represented by SEQ ID NO: 51, a heavy chain CDR2 including an amino acid sequence represented by SEQ ID NO: 52, a heavy chain CDR3 including an amino acid sequence represented by SEQ ID NO: 53, a light chain CDR1 including an amino acid sequence represented by SEQ ID NO: 54, a light chain CDR2 including an amino acid sequence represented by SEQ ID NO: 55, and a light chain CDR3 including an amino acid sequence represented by SEQ ID NO: 56;
(AB7) comprising a heavy chain CDR1 including an amino acid sequence represented by SEQ ID NO: 61, a heavy chain CDR2 including an amino acid sequence represented by SEQ ID NO: 62, a heavy chain CDR3 including an amino acid sequence represented by SEQ ID NO: 63, a light chain CDR1 including an amino acid sequence represented by SEQ ID NO: 64, a light chain CDR2 including an amino acid sequence represented by SEQ ID NO: 65, and a light chain CDR3 including an amino acid sequence represented by SEQ ID NO: 66;
(AB9) comprising a heavy chain CDR1 including an amino acid sequence represented by SEQ ID NO: 81, a heavy chain CDR2 including an amino acid sequence represented by SEQ ID NO: 82, a heavy chain CDR3 including an amino acid sequence represented by SEQ ID NO: 83, a light chain CDR1 including an amino acid sequence represented by SEQ ID NO: 84, a light chain CDR2 including an amino acid sequence represented by SEQ ID NO: 85, and a light chain CDR3 including an amino acid sequence represented by SEQ ID NO: 86;
(AB12) comprising a heavy chain CDR1 including an amino acid sequence represented by SEQ ID NO: 111, a heavy chain CDR2 including an amino acid sequence represented by SEQ ID NO: 112, a heavy chain CDR3 including an amino acid sequence represented by SEQ ID NO: 113, a light chain CDR1 including an amino acid sequence represented by SEQ ID NO: 114, a light chain CDR2 including an amino acid sequence represented by SEQ ID NO: 115, and a light chain CDR3 including an amino acid sequence represented by SEQ ID NO: 116;
(AB13) comprising a heavy chain CDR1 including an amino acid sequence represented by SEQ ID NO: 121, a heavy chain CDR2 including an amino acid sequence represented by SEQ ID NO: 122, a heavy chain CDR3 including an amino acid sequence represented by SEQ ID NO: 123, a light chain CDR1 including an amino acid sequence represented by SEQ ID NO: 124, a light chain CDR2 including an amino acid sequence represented by SEQ ID NO: 125, and a light chain CDR3 including an amino acid sequence represented by SEQ ID NO: 126;
(AB14) comprising a heavy chain CDR1 including an amino acid sequence represented by SEQ ID NO: 131, a heavy chain CDR2 including an amino acid sequence represented by SEQ ID NO: 132, a heavy chain CDR3 including an amino acid sequence represented by SEQ ID NO: 133, a light chain CDR1 including an amino acid sequence represented by SEQ ID NO: 134, a light chain CDR2 including an amino acid sequence represented by SEQ ID NO: 135, and a light chain CDR3 including an amino acid sequence represented by SEQ ID NO: 136;
(AB15) comprising a heavy chain CDR1 including an amino acid sequence represented by SEQ ID NO: 141, a heavy chain CDR2 including an amino acid sequence represented by SEQ ID NO: 142, a heavy chain CDR3 including an amino acid sequence represented by SEQ ID NO: 143, a light chain CDR1 including an amino acid sequence represented by SEQ ID NO: 144, a light chain CDR2 including an amino acid sequence represented by SEQ ID NO: 145, and a light chain CDR3 including an amino acid sequence represented by SEQ ID NO: 146; and
(AB19) comprising a heavy chain CDR1 including an amino acid sequence represented by SEQ ID NO: 181, a heavy chain CDR2 including an amino acid sequence represented by SEQ ID NO: 182, a heavy chain CDR3 including an amino acid sequence represented by SEQ ID NO: 183, a light chain CDR1 including an amino acid sequence represented by SEQ ID NO: 184, a light chain CDR2 including an amino acid sequence represented by SEQ ID NO: 185, and a light chain CDR3 including an amino acid sequence represented by SEQ ID NO: 186.

2. An antibody that specifically binds to human CXCR3, wherein
the antibody specifically binds to an extracellular domain of human CXCR3A,
the antibody comprises an activity of blocking a CXCR3A-dependent cell function,
the antibody does not specifically bind to a human vascular endothelial cell, and
the antibody satisfies any one of the following (C1) to (C9) and (C12) to (C27):
(C26) comprising a heavy chain variable region including an amino acid sequence represented by SEQ ID NO: 257 and a light chain variable region including an amino acid sequence represented by SEQ ID NO: 259;
(C23) comprising a heavy chain variable region including an amino acid sequence represented by SEQ ID NO: 227 and a light chain variable region including an amino acid sequence represented by SEQ ID NO: 229;
(C24) comprising a heavy chain variable region including an amino acid sequence represented by SEQ ID NO: 237 and a light chain variable region including an amino acid sequence represented by SEQ ID NO: 239;
(C25) comprising a heavy chain variable region including an amino acid sequence represented by SEQ ID NO: 247 and a light chain variable region including an amino acid sequence represented by SEQ ID NO: 249;
(C27) comprising a heavy chain variable region including an amino acid sequence represented by SEQ ID NO: 267 and a light chain variable region including an amino acid sequence represented by SEQ ID NO: 269;
(C1) comprising a heavy chain variable region including an amino acid sequence represented by SEQ ID NO: 7 and a light chain variable region including an amino acid sequence represented by SEQ ID NO: 9;
(C2) comprising a heavy chain variable region including an amino acid sequence represented by SEQ ID NO: 17 and a light chain variable region including an amino acid sequence represented by SEQ ID NO: 19;
(C3) comprising a heavy chain variable region including an amino acid sequence represented by SEQ ID NO: 27 and a light chain variable region including an amino acid sequence represented by SEQ ID NO: 29;
(C5) comprising a heavy chain variable region including an amino acid sequence represented by SEQ ID NO: 47 and a light chain variable region including an amino acid sequence represented by SEQ ID NO: 49;
(C8) comprising a heavy chain variable region including an amino acid sequence represented by SEQ ID NO: 77 and a light chain variable region including an amino acid sequence represented by SEQ ID NO: 79;
(C16) comprising a heavy chain variable region including an amino acid sequence represented by SEQ ID NO: 157 and a light chain variable region including an amino acid sequence represented by SEQ ID NO: 159;
(C17) comprising a heavy chain variable region including an amino acid sequence represented by SEQ ID NO: 167 and a light chain variable region including an amino acid sequence represented by SEQ ID NO: 169;
(C18) comprising a heavy chain variable region including an amino acid sequence represented by SEQ ID NO: 177 and a light chain variable region including an amino acid sequence represented by SEQ ID NO: 179;
(C20) comprising a heavy chain variable region including an amino acid sequence represented by SEQ ID NO: 197 and a light chain variable region including an amino acid sequence represented by SEQ ID NO: 199; and
(C21) comprising a heavy chain variable region including an amino acid sequence represented by SEQ ID NO: 207 and a light chain variable region including an amino acid sequence represented by SEQ ID NO: 209.
(C22) comprising a heavy chain variable region including an amino acid sequence represented by SEQ ID NO: 217 and a light chain variable region including an amino acid sequence represented by SEQ ID NO: 219;
(C4) comprising a heavy chain variable region including an amino acid sequence represented by SEQ ID NO: 37 and a light chain variable region including an amino acid sequence represented by SEQ ID NO: 39;
(C6) comprising a heavy chain variable region including an amino acid sequence represented by SEQ ID NO: 57 and a light chain variable region including an amino acid sequence represented by SEQ ID NO: 59;
(C7) comprising a heavy chain variable region including an amino acid sequence represented by SEQ ID NO: 67 and a light chain variable region including an amino acid sequence represented by SEQ ID NO: 69;
(C9) comprising a heavy chain variable region including an amino acid sequence represented by SEQ ID NO: 87 and a light chain variable region including an amino acid sequence represented by SEQ ID NO: 89;
(C12) comprising a heavy chain variable region including an amino acid sequence represented by SEQ ID NO: 117 and a light chain variable region including an amino acid sequence represented by SEQ ID NO: 119;
(C13) comprising a heavy chain variable region including an amino acid sequence represented by SEQ ID NO: 127 and a light chain variable region including an amino acid sequence represented by SEQ ID NO: 129;
(C14) comprising a heavy chain variable region including an amino acid sequence represented by SEQ ID NO: 137 and a light chain variable region including an amino acid sequence represented by SEQ ID NO: 139;
(C15) comprising a heavy chain variable region including an amino acid sequence represented by SEQ ID NO: 147 and a light chain variable region including an amino acid sequence represented by SEQ ID NO: 149; and
(C19) comprising a heavy chain variable region including an amino acid sequence represented by SEQ ID NO: 187 and a light chain variable region including an amino acid sequence represented by SEQ ID NO: 189.

3. A second antibody that specifically binds to human CXCR3, wherein
the second antibody specifically binds to an extracellular domain of human CXCR3A,
the second antibody comprises an activity of blocking a CXCR3A-dependent cell function,
the second antibody does not specifically bind to a human vascular endothelial cell, and
the second antibody competitively inhibits a binding between a first antibody that is the antibody according to claim 2 and a receptor.

4. The antibody according to claim 1 or 3, wherein the antibody or the second antibody is a humanized antibody or a chimeric antibody.

5. The antibody according to any one of claims 1 to 4, wherein the antibody or the second antibody is a multispecific antibody.

6. The antibody according to any one of claims 1 to 5, wherein the antibody or the second antibody comprises an internalization activity.

7. The antibody according to any one of claims 1 to 6, wherein the antibody or the second antibody is a modified antibody bonded with another molecule.

8. The antibody according to claim 7, wherein the modified antibody is an antibody-drug conjugate.

9. A nucleic acid encoding the antibody according to any one of claims 1 to 6.

10. A cell comprising the nucleic acid according to claim 9.

11. A medicament comprising the antibody according to any one of claims 1 to 8 as an active ingredient.

12. The medicament according to claim 11, wherein the medicament is for use in the treatment of a disease, disorder, or symptom involving impairment of a CXCR3-dependent cell function.

13. The medicament according to claim 12, wherein the disease, disorder, or symptom is a Th1-type immune disease.

14. The medicament according to claim 13, wherein the Th1-type immune disease is a cardiovascular disorder, a neurological disorder, an inflammatory disease, an autoimmune disease, a metabolic disease, an infectious disease, a hematological cancer, or a solid cancer.
